# EUROPÄISCHE PATENTANMELDUNG

(11) **EP 2 006 388 A1**
(43) Veröffentlichungstag der Anmeldung: **24.12.2008**
(21) Anmeldenummer: 07110607.4
(22) Anmeldetag: 19.06.2007
(51) Int. Cl.: C12N 15/90

(54) **Nukleinsäurekonstrukte zur Inaktivierung und konditionalen Mutagenese von Genen**

(71) Anmelder: Helmholtz-Zentrum für Infektionsforschung GmbH, 38124 Braunschweig (DE)
(72) Erfinder: Hafner, Martin, 38300 Wolfenbüttel (DE); Müller, Werner, 38124 Braunschweig (DE); Nawrath, Karina, 38112 Braunschweig (DE); Kochut, Annika, 38272 Burgdorf (DE)
(74) Vertreter: Taruttis, Stefan Georg

(57) **Zusammenfassung**

Die vorliegende Erfindung betrifft Nukleinsäurekonstrukte, die zur Inaktivierung von Genen bei Eukaryonten eingesetzt werden können. Mit den Nuklcinsäurckonstrukten können Zellen erhalten werden, bei denen durch Insertion eines Nukleinsäurekonstrukts zumindest ein Gen inaktiviert, konditional inaktivierbar, zunächst inaktiviert und konditional reaktivierbar ist oder seine Aktivität mehrfach konditional geändert werden kann. Dies betrifft insbesondere die gezielte Mutagenese von Genen in nicht-menschlichen ES-Zellen und daraus herstellbare nicht-menschliche Säugetiere.

Die Nukleinsäurekonstrukte weisen eine Spleißakzeptorstelle, in 5' Position von der Spleißakzeptorstelle den synthetischen oder natürlichen Abschnitt einer Intronsequenz, der alle für die Funktion eines 3'-Endes eines Introns notwendigen funktionalen Elemente enthält, vorzugsweise umfassend einen 10-50 Nukleotide in 5' von der Spleißakzeptorstelle beabstandeten Verzweigungspunkt, zwischen Verzweigungspunkt und Spleißakzeptorstelle einen Polypyrimidinabschnitt, in 3' von der Spleißakzeptorstelle eine Spleißdonorstelle, wobei zwischen Spleißakzeptorstelle und Spleißdonorstelle ein funktionales Exon gebildet wird, das mindestens ein Stopkodon enthält und bevorzugt mehr als 50 bp von der Spleißdonorstelle beabstandet ist sowie 3' von der Spleißdonorstelle einen natürlichen oder synthetischen Intronabschnitt, der alle für die Funktion eines 5'-Endes eines Introns notwendigen Elemente enthält, auf. Die Nukleinsäurekonstrukte können Bestandteile umfänglicherer Konstrukte zur gezielten Mutagenese mittels homolger Rekombination oder für die ungerichtete Mutagenese als Genfallen, retrovirale Vektoren oder Transposons ausgeführt sein.

SEQUENCE LISTING

<110>  Helmholtz-Zentrum für Infektionsforschung mbH

<120>  Nukleinsäurekonstrukte zur Inaktivierung und konditionalen
       Mutagenese von Genen

<130>  G1011EP

<160>  4

<170>  PatentIn version 3.4

<210>  1
<211>  469
<212>  DNA
<213>  artificial

<220>
<223>  NMD-Kassette in Vektor, spezifisch für beta-7-Integringen der
       Maus


<220>
<221>  Intron
<222>  (7)..(161)
<223>  3´-Ende des Introns 13 des beta-7-Integringens der Maus

<220>
<221>  misc_feature
<222>  (87)..(91)
<223>  Verzweigungspunkt

<220>
<221>  misc_feature
<222>  (162)..(322)
<223>  modifiziertes Exon 14 des beta-7-Integringens der Maus

<220>
<221>  misc_feature
<222>  (245)..(247)
<223>  prämatures Stopkodon im natürlichen Leserahmen des Exons 14 des
       beta-7-Integringens der Maus

<220>
<221>  misc_feature
<222>  (323)..(463)
<223>  5´-Ende von Intron 14 des beta-7-Integringens der Maus

<400>  1
ctgcagccaa cttagggcaa aggcagagat gagatgaagg ccctaggagg gaggacaacc      60

ttgggctctg ctgggtcccg ttctcaatga cagggctggc tggggaggct ggacatcctt     120

tgggtagctg tgggtcagcc cttaaacctt ggccccacca gagggagtgg atcacacccg     180

tgccatcata ctgggctgca cagggggcat cgtggcagtg ggactagggc tggttctggc     240

ttactgactc tctgtggaaa tctacgaccg acgggagtac aggcgctttg agaaggagca     300

gcagcaactc aactggaagc aggtgaggcc agcgactgct gccacaggct gggctttcat     360

ggtgacgtct cttaactttc tgtatcccta acacataacc agctttaaag cttccccgtg     420

caagtccctc cctcggcata accaggcctc ggagtgcaga tctctgcag                 469


<210>  2
<211>  377

<212>  DNA
<213>  artificial

<220>
<223>  NMD-Kassette


<220>
<221>  misc_feature
<222>  (8)..(94)
<223>  3´-Ende des Introns 22 des menschlichen RB1-Gens

<220>
<221>  misc_feature
<222>  (32)..(38)
<223>  Verzweigungspunkt

<220>
<221>  misc_feature
<222>  (71)..(92)
<223>  Polypyrimidinabschnitt

<220>
<221>  misc_feature
<222>  (95)..(95)
<223>  Spleißakzeptorstelle

<220>
<221>  misc_feature
<222>  (95)..(270)
<223>  modifiziertes Exon 23 des menschlichen RB1-Gens

<220>
<221>  misc_feature
<222>  (113)..(115)
<223>  prämatures Stopkodon, Rahmen 1

<220>
<221>  misc_feature
<222>  (117)..(119)
<223>  prämatures Stopkodon, Rahmen 2

<220>
<221>  misc_feature
<222>  (121)..(123)
<223>  prämatures Stopkodon, Rahmen 3

<220>
<221>  misc_feature
<222>  (270)..(270)
<223>  Spleißdonorstelle

<220>
<221>  misc_feature
<222>  (271)..(370)
<223>  5´-Ende des Introns 23 des menschlichen RB1-Gens

<400>  2
atcgatgcaa aagtatcctt tgattggaaa aatctaatgt aatgggtcca ccaaaacatt     60

aaataaataa tctacttttt tgtttttgct ctagccccct accttgtcac cgtaactagc    120

taagcttcct cacattcctc gaagccctta caagtttcct agttcaccct tacggattcc    180

tggagggaac atctatattt cacccctgaa gagtccatat aaaatttcag aaggtctgcc    240

aacaccaaca aaaatgactc caagatcaag gtgtgtgttt tctctttagg gaagtagtaa    300

agaatgagag ggggattatt ttgatccaag aataaaaaat ataaagcatt cttcatttca    360

aataagctag catcgat                                                    377

<210>  3
<211>  475
<212>  DNA
<213>  artificial

<220>
<223>  NMD-Kassette


<220>
<221>  misc_feature
<222>  (7)..(161)
<223>  3´-Ende des Introns 13 des beta-7-Integringens der Maus

<220>
<221>  misc_feature
<222>  (87)..(91)
<223>  Verzweigungspunkt

<220>
<221>  misc_feature
<222>  (162)..(162)
<223>  Spleißakzeptorstelle

<220>
<221>  misc_feature
<222>  (162)..(328)
<223>  modifiziertes Exon 14 des beta-7-Integringens der Maus

<220>
<221>  misc_feature
<222>  (225)..(227)
<223>  prämatures Stopkodon, Rahmen 3

<220>
<221>  misc_feature
<222>  (239)..(241)
<223>  prämatures Stopkodon, Rahmen 2

<220>
<221>  misc_feature
<222>  (243)..(245)
<223>  prämatures Stopkodon, Rahmen 3

<220>
<221>  misc_feature
<222>  (247)..(249)
<223>  prämatures Stopkodon, Rahmen 1

<220>
<221>  misc_feature
<222>  (251)..(253)
<223>  prämatures Stopkodon, Rahmen 2

<220>
<221>  misc_feature
<222>  (328)..(328)
<223>  Spleißdonorstelle

<220>
<221>  misc_feature
<222>  (329)..(469)
<223>  5´-Ende des Introns 14 des beta-7-Integringens der Maus

<400>  3

ctgcagccaa cttagggcaa aggcagagat gagatgaagg ccctaggagg gaggacaacc 60

ttgggctctg ctgggtcccg ttctcaatga cagggctggc tggggaggct ggacatcctt 120

tgggtagctg tgggtcagcc cttaaacctt ggccccacca gagggagtgg atcacacccg 180

tgccatcata ctgggctgca cagggggcat cgtggcagtg ggactagggc tggttcgcta 240

gctaactaac tgactctctg tggaaatcta cgaccgacgg gagtacaggc gctttgagaa 300

ggagcagcag caactcaact ggaagcaggt gaggccagcg actgctgcca caggctgggc 360

tttcatggtg acgtctctta actttctgta tccctaacac ataaccagct ttaaagcttc 420

cccgtgcaag tccctccctc ggcataacca ggcctcggag tgcagatctc tgcag 475


<210> 4
<211> 2842
<212> DNA
<213> artificial

<220>
<223> Transponierbare, zweifach invertierbare NMD-Kassette mit
 deletierbarer Expressionskassette eines Resistenzgens


<220>
<221> misc_feature
<222> (1)..(60)
<223> multiple Klonierstelle

<220>
<221> misc_feature
<222> (56)..(74)
<223> 19 bp Mosaik-Endsequenz

<220>
<221> misc_feature
<222> (92)..(139)
<223> frt LE Erkennungssequenz

<220>
<221> misc_feature
<222> (140)..(173)
<223> lox66 Erkennungssequenz

<220>
<221> misc_feature
<222> (188)..(550)
<223> NMD-Kassette

<220>
<221> misc_feature
<222> (188)..(274)
<223> 3´-Ende des Introns 22 den menschlichen RB1-Gens

<220>
<221> misc_feature
<222> (212)..(218)
<223> Verzweigungspunkt

<220>
<221> misc_feature
<222> (251)..(272)
<223> Polypyrimidinabschnitt

<220>
<221> misc_feature

<222>  (275)..(275)
<223>  Spleißakzeptorstelle

<220>
<221>  misc_feature
<222>  (275)..(450)
<223>  modifiziertes Exon 23 des menschlichen RB1-Gens (Retinoblastom1)

<220>
<221>  misc_feature
<222>  (293)..(295)
<223>  prämatures Stopkodon, Rahmen 1

<220>
<221>  misc_feature
<222>  (297)..(299)
<223>  prämatures Stopkodon, Rahmen 2

<220>
<221>  misc_feature
<222>  (301)..(303)
<223>  prämatures Stopkodon, Rahmen 3

<220>
<221>  misc_feature
<222>  (450)..(450)
<223>  Spleißdonorstelle

<220>
<221>  misc_feature
<222>  (451)..(550)
<223>  5´-Ende des Introns 23 den menschlichen RB1-Gens

<220>
<221>  misc_feature
<222>  (567)..(600)
<223>  (komplementär) lox71 Erkennungssequenz

<220>
<221>  misc_feature
<222>  (601)..(648)
<223>  (komplementär) frt RE Erkennungssequenz

<220>
<221>  misc_feature
<222>  (655)..(704)
<223>  attP50 Erkennungssequenz

<220>
<221>  promoter
<222>  (757)..(1343)
<223>  eukaryontischer pGK-Promotor

<220>
<221>  promoter
<222>  (1350)..(1422)
<223>  prokaryontischer gb2-Promotor

<220>
<221>  CDS
<222>  (1423)..(2226)
<223>  Neomycin-/Kanamycin-Resistenz

<220>
<221>  polyA_signal
<222>  (2227)..(2686)

<220>
<221>  misc_feature

<222>  (2687)..(2739)
<223>  attB50 Erkennungssequenz

<220>
<221>  misc_feature
<222>  (2763)..(2781)
<223>  (komplementär) 19bp Mosaik-Endsequenz

<220>
<221>  misc_feature
<222>  (2777)..(2842)
<223>  multiple Klonierungsstelle

<400>  4
ggtaccgcga tcgcaacgtt gtgcactgat cacaattgca tatgacatgt gacagctgtc      60

tcttatacac atctcaaccc tcgagaagct tgaagttcct attccgaagt tcatattctc     120

tagaaagtat aggaacttca taacttcgta taatgtatgc tatacgaacg gtactgcagc     180

atcgatgcaa aagtatcctt tgattggaaa aatctaatgt aatgggtcca ccaaaacatt     240

aaataaataa tctacttttt tgtttttgct ctagccccct accttgtcac cgtaactagc     300

taagcttcct cacattcctc gaagccctta caagtttcct agttcaccct tacggattcc     360

tggagggaac atctatattt cacccctgaa gagtccatat aaaatttcag aaggtctgcc     420

aacaccaaca aaaatgactc caagatcaag gtgtgtgttt tctctttagg gaagtagtaa     480

agaatgagag ggggattatt ttgatccaag aataaaaaat ataaagcatt cttcatttca     540

aataagctag catcgatggt ttaaacataa cttcgtatag catacattat acgaacggta     600

gaagttcata tactttctag agaataggaa cttcggaata ggaacttcgt cgacgtagtg     660

ccccaactgg ggtaaccttt gagttctctc agttgggggc gtagttataa tctagaacta     720

gtggatccac gattcgaggg cccctgcagg tcaattctac cgggtagggg aggcgctttt     780

cccaaggcag tctggagcat gcgctttagc agccccgctg ggcacttggc gctacacaag     840

tggcctctgg ctcgcacaca ttccacatcc accggtaggc gccaaccggc tccgttcttt     900

ggtggcccct tcgcgccacc ttctactcct cccctagtca ggaagttccc ccccgccccg     960

cagctcgcgt cgtgcaggac gtgacaaatg gaagtagcac gtctcactag tctcgtgcag    1020

atggacagca ccgctgagca atggaagcgg gtaggccttt ggggcagcgg ccaatagcag    1080

ctttgctcct tcgctttctg ggctcagagg ctgggaagca atggaagcgg gtaggccttt    1140

ggggcagcgg ccaatagcag ctttgctcct tcgctttctg ggctcagagg ctgggaaggg    1200

gtgggtccgg gggcgggctc aggggcgggc tcaggggcgg ggcgggcgcc cgaaggtcct    1260

ccggaggccc ggcattctgc acgcttcaaa agcgcacgtc tgccgcgctg ttctcctctt    1320

cctcatctcc gggcctttcg acctgcagca gcacgtgttg acaattaatc atcggcatag    1380

tatatcggca tagtataata cgacaaggtg aggaactaaa cc atg gga tcg gcc      1434
                                               Met Gly Ser Ala
                                               1

att gaa caa gat gga ttg cac gca ggt tct ccg gcc gct tgg gtg gag      1482
Ile Glu Gln Asp Gly Leu His Ala Gly Ser Pro Ala Ala Trp Val Glu
5                   10                  15                  20

agg cta ttc ggc tat gac tgg gca caa cag aca atc ggc tgc tct gat      1530

Arg Leu Phe Gly Tyr Asp Trp Ala Gln Gln Thr Ile Gly Cys Ser Asp
                25                  30                  35

gcc gcc gtg ttc cgg ctg tca gcg cag ggg cgc ccg gtt ctt ttt gtc      1578
Ala Ala Val Phe Arg Leu Ser Ala Gln Gly Arg Pro Val Leu Phe Val
            40                  45                  50

aag acc gac ctg tcc ggt gcc ctg aat gaa ctg cag gac gag gca gcg      1626
Lys Thr Asp Leu Ser Gly Ala Leu Asn Glu Leu Gln Asp Glu Ala Ala
        55                  60                  65

cgg cta tcg tgg ctg gcc acg acg ggc gtt cct tgc gca gct gtg ctc      1674
Arg Leu Ser Trp Leu Ala Thr Thr Gly Val Pro Cys Ala Ala Val Leu
    70                  75                  80

gac gtt gtc act gaa gcg gga agg gac tgg ctg cta ttg ggc gaa gtg      1722
Asp Val Val Thr Glu Ala Gly Arg Asp Trp Leu Leu Leu Gly Glu Val
85                  90                  95                  100

ccg ggg cag gat ctc ctg tca tct cac ctt gct cct gcc gag aaa gta      1770
Pro Gly Gln Asp Leu Leu Ser Ser His Leu Ala Pro Ala Glu Lys Val
                105                 110                 115

tcc atc atg gct gat gca atg cgg cgg ctg cat acg ctt gat ccg gct      1818
Ser Ile Met Ala Asp Ala Met Arg Arg Leu His Thr Leu Asp Pro Ala
            120                 125                 130

acc tgc cca ttc gac cac caa gcg aaa cat cgc atc gag cga gca cgt      1866
Thr Cys Pro Phe Asp His Gln Ala Lys His Arg Ile Glu Arg Ala Arg
        135                 140                 145

act cgg atg gaa gcc ggt ctt gtc gat cag gat gat ctg gac gaa gag      1914
Thr Arg Met Glu Ala Gly Leu Val Asp Gln Asp Asp Leu Asp Glu Glu
    150                 155                 160

cat cag ggg ctc gcg cca gcc gaa ctg ttc gcc agg ctc aag gcg cgc      1962
His Gln Gly Leu Ala Pro Ala Glu Leu Phe Ala Arg Leu Lys Ala Arg
165                 170                 175                 180

atg ccc gac ggc gag gat ctc gtc gtg acc cat ggc gat gcc tgc ttg      2010
Met Pro Asp Gly Glu Asp Leu Val Val Thr His Gly Asp Ala Cys Leu
                185                 190                 195

ccg aat atc atg gtg gaa aat ggc cgc ttt tct gga ttc atc gac tgt      2058
Pro Asn Ile Met Val Glu Asn Gly Arg Phe Ser Gly Phe Ile Asp Cys
            200                 205                 210

ggc cgg ctg ggt gtg gcg gac cgc tat cag gac ata gcg ttg gct acc      2106
Gly Arg Leu Gly Val Ala Asp Arg Tyr Gln Asp Ile Ala Leu Ala Thr
        215                 220                 225

cgt gat att gct gaa gag ctt ggc ggc gaa tgg gct gac cgc ttc ctc      2154
Arg Asp Ile Ala Glu Glu Leu Gly Gly Glu Trp Ala Asp Arg Phe Leu
    230                 235                 240

ctg ctt tac ggt atc ggc gct ccc gat tcg cag cgc atc gcc ttc tat      2202
Leu Leu Tyr Gly Ile Gly Ala Pro Asp Ser Gln Arg Ile Ala Phe Tyr
245                 250                 255                 260

cgc ctt ctt gac gag ttc ttc tga ataaacaata aagatgtcca ctaaaatgga      2256
Arg Leu Leu Asp Glu Phe Phe
                265

agtttttcct gtcatacttt gttaagaagg gtgagaacag agtacctaca ttttgaatgg      2316

aaggattgga gctacggggg tgggggtggg gtgggattag ataaatgcct gctcttttac      2376

tgaaggctct ttactattgc tttatgataa tgtttcatag ttggatatca taatttaaac      2436

aagcaaaacc aaattaaggg ccagctcatt cctcccactc atgatctata gatctataga 2496

tctctcgtgg gatcattgtt tttctcttga ttcccacttt gtggttctaa gtactgtggt 2556

ttccaaatgt gtcagtttca tagcctgaag aacgagatca gcagcctctg ttccacatac 2616

acttcattct cagtattgtt ttgcaagttc taattccatc agaagctgac tctagatcct 2676

gcaggaattc ccgcggtgcg ggtgccaggg cgtgcccttg ggctccccgg gcgcgtactc 2736

cacctcgaca agcttctcga gggttgagat gtgtataaga gacagctgtc acatgtcata 2796

tgcaattgtg atcagtgcac aacgttcgat cggcggccgc gagctc 2842

## Beschreibung

Die vorliegende Erfindung betrifft Nukleinsäurekonstrukte, die zur Mutagenese von Genen bzw. deren Allele bei Eukaryonten eingesetzt werden können, vorzugsweise zur Geninaktivierung. Diese Inaktivierung ist vorzugsweise konditional d.h. die Inaktivierung eines Zielgens kann in Zielzellen gesteuert ausgelöst werden. Alternativ kann ein betroffenes Gen zunächst inaktiviert und später konditional reaktiviert werden. Außerdem sieht die Erfindung vor den Aktivitätszustand von Genen mehrfach konditional zu ändern. Dabei bezieht sich die Erfindung auf die Inaktivierung der Genaktivität dadurch, dass ein Primärtranskript so verändert wird, dass dieses degradiert wird und aus diesem weniger oder im wesentlichen kein Translationsprodukt entsteht, so dass die Aktivität des Genprodukts reduziert bzw. eliminiert ist. Zur Bezeichnung der Wirkung der Reduktion oder Verhinderung der Synthese des natürlichen Genprodukts wird vorliegend der Ausdruck der Geninaktivierung verwendet. Dabei kann im Rahmen der Erfindung der Ausdruck "Gen" ein einzelnes Gen, z.B. in einem haploiden Genom, ein einzelnes Allel eines Gens in di- oder polyploiden Genomen, sowie mehrere oder alle in einem Genom vorliegenden Allele bezeichnen, z.B. wenn ein erfindungsgemäßes Allel in einem diploiden Organismus homozygot vorliegt.

Das Einbringen erfindungsgemäßer Nukleinsäurekonstrukte kann gezielt in Introns oder Exons bestimmter Gene (gezielte Mutagenese) oder zufällig in beliebige Gene erfolgen (zufällige oder ungerichtete Mutagenese).

Insbesondere betrifft die Erfindung die Erzeugung von Eukaryonten, beispielsweise Hefe, pflanzlichen und tierischen Zellen oder pflanzlichen oder tierischen Organismen, bei denen nach Insertion eines erfindungsgemäßen Nukleinsäurekonstrukts zumindest ein Gen inaktiviert oder inaktivierbar ist oder nach erfolgter Inaktivierung reaktivierbar ist. Dies betrifft insbesondere die Inaktivierung von Genen in nicht-menschlichen embryonalen Stammzellen (ES-Zellen), die zur Bildung eines kompletten Organismus fähig oder dazu beizutragen in der Lage sind und daraus erzeugte nicht-menschliche Tiere, z.B. nicht-menschliche Säuger, die erfindungsgemäß manipulierte Gene enthalten.

In einer ersten Ausführungsform stellt die Erfindung Nukleinsäurekonstrukte zur gezielten Mutagenese von Genen bereit, die mittels homologer Rekombination gezielt sequenzgenau in bestimmte Zielgene inserierbar sind.

Eine Variante dieser ersten Ausführungsform stellt die Erfindung Nukleinsäurekonstrukte und diese enthaltende Gene bereit, die zur vollständigen Geninaktivierung verwendet werden können

In einer zweiten Variante dieser ersten Ausführungsform sind die erfindungsgemäßen Nukleinsäurekonstrukte zur konditionalen Geninaktivierung verwendbar, so dass diese Nukleinsäurekonstrukte enthaltende Gene konditional inaktivierbar sind.

In Fortbildung der Erfindung können erfindungsgemäße Nukleinsäurekonstrukte in einer dritten Variante der ersten Ausführungsform außerdem zur konditionalen Reaktivierung zuvor inaktivierter eukaryontischer Gene eingesetzt werden.

Weiter sieht eine vierte Variante dieser Ausführungsform erfindungsgemäße Nukleinsäurekonstrukte vor, die einen mehrfachen konditionalen Aktivitätswechsel eines Gens ermöglichen.

Die erfindungsgemäßen Nukleinsäurekonstrukte zur vollständigen oder konditionalen Inaktivierung können auch zur Inaktivierung von Spleißvarianten eukaryontischer Gene, sowie zur Reaktivierung zuvor inaktivierter Spleißvarianten eingesetzt werden. Weiterhin können die erfindungsgemäßen Nukleinsäurekonstrukte zur Erzeugung komplett hypomorpher oder konditional hypomorpher Allele oder Mutationen bzw. hypomorpher phänotypischer Zellen sowie daraus erzeugter nicht-menschlicher Tiere eingesetzt werden, die konditional hypomorphe Allele aufweisen. In derartigen Organismen oder Zellen mit hypomorphem Phänotyp ist die Expression des Zielgens oder einer Spleißvariante des Zielgens um einen bestimmten Anteil reduziert, jedoch nicht vollständig inhibiert.

In einer zweiten Ausführungsform betrifft die Erfindung die Verwendung der Nukleinsäurekonstrukte als Genfallen für die ungerichtete Mutagenese, insbesondere in Verbindung mit der Erzeugung nicht-menschlicher Säuger, die komplett inaktivierte, konditional inaktivierbare oder zunächst inaktivierte, konditional reaktivierbare Gene haben oder Gene aufweisen, deren Aktivität mehrfach konditional geändert werden kann.

Bei der Verwendung als Genfalle sind erfindungsgemäßen Nukleinsäurekonstrukte auch dazu geeignet, dass Gene inaktiviert werden können, die in ES-Zellen nur schwach oder nicht exprimiert werden oder während der Embryogenese essentiell sind.

Bei der Verwendung der erfindungsgemäßen Nukleinsäurekonstrukte als Genfalle können diese in einer ersten Variante dieser Ausführungsform als Teil eines umfänglicheren Nukleinsäurekonstrukts eingesetzt werden, oder in einer zweiten Variante selbst direkt als Genfalle verwendet werden.

In einer dritten Ausführungsform betrifft die Erfindung die Verwendung erfindungsgemäßer Nukleinsäurekonstrukte als Bestandteile retroviraler Vektoren, die zur ungerichteten Mutagenese eukaryontischer Gene vorgesehen sind, insbesondere in Verbindung mit der Erzeugung nicht-menschlicher Säuger und anderer Tiere sowie Pflanzen, die komplett inaktivierte, konditional inaktivierbare oder zunächst inaktivierte, konditional reaktivierbare Gene haben oder Gene aufweisen, deren Aktivität mehrfach konditional geändert werden kann.

In einer vierten Ausführungsform sieht die Erfindung die Verwendung erfindungsgemäßer Nukleinsäurekonstrukte als Bestandteil umfänglicherer Nukleinsäurekonstrukte vor, die in Eukaryonten als Transposons verwendet werden können. Dabei ist erfindungsgemäß bevorzugt, entsprechende Nukleinsäurekonstrukte zur Erzeugung nicht-menschlicher Säuger zu verwenden, die komplett inaktivierte, konditional inaktivierbare oder zunächst inaktivierte, konditional reaktivierbare Gene haben.

In einer fünften Ausführungsform betrifft die vorliegende Erfindung die Verwendung von Nukleinsäurekonstrukten zur Gentherapie, mit denen eine gezielte Inaktivierung einzelner Gene bzw. von deren Allelen möglich wird. Dabei sind erfindungsgemäße Nukleinsäurekonstrukte bevorzugt zur Inaktivierung dominant wirkender, die Erkrankung verursachenden Allele in Patienten vorgesehen, die für heterozygot für dieses Allel sind.

In einer sechsten Ausführungsform sieht die Erfindung Nukleinsäurekonstrukte vor, die als prokaryontische Transposons wirken und bevorzugt als Bestandteil von Transposomen zur Herstellung von Banken umfänglicherer Nukleinsäurekonstrukte vorgesehen sind, die für die gezielte Mutagenese in Eukaryonten, vorzugsweise in murinen embryonalen Stammzellen (ES-Zellen) verwendet werden können.

Sowohl bei der Verwendung erfindungsgemäßer Nukleinsäurekonstrukte zur gezielten Mutagenese als auch bei der Verwendung entsprechender Konstrukte als Teile von Genfallen sowie Transposons und Transposomen ist es bevorzugt, dass die erfindungsgemäßen Nukleinsäurekonstrukte so gestaltet sind, dass sie mittels rekombinasevermittelter einfacher oder mehrfacher Inversion oder durch rekombinasevermittelte Deletion zur Inaktivierung bzw. Reaktivierung eines Zielgens einfach oder mehrfach geeignet sind.

### Stand der Technik

Im Stand der Technik kann eine Inaktivierung von Genen in Eukaryonten durch gezielte oder durch zufällige Mutagenese erzielt werden. Die gezielte Mutagenese beruht auf der homologen Rekombination eines Nukleinsäurekonstruktes in einem bestimmten Lokus. Von der gezielten Mutagenese sind Verfahren zu unterscheiden, bei denen die Inaktivierung eukaryontischer Gene auf der ungerichteten, also zufälligen Insertion von Nukleinsäurekonstrukten beruht. Zum einen handelt es sich dabei um Nukleinsäurekonstrukte, die in Zellen transfiziert werden und als sogenannte Genfallen wirken. Andererseits sind Verfahren bekannt, bei denen stabil in das Genom einer Zelle oder eines Organismus integrierte Nukleinsäurekonstrukte als Tranposons wirken, die in Gegenwart einer geeigneten Transposase mobilisiert werden und nach Integration in ein anderes Gen dieses inaktivieren.

Herkömmliche Konstrukte zur gezielten Mutagenese, die eine vollständige Geninaktivierung verursachen, herkömmliche Genfallen sowie Transposons sind dadurch nachteilig, dass betroffene Allele in nicht-menschlichen Säugern, z.B. Mäusen, die nach einer Behandlung mit solchen Konstrukten erzeugt wurden, sofort dauerhaft und vollständig inaktiviert sind. Wenn ein Zielgen für die Embryogenese essentiell ist, so führt seine Inaktivierung zumindest im homozygoten Zustand zu einem embryonal letalen Phänotyp. Daher ist es nicht möglich, mit bekannten Nukleinsäurekonstrukten vitale Mausmutanten zu erzeugen, bei denen ein für die Embryogenese essentielles Gen homozygot vollständig inaktiviert ist. In diesem Fall können Funktionen des Zielgens im adulten Organismus nicht untersucht werden. Außerdem besteht hier das Risiko, dass ein stark ausgeprägter Phänotyp in einem bestimmten Zelltyp schwächer ausgeprägte Phänotypen in anderen Zelltypen überdeckt, so dass letztere nicht erkannt werden können.

Deshalb wurden Methoden zur konditionalen Geninaktivierung entwickelt, mit denen ein Zielgen im adulten Organismus nur in einem bestimmten Zelltyp und/oder ab einem bestimmten Zeitpunkt inaktiviert vorliegt (Gu H et al. (1994): Deletion of a DNA polymerase beta gene segment in T cells using cell type-specific gene targeting. Science 265(5168):103-106).

Herkömmliche, auf gezielter Mutagenese basierende Verfahren zur konditionalen Geninaktivierung beruhen auf der Deletion ganzer Exons *in vivo,* insbesondere von solchen, die funktionale Domänen des Zielgens kodieren, und/oder dessen erstes Exon, das für die Initiation der Transkription erforderlich ist. Nachteilig dabei ist, dass es nach der Deletion zur Translation fehlerhafter Proteine kommen kann, die noch Restaktivität besitzen oder andere unvorhersehbare Effekte verursachen.

Alternativ werden Exons deletiert, deren flankierende Introns in unterschiedlichen Phasen vorliegen, d.h., dass sie ein Kodon jeweils an einer anderen Position unterbrechen. Dies hat zur Folge, dass das Spleißen nach erfolgter Deletion des Exons zu einer Verschiebung des Leserahmens führt und es somit nicht mehr zur Expression eines funktionalen Genproduktes kommt.

Zur Inaktivierung von Genen ist es daher bisher erforderlich, die Struktur, insbesondere die Intronphasen des betreffenden Gens zu kennen, da es bevorzugt nur dann zu einer vollständigen Inaktivierung durch Deletion eines Exons kommt, wenn das Leseraster der verbleibenden Exons zumindest teilweise verschoben wird. Dieses Verfahren ist dann nachteilig, wenn ein Exon zur Deletion vorgesehen wurde, dass dem alternativen Spleißen unterliegt und somit nicht Teil aller Transkripte des Gens ist, da dann ein Teil der Transkripte von der Deletion des Exons unbeeinflußt bleibt und von diesen weiterhin zumindest eine Form des Genproduktes kodiert wird.

Es ist bekannt, für das konditionale Inaktivieren von Genen in Eukaryonten Exons des Zielgens mit Erkennungssequenzen (loxP) der Cre-Rekombinase oder FRT-Sequenzen, für die die Flp-Rekombinase spezifisch ist, zu flankieren. Eine Übersicht findet sich in Branda und Dymecki (Branda CS und Dymecki SM (2004): Talking about a revolution: The impact of site-specific recombinases on genetic analyses in mice. Dev Cell 6(1):7-28). Branda und Dymecki stellen die Verwendung von Erkennungssequenzen für Rekombinase, z.B. loxP, in Verbindung mit der spezifischen aktiven Rekombinase, z.B. Cre vor. Dabei werden Zellen genetisch so manipuliert, dass sie mit loxP flankierte Exons enthalten, die anschließend *in vivo* deletiert werden, so dass maximal eine unvollständige mRNA generiert werden kann, die zu einem verkürzten oder abwesendem Translationsprodukt führen würde.

Nachteile der bekannten Verfahren zur gezielten Mutagenese eukaryontischer Gene, die auch in Branda und Dymecki (a.a.O.) beschrieben werden, liegen darin, dass ein hoher Kenntnisstand bezüglich des zu inaktivierenden Gens notwendig und die Klonierung entsprechender Konstrukte sehr aufwendig ist. Mit gängigen Methoden sind jeweils mehrere Klonierungsschritte zur Herstellung eines Targetingvektors notwendig. Zum Teil können diese Probleme umgangen werden indem die Strategie des sogenannten "targeted trapping" verwendet, bei der Teile von Nukleinsäurekonstrukten, die ursprünglich zur Verwendung als Genfallen (s.u.) *in vivo* vorgesehen waren, gezielt in klonierte genomische Fragmente eines Zielgens inseriert werden, die dann mittels homologer Rekombination in den betreffenden Lokus ins Genom einer murinen ES-Zelle inseriert werden. Nachteilig beim "targeted trapping" ist die Notwendigkeit der Berücksichtigung der Intronphase des Introns, in das ein entsprechendes Konstrukt eingebracht werden soll, so dass für jede der drei möglichen Intronphasen entsprechende Konstrukte bereitgestellt werden müssen. Das genannte Verfahren setzt bei herkömmlicher Klonierungsmethodik mittels Restriktionsverdau und Ligation außerdem das Vorhandensein geeigneter Restriktionsschnittstellen in dem zu verwendenden genomischen Fragment sowie in den verwendeten Nukleinsäurekonstrukten voraus. Die Abhängigkeit von geeigneten Restriktionsschnittstellen läßt sich durch Klonierung mittels homologer Rekombination in *E. coli* (ET-Klonierung, Recombineering) umgehen (Muyrers JP et al. (2001): Recombinogenic engineering - new options for cloning and manipulating DNA. Trends Biochem Sci 26(5):325-331), wobei wiederum ein Mindestmaß an Kenntnissen über die Sequenz des zu inaktivierenden Gens notwendig ist. Vor allem das Vorhandensein repetitver Sequenzen (z. B. B1-, B2- und LINE-Elementen sowie Abschnitte von Wiederholungen einzelner Di- oder Trinukleotide) in den zur Insertion vorgesehenen Sequenzen kann eine Klonierung mittels homologer Rekombination erheblich erschweren oder unmöglich machen, da die zur homologen Rekombination vorgesehenen Bereiche des zu inserierenden Konstruktes in diesem Fall homolog zu mehreren Zielsequenzen sind.

Als Alternative zu herkömmlichen Klonierungsverfahren für die Herstellung von Nukleinsäurekonstrukten für die gezielte Mutagenese von Genen in Eukaryonten wurden auf Transposition basierende Verfahren beschrieben. Dabei werden von Erkennungssequenzen für eine Transposase flankierte Kassetten in genomische Fragmente durch Wirkung der betreffenden Transposase inseriert. Zhang et al. (Zhang C et al (2005): Transposon-mediated generation of targeting vectors for the production of gene knockouts. Nucleic Acids Res 33 (3):e24), sowie Aoyama et al. (Aoyama M et al. (2005): Simple and straightforward construction of a mouse gene targeting vector using in vitro transposition reactions. Nucleic Acids Res 33(5):e52) beschreiben für die Herstellung konditionaler Mausmutanten mittels homolger Rekombination in ES-Zellen geeignete Nukeinsäurekonstrukte, die mittels Transposition in Bakterien erzeugt wurden. Nachteilig bei diesen Verfahren ist, dass zwei unabhängige Transposasereaktionen durchgeführt werden müssen, damit ein zu deletierender Bereich des Zielgens endständig von zur Deletion *in vivo* geeigneten Erkennungsstellen für eine Rekombinase flankiert ist. Da die Reaktionen ungerichtet erfolgen, müssen nach der ersten Reaktion zunächst Klone identifiziert werden, die für die zweite Reaktion in Frage kommen. Da die zweite Reaktion die für die Deletion des Bereiches des Gens notwendige zweite Erkennungsstelle für eine Rekombinase in einer geeigneten Position sowie in der korrekten Orientierung inserieren muß, ist auch nach der zweiten Reaktion eine aufwendige Analyse notwendig und zumindest die Hälfte der Reaktionsprodukte, die nämlich die zweite Erkennungsstelle in der falschen Orientierung aufweisen, ist nicht weiter zu gebrauchen. Außerdem können nur solche Konstrukte verwendet werden, bei denen die Deletion des mittels der Transposasereaktion mit Erkennungsstellen für eine Rekombinase flankierten Exons, vorhersagbar zu einer Inaktivierung des Zielgens führen.

Neben Konstrukten zur gezielten Geninaktivierung sind Nukleinsäurekonstrukte bekannt, die in embryonalen Stammzellen (ES-Zellen) als Genfalle einsetzbar sind. Da die Intronbereiche eukaryontischer Gene länger als die Exonbereiche sind, ist die Wahrscheinlichkeit dafür, den Intronbereich eines eukaryontischen Gens durch Insertion einer von außen zugeführten Nukleinsäure zu treffen, statistisch größer als die Wahrscheinlichkeit, eines der Exons zu treffen. Deshalb werden Genfallen so aufgebaut, dass sie nach Insertion in ein Intron eines Gens dieses inaktivieren, indem die Genfalle Teil des entsprechenden Primärtranskriptes und nach erfolgter Prozessierung desselben Teil des reifen Transkripts wird. Für die Wirksamkeit einer Genfalle notwendig ist außerdem, dass sie im reifen Transkript das Leseraster nicht unterbricht. Da die Phase, in der ein zufällig von einer Genfalle getroffenes Intron ein Kodon unterbricht, naturgemäß nicht bekannt ist, sind bei der Verwendung von Genfallen insgesamt drei Konstrukte zur Inaktivierung jedes möglichen Gens erforderlich, nämlich jeweils ein Konstrukt für jede mögliche Intronphase. Dementsprechend muß eine herkömmliche Genfalle außerdem in der korrekten Orientierung inseriert werden, um ihre Wirkung zu entfalten.

Bei der Verwendung bekannter Nukleinsäurekonstrukte, die aufgrund der Erzeugung eines Fusionsproteins, das von Teilen des Zielgens und dem in dem Nukleinsäurekonstrukt beinhalteten Reporter-/Selektionsmarkergen kodiert wird, als Genfalle wirken, wird das Zielgen häufig nicht vollständig inaktiviert, so dass die Mutagenese zu sogenannten hypomorphen Mutanten führt, die keinen eindeutigen Phänotyp aufweisen.

Weiterhin können mit der Mehrzahl der bekannten Vektoren, die als Genfalle eingesetzt werden, nur die Insertion in in ES-Zellen exprimierte Gene identifiziert werden, da die Expression solcher Nukleinsäuren von der Aktivität des endogenen Promotors des Zielgens abhängt. Sie können also nicht direkt zur Selektion transformierter Zellen eingesetzt werden, bei denen die Insertion in ein inaktives Gen stattgefunden hat, da diesen Nukleinsäurekonstrukten für die eigene vollständige Aktivität die Ergänzung durch zumindest ein aktives funktionales Element des eukaryontischen Zielgens fehlt.

Bekannte Genfallen haben außerdem den Nachteil, dass sie bestimmte Loci zur Insertion bevorzugen, während andere gar nicht oder mit so geringer Frequenz getroffen werden, dass sie in den entsprechenden Zellbanken nicht repräsentiert sind. Für eine Mutagenese solcher Gene bleibt deshalb nur die gezielte Mutagenese mittels homologer Rekombination. Die bekannten, als Genfalle für ES-Zellen vorgesehenen Nukleinsäurekonstrukte sind zunächst nicht für die gezielte Mutagenese eines bestimmten Gens geeignet, können aber bei der Verwendung als Teil von umfänglicheren Nukleinsäurekonstrukten zur gezielten Geninaktivierung ("targeted trapping") mittels homologer Rekombination verwendet werden (Skarnes WC, (2005): Two ways to trap a gene in mice. Proc Natl Acad Sei USA 102 (37):13001-13002)*.*

Weiterhin ist die Mehrzahl der bekanten als Genfallen vorgesehenen Nukleinsäurekonstrukte nicht zur Herstellung von Mutanten geeignet, in denen ein Gen konditional inaktiviert werden kann.

Xin et al. (Xin HB et al. (2005): Gene trap and gene inversion methods for conditional gene inactivation in the mouse. Nucleic Acids Res 33(2):e14) beschreiben ein als Genfalle geeignetes Nukleinsäurekonstrukt, das eine konditionale Inaktivierung eines eukaryontischen Zielgens durch Insertion einer Nukleinsäurekassette ermöglicht, die durch Inversion in Transkriptionsrichtung orientiert werden kann. Diese Kassette enthält einen Spleißakzeptor, eine IRES (interne Ribosomeneintrittsstelle), einen offenen Leserahmen für grün fluoreszierendes Protein (GFP) als Reportergen, sowie ein polyA-Signal. Das IRES-Element ermöglicht die Aktivität der Expressionskassette unabhängig vom Leseraster, führt jedoch in der Regel zu einer schwächeren Translation als ein herkömmliches Initiationskodon. Dieses System von Xin et al. enthält ein Resistenzgen mit eigenem Promotor, das bis auf ein fehlendes polyA-Signal eine vollständige Expressionskassette darstellt. Daher ist dieses Resistenzgen nur aktiv, wenn es in ein Intron stromaufwärts eines eukaryontischen polyA-Signals integriert ist. Die Insertion dieses Konstrukts inaktiviert jedoch nicht notwendigerweise das betroffene Allel, beispielsweise, wenn die Insertion zwischen zwei alternativ genutzten polyA-Sequenzen stattfindet. Außerdem kann, falls die Insertion des Nukleinsäurekonstrukts stromaufwärts von einem der letzten Exons des Zielgens erfolgt, von den entsprechenden bi-cistronischen Transkripten zusätzlich zu GFP, das vom zweiten Cistron kodiert wird, ein nahezu vollständiges funktionales Produkt des betroffenen Zielgens exprimiert werden. In diesen Fällen wäre daher keine bzw. keine vollständige Wirkung als Genfalle zu erwarten, d.h. das Zielgen würde nicht oder nur unvollständig inaktiviert

Schnütgen et al. (Schnütgen F et al. (2005): Genomewide production of multipurpose alleles for the functional analysis of the mouse genome. Proc Natl Acad Sci USA 102(20):7221-7226) beschreiben eine konditionale Genfalle, die in 5' von einem Selektionsmarkergen einen Spleißakzeptor enthält. In 3' vom Selektionsmarkergen folgt ein polyA-Signal. Die Kassette ist so von mutierten Erkennungssequenzen für zwei Rekombinasen flankiert, dass zum einen die Inversion der Kassette und, nach deren erfolgter Drehung, eine Deletion dieser Kassette möglich ist. Die Inaktivierung eines Zielgens erfolgt dabei nach Insertion in ein Intron des Gens, indem das Selektionsmarker-Exon im Leserahmen an das stromaufwärts gelegene Exon gespleißt wird. Das Zielgen liegt wegen der Ausführung als Genfalle also zunächst inaktiv vor und kann durch Einwirkung einer ersten Rekombinase in ein konditionales Allel umgewandelt werden, dass später durch die Wirkung der zweiten Rekombinase wieder inaktiviert werden kann. An diesem Konstrukt ist die Größe der Kassette von größer 2 kb nachteilig, sowie die Anzahl der notwendigen Erkennungssequenzen für Rekombinasen, die Mutationen in den so genannten Spacer-Sequenzen enthalten. Zur Verhinderung einer erneuten Inversion muss bei dieser Vorgehensweise in einer zweiten Reaktion eine der verbleibenden Erkennungssequenzen deletiert werden, wofür ein Mindestabstand zwischen den Erkennungssequenzen erforderlich ist. Ein wesentlicher Nachteil liegt hier wiederum darin, dass für jede der drei möglichen Intronphasen eines Zielgens ein geeignetes Nukleinsäurekonstrukt eingesetzt werden muss.

Chen et al. (Chen YT et al. (2004): Inducible gene trapping with drug-selectable markers and Cre/loxP to identify developmentally regulated genes. Mol Cell Biol 24(22):9930-9941) beschreiben ein zur Verwendung als Genfalle geeignetes Nukleinsäurekonstrukt, mit dem in ES-Zellen nicht exprimierte Gene identifiziert werden können. Dieses System beruht auf der Aktivierung eines positiv und negativ selektierbaren Selektionsmarkergens (puroΔTK), das durch eine loxP-flankierte Sequenz inaktiviert und stabil in das Genom integriert ist. Das Selektionsmarkergen steht unter der Kontrolle eines ubiquitär aktiven Lokus (rosa26). Die Aktivierung dieses Selektionsgens erfolgt durch die von Cre-Rekombinase vermittelte Deletion, die das inaktivierende Element der Genfalle darstellt und von einem Gen nach Insertion des Nukleinsäurekonstrukts exprimiert wird. In geeigneten Selektionsmedien kann gegen Transformanten selektiert werden, die Thymidinkinase exprimieren, d.h. gegen solche, die keine Cre-Rekombinase exprimieren. Die Differenzierung der überlebenden Zellen kann beispielsweise durch Retinsäure induziert werden. Anschließend können zum Beispiel durch Zugabe von Puromycin Klone selektiert werden, die im Lauf der Differenzierung Cre-Rekombinase translatiert haben und, aufgrund der Kopplung mit dem Gen der Cre-Rekombinase, das eukaryontische Zielgen.

Das von Chen et al. verwendete Nukleinsäurekonstrukt enthält anstelle eines herkömmlichen Selektionsmarkers den offenen Leserahmen für Cre-Rekombinase als eigentliche Genfalle, wobei deren Aktivierung zur Expression des Selektionsmarkers (puroΔTK) im rosa26-Lokus führt. An diesem Verfahren ist nachteilig, dass das als Genfalle eingesetzte Cre-Konstrukt nur in solchen Zellen seine Aktivität entfalten kann, die bereits die Expressionskassette für das Selektionsmarkergen enthalten. Entsprechende Zellen sind bisher nur von einem Mausstamm verfügbar, so dass für Arbeiten in einem anderen genetischen Hintergrund umfangreiche Rückkreuzungen von aus diesen Zellen gewonnenen Tieren notwendig sind.

Zusammenfassend weisen herkömmliche Nukleinsäurekonstrukte, die als Genfalle eingesetzt werden, in der Mehrzahl alle folgenden Nachteile auf: Es sind jeweils Konstrukte für alle drei möglichen Intronphasen und Leseraster erforderlich, um eine Inaktivierung jedes beliebigen Zielgens zu gewährleisten. Außerdem sind sie nur wirksam, wenn die Insertion in der geeigneten Orientierung erfolgt. Die Mehrzahl der bekannten Nukleinsäurekonstrukte, die als Genfalle eingesetzt werden, ist überdies auf die Aktivität des Zielgens angewiesen, um einen phänotypisch nachweisbaren Effekt zu erzielen. Ein weiterer Nachteil herkömmlicher Konstrukte liegt darin, dass mit der zum Nachweis des Integrationsortes verwendeten RT-PCR nur das Intron identifiziert werden kann, in dem die Integration erfolgte, ohne dass der genaue Ort innerhalb des betreffenden Introns sequenzgenau ermittelt werden kann. Daneben ist nachteilig, dass die Mehrheit der bekannten, als Genfalle einsetzbaren Nukleinsäurekonstrukte nicht zur konditionalen Geninaktivierung geeignet ist.

Neben transfektionsbasierten Genfallen wurden auch Nukleinsäurekonstrukte auf Basis retroviraler Vektoren zur Geninaktivierung entwickelt (von Melchner et al. (1989): Identification of cellular promoters by using a retrovirus promoter trap. J Virol 63(8):3227-3233). Diese bestehen im Prinzip aus Genfallen, die von "long terminal repeats" (LTRs, langen endständigen Wiederholungseinheiten) und weiteren viralen Elementen flankiert sind, die zur Verpackung einer entsprechenden RNA, für die reverse Transkription derselben in der Zielzelle und für die Insertion der resultierenden DNA in das Genom der Zielzelle notwendig sind. Retrovirale Vektoren besitzen, da sie auf Genfallen basieren, die gleichen Vor- und Nachteile (Stanford WL et al. (2001): Gene-trap mutagenesis: past, present and beyond. Nat Rev Genet 2(10):756-68.), bieten aber gegenüber herkömmlichen Gefallen den Vorteil, dass sie bevorzugt in 5' gelegene Bereiche von Genen inserieren und daher eher zu einer vollständigen Inaktivierung des Zielgens führen als Genfallen. Außerdem werden mit retroviralen Vektoren seltener Doppel- oder Mehrfachinsertionen beobachtet als mit Genfallen. Nachteilig können retrovirale Vektoren sein, wenn sie regulatorische Elemente beinhalten, die die Expression des Zielgens oder benachbarter Loci beeinflußt.

Neben der gezielten Mutagenese und dem Einsatz von Gefallen und retroviralen Vektoren können eukaryontische Gene auch *in vivo* mittels Transposition geeigneter Kassetten inaktiviert werden (Bestor TH (2005): Transposons reanimated in mice. Cell 122(3):322-325). Dabei handelt es sich um synthetische Transposons (z.B. sleeping beauty und Piggybac (Bestor TH (2005): Transposons reanimated in mice. Cell 122 (3) : 322-325) sowie frog prince (Miskey C (2003): The Frog Prince: a reconstructed transposon from Rana pipiens with high transpositional activity in vertebrate cells. Nucleic Acids Res 31 (23):6873-81.)), bei denen ein einer Genfalle entsprechender Anteil von Erkennungssequenzen für eine Transposase flankiert wird. Entsprechende Konstrukte werden zunächst stabil im Genom des Zielorganismus inseriert. Später wird das im Genom befindliche Transposon durch Expression der geeigneten Transposase mobilisiert, z.B. durch Verpaarung mit einer Mauslinie, die die entsprechende Transposase exprimiert. In der Folge inseriert das Transposon in einer anderen Position des Genoms. Erfolgt die Insertion in einem Gen, wird dieses inaktiviert, sofern die Insertion in der geeigneten Orientierung erfolgt.
Die Insertion eines mobilisierten Transposons erfolgt dabei bevorzugt auf dem gleichen Chromosom in der Nähe des Locus, in dem sich das Transposon ursprünglich befand. Dies kann nachteilig sein, z.B. wenn die Mutagenese aller Gene eines Organismus angestrebt wird, bietet aber Vorteile, wenn mehrere benachbarte Gene untersucht werden müssen, z.B. in Fällen, in denen ein bestimmter Phänotyp genetisch einer bestimmten Chromosomenregion zugeordnet werden kann, eine Identifizierung des den Phänotyp verursachenden Gens aber mit genetischen Methoden nicht möglich ist. Ein wesentlicher Nachteil bei der Verwendung von Transposons liegt darin, dass bisher keine Nukleinsäurekassetten zur Verwendung in Transposons beschrieben wurden, die eine konditionale Mutagenese ermöglichen würden, so dass bisher damit nur Gene untersucht werden können, deren Inaktivierung nicht zu einem embryonal letalen Phänotyp führt. Vorteilhaft sind Transposons zur Geninaktivierung, da entsprechende Systeme auch für andere Organismen als die Maus zur Verfügung stehen, z.B. für die Ratte (Kitada K (2007): Transposon-tagged mutagenesis in the rat. Nat Methods 4(2):131-133).

### Aufgabe der Erfindung

Es ist eine Aufgabe der vorliegenden Erfindung, die Nachteile des Standes der Technik zu überwinden. Für diesen Zweck sind erfindungsgemäße Nukleinsäurekonstrukte und Verfahren zur Verfügung zu stellen, mit denen Gene in eukaryontischen Zellen vollständig oder vorzugsweise konditional inaktivierbar sind, wobei die Inaktivierung des Zielgens vollständig oder anteilig ist, so dass das Zielgen bzw. ein alternatives Spleißprodukt in den Zielzellen vollständig inaktiv oder nur teilweise inaktiv ist, was zu einem vollständigen oder hypomorphen Phänotyp führt. Zielzellen sind beispielsweise Hefe-, pflanzliche und tierische Zellen.

Neben Nukleinsäurekonstrukten zur Inaktivierung, bevorzugt zur konditionalen Inaktivierung, strebt die Erfindung Nukleinsäurekonstrukte an, die eine konditionale Reaktivierung von zuvor inaktivierten Zielgenen ermöglichen, sowie Nukleinsäurekonstrukte, die einen mehrfachen Aktivitätswechsel eines Gens erlauben.
Die Erfindung sieht Nukleinsäurekassetten als Bestandteil von Nukleinsäurekonstrukten zur gezielten Mutagenese mittels homologer Rekombination vor.

Dabei sieht die Erfindung vor, dass erfindungsgemäße Nukleinsäurekassetten für die Herstellung von umfänglicheren Konstrukten für die gezielte Mutagenese mittels konventioneller Klonierungsstrategien durch Restriktion und Ligation, mittels Klonierung durch homologe Rekombination in Bakterien sowie mittels Transposition *in vivo* und *in vitro* anwendbar sind.

Es ist außerdem Aufgabe der Erfindung, für die ungerichtete Mutagenese als Genfalle verwendbare Nukleinsäurekonstrukte sowie Nukleinsäurekonstrukte auf Basis retroviraler Vektoren oder in Form von Transposons bereitzustellen, wobei die Nukleinsäurekonstrukte vorzugsweise auch geeignet sind, für die Embryonalentwicklung essentielle Gene in einem Organismus zu inaktivieren, der die Embryogenese durchlaufen hat, indem diese Gene konditional inaktivierbar sind. Weiter wird bevorzugt, dass die für die ungerichtete Mutagenese vorgesehenen Nukleinsäurekonstrukte geeignet sind, zuvor inaktivierte Gene zu reaktivieren oder einen mehrfachen konditionalen Aktivitätswechsel betroffener Gene ermöglichen.

Eine weitere Aufgabe der Erfindung ist es, Nukleinsäurekonstrukte und Verfahren bereitzustellen, die es ermöglichen Bibliotheken von Nukleinsäurekonstrukten zu erzeugen, die für die gezielte Mutagenese eukaryontischer Gene geeignet sind. Entsprechende Bibliotheken sollen genomische Subfragmente enthalten und möglichst alle im Genom enthaltenen Gene repräsentieren, so dass diese zur Herstellung komplett inaktivierter, konditional inaktivierbarer sowie konditional reaktivierbarer Allele oder Allele mit mehrfach konditional veränderbarem Aktivitätszustand genutzt werden können.

Weiterhin ist es Aufgabe der vorliegenden Erfindung, eukaryontische Zellen *ex vivo* oder nicht-menschliche Organismen bereitzustellen, die inaktivierte, konditional inaktivierbare oder inaktivierte und konditional reaktivierbare Gene aufweisen oder Gene beinhalten, deren Aktivitätszustand konditional mehrfach geändert werden kann, wie beispielsweise Versuchstiere. Derartige Versuchstiere sind zum Beispiel Nagetiere, Mäuse und Ratten, oder Schweine, Schafe oder Kühe. Darüber hinaus ist eine Verwendung erfindungsgemäßer Nukleinsäurekonstrukte in anderen nicht-menschlichen Eukaryonten vorgesehen, z. B. in Fischen, aber auch z.B. in Pflanzen.

Hinsichtlich einer weiteren Ausführungsform der Erfindung ist es das Ziel, Nukleinsäurekonstrukte und Verfahren bereitzustellen, mit dem Zielgene in im wesentlichen jeder nicht menschlichen embryonalen Stamm- (ES-) Zelle inaktiviert werden können, insbesondere auch Zielgene für solche Phänotypen, die nur vor einem bestimmten genetischen Hintergrund ausgeprägt werden.

Es ist ein vorrangiges Ziel der vorliegenden Erfindung, Nukleinsäurekonstrukte und Verfahren bereitzustellen, mit denen aus ES-Zellen direkt Mäuse oder andere Versuchstiere erzeugt werden können, die ein konditional inaktivierbares, ein inaktiviertes, reaktivierbares Allel tragen oder ein Allel beinhalten, dessen Aktivitätszustand einfach oder mehrfach geändert werden kann.

In einer noch weiteren Ausführungsform hat die Erfindung zum Ziel, Nukleinsäurekonstrukte und Verfahren bereitzustellen, mit denen eukaryontische Zellen gentherapiert werden können, indem in heterozygoten Zellen ein dominant wirkendes mutiertes Allel selektiv inaktiviert ist. Entsprechend sollen auch tierische, beispielsweise menschliche kultivierte Zellen *ex vivo* bereitgestellt werden, beispielsweise angezogene Primärkulturen, die aus einer Biopsie erhältlich sind, in denen ein bestimmtes mutiertes Allel eines Zielgens inaktiviert ist. Derartige Zellen mit inaktiviertem Zielgen können zur (Re-) Transplantation auf den Spender eingesetzt werden.

### Allgemeine Beschreibung der Erfindung

Die Erfindung stellt Nukleinsäurekonstrukte, diese enthaltende Zellen und nicht-menschliche Organismen, wie Tiere und Pflanzen, sowie Verfahren bereit, die die vorgenannten Aufgaben lösen.

Die Erfindung beruht auf der Beobachtung, dass zumindest mRNA im Wesentlichen nur dann von einer eukaryontischen Zelle synthetisiert wird, wenn ihre prä-mRNA korrekt prozessierbar ist. Die korrekte Prozessierung von prä-mRNA voraus, dass diese keine prämaturen Stopkodons enthält, die innerhalb des offenen Leserahmens der prä-mRNA innerhalb nicht-terminaler Exons angeordnet sind. Derzeit wird angenommen, dass ein zellulärer Korrekturmechanismus während einer vermuteten ersten Runde der Translation, die auch als Pioniertranslation bezeichnet wird, dafür verantwortlich ist, dass derartige prämature Stopkodons die Degradation des Transkripts auslösen. Dieses Phänomen wird als Nonsense vermittelte RNA-Degradation oder Nonsense mediierte RNA-Degradation (NMD) bezeichnet. In Vertebraten wird NMD ausgelöst, wenn ein Stopkodon innerhalb eines Exons mehr als 50 Nukleotide stromaufwärts der Spleißdonorstelle des betreffenden Exons lokalisiert ist. Bekannte Ausnahmen von dieser Regel sind bisher nur Immunglobulingene und T-Zellrezeptorgene, bei denen ein wesentlich geringerer Abstand (8 bis 10 bp oder kleiner) zwischen einem prämaturen Stopkodon und der stromabwärts folgenden Spleißdonorstelle des Exons zur Auslösung von NMD ausreicht (Chan WK et al. (2007): An alternative branch of the nonsense-mediated decay pathway. EMBO J26(7):1820-1830*).*

Die Detektion prämaturer Stopkodons erfolgt jedoch nicht in allen Eukaryonten nach der beschriebenen Regel, wonach ein mehr als 50 bp stromaufwärts von einer Spleißdonorstelle in einem nicht-terminalen Exon lokalisiertes Stopkodon als prämatures Stopkodon erkannt wird. Beispielsweise erfolgt die Detektion in Hefe nach dem Spleißen über den Abstand eines Stopkodons zur poly-Adenylierungsstelle des Transkripts. Im Ergebnis führt ein prämatures Stopkodon in einem nicht-terminalen Exon aber auch hier zur NMD, da dies einen als NMD auslösendes Signal wirkenden Abstand zur poly-Adenylierungsstelle aufweist.

Natürliche Substrate für NMD sind prä-mRNAs, die von entsprechend mutierten Allelen synthetisiert werden, sowie inkorrekte Spleißprodukte, die ein entsprechend lokalisiertes prämatures Stopkodon innerhalb eines nicht-terminal angeordneten Exons im offenen Leserahmen enthalten.

Alle Ausführungsformen der Erfindung beruhen auf der Insertion eines NMD vermittelnden Exons zusammen mit für die Funktionalität des Exons essentiellen flankierenden Intronsequenzen in ein eukaryontisches Gen. Diese Insertion kann in ein Intron erfolgen, das dadurch in zwei Introns geteilt wird. Alternativ kann die Insertion in ein Exon erfolgen, z.B. bei Genen mit nur einem Exon, indem das NMD vermittelnde Exon mit kompletten Introns flankiert ist. Im Ergebnis wird das Exon des Zielgens in zwei Exons geteilt, das Zielgen besteht nach dieser Manipulation also aus drei Exons.

Bei allen Ausführungsformen ist optional vorgesehen, das NMD vermittelnde Exon mit in den angrenzenden Intronsequenzen lokalisierten Erkennungssequenzen für mindestens eine Rekombinase zu flankieren, die eine Invertierung oder eine Deletion der zwischen den Erkennungssequenzen lokalisierten Sequenzabschnitte mittels der betreffenden Rekombinase ermöglichen und so eine konditionale Regulation der Aktivität des Zielgens ermöglichen. Für die Insertion erfindungsgemäßer Nukleinsäurekonstrukte in Zielgene sind Ausführungsformen für die gezielte Insertion in ein bestimmtes Gen mittels homologer Rekombination sowie Ausführungsformen vorgesehen, die auf zufälliger Insertion erfindungsgemäßer Nukleinsäurekonstrukte in beliebige Gene beruhen. Für die zufällige Insertion werden erfindungsgemäße Nukleinsäurekonstrukte als Genfallen, retrovirale Vektoren oder Transposons ausgeführt. Im Ergebnis führen alle Ausführungsformen dazu, dass das NMD vermittelnde Exon zwischen zwei Exons eines Gens zu liegen kommt, unabhängig davon, ob es sich um zwei natürliche Exons handelt, oder ob die beiden flankierenden Exons des Gens durch Insertion eines erfindungsgemäßen Nukleinsäurekonstrukts in ein endogenes Exon erzeugt wurden. Mit den flankierenden Exons ist das NMD vermittelnde Exon über funktionale Introns verbunden, oder dieser Zustand kann mittels der Wirkung einer Rekombinase herstellt oder beendet werden.
In der bevorzugten Ausführungsform werden Nukleinsäurekonstrukte bereitgestellt, die gezielt mittels homologer Rekombination in ein Intron eines Zielgens integrierbar sind und daher für die Inaktivierung mindestens ein Intron enthaltender Gene, z.B. solche eukaryontischer Zellen bzw. Organismen geeignet sind. In einer ersten Variante dieser Ausführungsform weist das Nukleinsäurekonstrukt ein Exon mit mindestens einem im Leserahmen des Zielgens befindlichen prämaturen Stopkodon sowie angrenzende Intronabschnitte auf, die zusammen mit den stromaufwärts und stromabwärts des Insertionspunktes gelegenen Intronsequenzen des Zielgens komplette, funktionale Introns bilden, so dass das prämature Stopkodon des zusätzlich in das Zielgen eingeführten Exons beim Spleißen des Primärtranskriptes erkannt wird und das Transkript der NMD zugeführt wird. Entsprechend können die Intronsequenzen in 5' bzw. 3' des Exons Abschnitte sein, denen in 5' bzw. 3' die funktionalen Elemente vollständiger Introns fehlen, die für das Spleißen notwendig sind. Diese fehlenden funktionalen Elemente können erfindungsgemäß von Abschnitten des Gens, in das das Konstrukt integriert, bereitgestellt werden, und die Intronabschnitte des Konstrukts zu funktionalen Introns ergänzen.

In Fortbildung dieser Ausführungsform stellt die Erfindung Nukleinsäurekonstrukte bereit, die zur gezielten Insertion mittels homologer Rekombination in ein Exon eines Zielgens geeignet sind. Dies ist z.B. für intronfreie Zielgene vorteilhaft. Entsprechende Konstrukte weisen ein von vollständigen Introns flankiertes Exon mit mindestens einem prämaturen Stopkodon auf. Dabei wird der Insertionspunkt in ein Exon des Zielgens so gewählt, dass die intronischen Anteile der Spleißdonorstelle am 5'-Ende sowie die intronischen Anteile der Spleißakzeptorstelle am 3'-Ende des Konstruktes zusammen mit den Exonsequenzen stromaufwärts und stromabwärts des Insertionspunktes funktionale Spleißstellen ergeben. Dadurch wird ein ursprüngliches Exon des Zielgens in zwei funktionale Exons geteilt und das von Introns flankierte, mindestens ein prämatures Stopkodon enthaltende mutierte Exon als zusätzliches Exon eingeführt.

In der ersten Ausführungsform ist das unmittelbare Ergebnis des in dem eingeführten Exon enthaltenen prämaturen Stopkodons die NMD des primären Transkripts und somit die Inaktivierung des Gens, in das ein erfindungsgemäßes Nukleinsäurekonstrukt eingeführt wurde.

In einer zweiten, dritten und vierten Variante der ersten Ausführungsform stellt die Erfindung Nukleinsäurekonstrukte bereit, die zur konditionalen Änderung des Aktivitätszustandes von Genen geeignet sind. Die Konditionalität wird dadurch hergestellt, dass das Exon, das das prämature Stopkodon enthält und von Intronabschnitten flankiert ist, von mindestens einem Paar Erkennungssequenzen für Rekombinase umfasst ist. Die Wechselwirkung mit der spezifischen Rekombinase führt durch die Inversion des eingeführten Exons zu dessen Anordnung im anderen Strang eines betroffenen DNA-Moleküls. Optional können die Erkennungssequenzen auch zur Deletion dazwischen angeordneter Sequenzen benutzt werden.

In der zweiten Variante der ersten Ausführungsform liegt das erfindungsgemäße, die NMD vermittelnde Exon zunächst im nicht kodogenen Strang im Intron eines Gens, so dass dieses normal exprimiert wird. Die Inversion führt dann zur Anordnung des Exons im kodogenen DNA-Strang, so dass das prämature Stopkodon zur NMD der Transkripte des betroffenen Gens führt.

In der dritten Variante der ersten Ausführungsform liegt das erfindungsgemäße Exon zunächst im kodogenen Strang im Intron eines Gens. Dabei führt das prämature Stopkodon in dem erfindungsgemäßen Exon führt zur NMD der Transkripte des betroffenen Gens. Die Inversion führt dann zur Anordnung des Exons im nicht kodogenen DNA-Strang, so dass die Transkripte des betroffenen Gens das NMD vermittelnde Signal nicht mehr enthalten und normal exprimiert werden. Es ist außerdem erfindungsgemäß vorgesehen, diese Reaktivierung eines Gens statt durch die Inversion einer NMD-Kassette und Überführung der NMD-vermittelnden Signale in den nicht kodogenen Strang alternativ durch rekombinasevermittelte Deletion der NMD-Kassette zu bewirken.

In der vierten Variante der ersten Ausführungsform sieht die Erfindung Nukleinsäurekonstrukte vor, die von Erkennungssequenzen unterschiedlicher Rekombinasen flankiert sind und mehrfache Inversionen oder zunächst eine oder mehrere Inversionen und eine anschließende Deletion der NMD-Kassetten ermöglichen. Dies erlaubt die gezielte Herstellung von Mutanten, in denen der Aktivitätszustand eines Zielgens mehrfach veränderbar ist.

In einer zweiten Ausführungsform betrifft die Erfindung Nukleinsäurekonstrukte, die als Genfallen einsetzbar sind. Dabei bieten die erfindungsgemäßen, als Genfalle einsetzbaren Nukleinsäurekonstrukte den Vorteil, dass sie unabhängig von homologer Rekombination in das Wirtsgenom integriert werden und daher zur Inaktivierung vieler verschiedener Gene geeignet sind. Bei der Verwendung eines erfindungsgemäßen Nukleinsäurekonstrukts als Genfalle werden wiederum dessen Eigenschaften genutzt, dass Gene, in die es integriert ist, konditional inaktivierbar oder konditional reaktivierbar sind, oder der Aktivitätszustand eines betroffenen Gens mehrfach geändert werden kann. Die Möglichkeit zur Änderung des Aktivitätszustandes eines betroffenen Gens bietet darüber hinaus gegenüber herkömmlichen Verfahren den Vorteil, dass ein erfindungsgemäßes Nukleinsäurekonstrukt, das zunächst in der nicht wirksamen Orientierung in ein Gen inseriert wurde, nachträglich in die wirksame Orientierung überführt werden kann. Ein weiterer wesentlicher Vorteil erfindungsgemäßer Nukleinsäurekonstrukte gegenüber herkömmlichen Genfallen liegt darin, dass sie unabhängig von der Intronphase wirken, so dass nur ein Konstrukt statt dreier Konstrukte für die drei möglichen Intronphasen bei herkömmlichen Verfahren benötigt wird.

Allgemein wirken bekannte Genfallen so, dass ein nicht funktionales Nukleinsäurekonstrukt in eine Zielzelle eingebracht wird, das durch die Insertion in einen geeigneten Genort komplementiert wird, so dass erst die Integration in das Gen einer Zielzelle in der richtigen Orientierung und Position zur Komplementierung der fehlenden Funktion führt. Die Komplementierung mit einem funktionalen Element des Zielgenoms führt zur Herstellung der Aktivität des Genfallenkonstrukts, beispielsweise zur Expression eines Resistenzmarker- oder Reportergens. So fehlt beispielsweise als Promotor-Fallen verwendeten Konstrukten ein entsprechender Promotorbereich, der durch die zufällige Insertion des Konstrukts hinter einen genomischen Promotor komplementiert wird, so dass das inserierte Reporter- oder Resistenzmarkergen exprimiert werden kann. Entsprechend umfassen Genfallen der Erfindung auch Nukleinsäurekonstrukte, denen zumindest ein funktionales Element zur nachweisbaren Aktivität fehlt. Solche funktionalen Elemente können z.B. Promotoren, Enhancer oder Polyadenylierungssequenzen sein.

In einer ersten Variante der Ausführungsform erfindungsgemäßer Nukleinsäurekonstrukte als Genfalle wirkt als Genfalle zunächst ein Reportergen/Selektionsmarkergen, das später deletiert werden kann, so dass die NMD-Kassette die inaktivierende Funktion übernehmen kann. Dabei ist vorgesehen, dass ein Reportergen und/oder Selektionsmarkergen Teil eines Exons ist, das an seinem 5'-Ende von einem Spleißakzeptor und an seinem 3'-Ende von einem Polyadenylierungssignal begrenzt wird und somit als terminales Exon wirkt. Die Erfindung sieht vor, dass das beschriebene Exon von gleich orientierten Erkennungsequenzen für eine Rekombinase flankiert wird, so dass *es in vivo* deletiert werden kann. Bei dieser Ausführungsvariante der Erfindung liegt eine NMD-Kassette stromabwärts der 3' gelegenen, zur Deletion des Reportergens/Selektionsmarkergens vorgesehenen Rekombinaseerkennungssequenz. In der einfachsten Ausführung handelt es sich bei der NMD-Kassette um eine minimale NMD-Kassette ohne flankierende Rekombinaseerkennungssequenzen, wobei das NMD-vermittelnde Exon sich im gleichen Strang wie die kodierende Sequenz des Reportergens befindet. Dies bietet den erfindungsgemäßen Vorteil, dass von der durch das Reportergen/Resistenzgen vermittelten Inaktivierung des Zielgens, die auf einer Fusionierung des Reportergens/Resistenzmarkergens mit Teilen des offenen Leserahmens des Zielgens und auf der Expression eines entsprechenden Fusionsproteins beruht und aufgrund möglicher Restaktivität der von dem Zielgen kodierten Anteile nicht vollständig sein muß, auf eine verläßliche, durch die NMD-Kassette vermittelte Inaktivierung des Zielgens umgeschaltet werden kann. Dies ist vorteilhaft, da die Inaktivierung auf einer Degradation der Transkripte beruht, so dass aufgrund fehlender Translation kein Genprodukt des Zielgens synthetisiert werden kann.

In Fortbildung dieser Ausführungsform sieht die Erfindung vor, die NMD-Kassette mit Erkennunggssequenzen für eine weitere Rekombinasen zu flankieren, die eine irreversible Inversion der NMD-Kassette ermöglichen. Abhängig von der ursprünglichen Orientierung der NMD-Kassette in einem entsprechenden Nukleinsäurekonstrukt wird es dadurch möglich, ein ursprünglich durch ein Reportergen/Resistenzmarkergen inaktiviertes Zielgen durch die Deletion des Reportergens/Resistenzmarkergens in ein konditional inaktivierbares oder konditional aktivierbares Allel umzuwandeln.

In einer weiteren Fortbildung dieser Ausführungsform ist erfindungsgemäß vorgesehen, die NMD-Kassette mit Erkennungssequenzen für mehrere Rekombinasen zu flankieren, so dass die Kassette mehrfach invertiert werden kann. Dabei ist vorgesehen, dass die durch eine Rekombinase vermittelte Inversion jeweils irreversibel erfolgt. Diese Möglichkeit zur mehrfachen Inversion der NMD-Kassette bietet den erfindungsgemäßen Vorteil, dass ein ursprünglich durch ein Reportergen/Resistenzmarkergen inaktiviertes Zielgen unabhängig von der ursprünglichen Orientierung der NMD-Kassette in einem entsprechenden Nukleinsäurekonstrukt nach Deletion des Reportergens/Resistenzmarkergens in ein konditional inaktivierbares oder ein konditional aktivierbares Allel umgewandelt werden kann.

In der beschriebenen ersten Variante der Ausführungsform erfindungsgemäßer Nukleinsäurekonstrukte zur Verwendung als Genfalle wirkt zunächst ein Reportergen und/oder Selektionsmarkergen als Genfalle, das im offenen Leserahmen eines Zielgens fusioniert werden muß, so dass es zur Expression des Reportergens und/oder Selektionsmarkergens kommt. Es ist deshalb notwendig und erfindungsgemäß vorgesehen, das ein Reportergen und/oder Resistenzmarkergen beinhaltendes Exon in drei unabhängigen Nukleinsäurekonstrukten in jeweils einer der drei möglichen Intronphasen auszuführen. Die Bestimmung des Integrationsortes von Konstrukten, bei denen zunächst ein Reportergen/Selektionsmarkergen als Genfalle wirkt, kann mittels RT-PCR erfolgen.

In einer zweiten Variante der Ausführungsform erfindungsgemäßer Nukleinsäurekonstrukte als Genfalle wirkt die NMD-Kassette selbst als Genfalle, wenn sie in ein Intron eines Gens inseriert wurde und sich im kodogenen Strang des Gens befindet. Das erfindungsgemäß bevorzugte Vorhandensein von Stopkodons in allen drei Leserastern des in der NMD-Kassette beinhalteten Exons bietet gegenüber herkömmlichen Genfallen den Vorteil, dass wegen der Unabhängigkeit von Intronphasen nicht drei unabhängige Konstrukte sondern nur ein universell einsetzbares Konstrukt benötigt wird.

Dabei ist erfindungsgemäß bevorzugt, die NMD-Kassette in entsprechenden Nukleinsäurekonstrukten mit Erkennungssequenzen für mindestens eine Rekombinase zu flankieren, die eine einmalige irreversible Inversion der NMD-Kassette ermöglichen. Alternativ kann die NMD-Kassette mit Erkennungssequenzen für mehrere Rekombinasen flankiert werden, die jeweils eine einmalige, d.h. durch die gleiche Rekombinase nicht reversible Inversion der NMD-Kassette ermöglichen. Diese Flankierung mit Erkennungssequenzen für eine oder mehrere Rekombinasen bietet den erfindungsgemäßen Vorteil, dass die Orientierung der NMD-Kassette nach der Insertion ins Genom geändert werden kann, so dass zunächst unwirksame NMD-Kassetten nachträglich in die wirksame Orientierung gebracht werden können, bzw. bei der Verwendung von Erkennungssequenzen mehrerer Rekombinasen der Aktivitätszustand eines getroffenen Allels einfach oder mehrfach geändert werden kann. Deshalb sieht die Erfindung vor, entsprechende Nukleinsäurekonstrukte zur Herstellung von inaktivierten Allelen, konditional inaktivierbaren und konditional reaktivierbaren Allelen verwenden.

Bei der Ausführung erfindungsgemäßer Nukleinsäurekonstrukte, bei denen die NMD-Kassette selbst als Genfalle wirkt, ist weiterhin vorgesehen, die NMD-Kassette mit einem Selektionsmarkergen zu verbinden. Dabei sind solche Selektionsmarkergene bevorzugt, für die sowohl in Eukaryonten als auch in Prokaryonten selektiert werden kann, z.B. das Kanamycinresistenzgen. Damit eine Selektion sowohl in Eukaryonten als auch in Prokaryonten erfolgen kann, sieht die Erfindung vorzugsweise vor, das Selektionsmarkergen mit einem prokaryontischen und einen eukaryontischen Promotor sowie mit einem eukaryontischen Polyadenylierungssignal und einem prokaryontischen Terminationssignal zu versehen. Außerdem ist eine Verbindung des Selektionsmarkergens mit einem prokaryontischen Replikationsursprung vorgesehen, so dass DNA-Fragmente, die die NMD-Kassette und das Selektionsmarkergen nach Zirkularisierung in geeigneten Prokaryonten, z.B. *E. coli* K12 Stämmen, vermehrt (plasmid rescue) und zur Identifizierung des jeweiligen Insertionspunktes mittels Sequenzierung genutzt werden können.

Da erfindungsgemäße, als Genfallen ausgeführte Nukleinsäurekonstrukte bevorzugt so ausgeführt sind, dass die Inaktivierung des Zielgens allein in Abhängigkeit von der Orientierung der NMD-Kassette erfolgt, die mittels geeigneter Rekombinasen verändert werden kann, sieht die Erfindung vor, mit diesen Genfallen auch Gene konditional zu inaktivieren, deren Aktivität für die Embryogenese essentiell ist und die daher mit herkömmlichen Genfallen nicht untersucht werden können.

In der dritten Ausführungsform betrifft die Erfindung Nukleinsäurekonstrukte für den retroviralen Gentransfer. Dafür sieht die Erfindung vor, eine bevorzugt zweifach invertierbare NMD-Kassette, die mit einem von Erkennungssequenzen für eine Rekombinase flankierten Resistenzgen gekoppelt ist, mindestens in 5' mit einem retroviralen Verpackungssignal, einer Primerbindestelle ("primer binding site") für eine als Primer für die reverse Transkriptase dienende tRNA, sowie einen 5' lange terminale Struktur- oder Wiederholungseinheit ("long terminal repeat") zu verbinden. In 3' wird das Konstrukt mindestens von einem Polypurinabschnitt und einem 3' "long terminal repeat" begrenzt. Dabei ist die Ausführung als selbstinaktivierende retrovirale Vektoren bevorzugt, bei denen die "long terminal repeats" im Zuge der reversen Transkription in nicht funktionale Formen umgewandelt werden.

In der vierten Ausführungsform sieht die Erfindung Nukleinsäurekonstrukte als Bestandteil synthetischer eukaryontischer Transposons vor. Dabei schließen Erkennungsequenzen für eine für den Einsatz in eukaryontischen Zellen geeignete Transposase (z.B. "sleeping beauty Transposase" oder "frog prince" Transposase) NMD-Kassetten sowie diese flankierende Erkennungssequenzen für Rekombinasen und in entsprechenden Konstrukten beinhaltete Resistenzgene ein und bilden so ein synthetisches Transposon. Dieses Transposon wird zunächst in das Genom eukaryontischer Zellen, z.B. murine ES-Zellen, gezielt oder zufällig inseriert. Durch Expression der spezifischen Transposase in den Zellen wird das die NMD-Kassette beinhaltende Transposon mobilisiert und an einem anderen Locus im Genom transponiert. Erfolgt die Insertion in ein Intron eines anderen Gens, wird dieses in Abhängigkeit von der Orientierung der inserierten NMD-Kassette inaktiviert oder verbleibt zunächst in seinem aktiven Zustand. Aufgrund der erfindungsgemäß bevorzugten Invertierbarkeit der NMD-Kassette kann der Aktivitätszustand dann durch Expression einer der spezifisch geeigneten Rekombinasen geändert werden. In einer Abwandlung dieser Variante erfolgt die Transposition der NMD-Kassette nicht in ES-Zellen, sondern in Nachkommen daraus hergestellter Mäuse, indem diese mit ein geeignetes Transposase-Allel besitzenden Mäusen verpaart werden. Neben der bevorzugten Anwendung in murinen ES-Zellen und Mäusen ist die Verwendung erfindungsgemäßer Nukleinsäurekonstrukte als Substrat für eukaryontische Transposasen auch in allen nicht menschlichen eukaryontischen Organismen vorgesehen, die Spleißen von prä-mRNA sowie NMD aufweisen. Dies sind vor allem Versuchstiere, wie Ratte und Zebrafisch, bevorzugt aber auch Pflanzen sowie einzellige Eukaryonten.

In der zweiten bis vierten Ausführungsform der Erfindung ist die Verwendung eines in Eukaryonten und Prokaryonten selektierbaren Resistenzgens bevorzugt, z.B. eine Neomycinresistenzkassette, die zusätzlich zu den für die eukaryontische Expression notwendigen Sequenzen zusätzlich regulatorische Sequenzen für die Expression in Prokaryonten enthält, so dass in diesen mit Kanamycin für die Anwesenheit der Kassette selektiert werden kann. Weiterhin ist eine Verbindung des Resistenzgens mit einem prokaryontischen Replikationsursprung bevorzugt. Zusammen ermöglichen diese Elemente die Identifikation des Integrationsorts, durch sogenanntes "Plasmid Rescue" und anschließende Sequenzierung. Zum Plasmid Rescue wird die genomische DNA einer Zielzelle bevorzugt mit einem Restriktionsenzym fragmentiert, das nicht innerhalb der erfindungsgemäßen Nukleinsäurekonstrukte schneidet. Die Verfahren schließen aber auch das willkürliche Fragmentieren der gesamten DNA und Isolieren solcher Fragmente ein, die das erfindungsgemäße Nukleinsäurekonstrukt zumindest anteilig enthalten. Anschließend erfolgt mittels Ligation eine Zirkularisierung dabei entstandener Fragmente. Enthalten diese Fragmente die bevorzugte Kombination von bifunktionalem Resistenzgen und prokaryontischem Replikationsursprung, bilden sie Plasmide, die in Bakterien vermehrt werden können. Die übrigen zirkulären Fragmente enthalten diese Elemente nicht und können deshalb nach Transformation in Bakterien keine Resistenz vermitteln und nicht repliziert werden. In den so gewonnenen Plasmiden können die genomischen Fragmente dann mit Primern sequenziert werden, die innerhalb der erfindungsgemäßen Sequenzen hybridisieren. Alternativ können die Insertionspunkte mit dem Fachmann bekannten PCR-Methoden ermittelt werden, z.B. indem Oligonukleotidanker an Fragmente der genomischen DNA ligiert werden und eine PCR mit Primern durchgeführt wird, von denen der eine innerhalb der Anker und der andere Primer innerhalb der erfindungsgemäßen Kassette hybridisiert.

In einem Aspekt der ersten, zweiten, dritten und vierten Ausführungsform betrifft die Erfindung eukaryontische Zellen, vorzugsweise ES-Zellen, die dadurch genetisch verändert wurden, dass sie die erfindungsgemäßen Nukleinsäurekonstrukte enthalten, wodurch Zielgene inaktiviert sind, konditional inaktivierbar sind bzw. nach Auslösen der Inaktivierung durch Wechselwirkung mit einer Rekombinase inaktiviert sind, außerdem Zellen, die erfindungsgemäße Nukleinsäurekonstrukte und dadurch zunächst inaktivierte, konditional reaktivierbare Zielgene enthalten sowie Zellen, die Zielgene enthalten, deren Aktivitätszustand durch erfindungsgemäße Nukleinsäurekonstrukte mehrfach änderbar ist.

Weiterhin betrifft die Erfindung in diesen Ausführungsformen Organismen, die aus diesen Zellen erzeugt wurden, beispielsweise Pflanzen und Tiere, wie nicht-menschliche Säuger, z.B. Nagetiere, bevorzugt Mäuse, die aus den mit erfindungsgemäßen Nukleinsäurekonstrukten behandelten ES-Zellen erzeugt wurden. Solche Organismen weisen daher inaktivierte Gene, konditional inaktivierbare Gene, inaktivierte konditional reaktivierbare Gene auf, oder Gene, deren Aktivitätszustand konditional änderbar ist.

In der fünften Ausführungsform stellt die Erfindung Nukleinsäurekonstrukte und Verfahren zur Gentherapie dominanter Erbkrankheiten, wie z. B. Epidermolysis Bullosa simplex, bereit, sowie *ex vivo* kultivierbare eukaryontische Zellen, die mit erfindungsgemäßen Nukleinsäurekonstrukten kontaktiert wurden. So werden Nukleinsäurekonstrukte bereitgestellt, die zur Gentherapie, insbesondere bei Menschen oder Tieren einsetzbar sind, mit denen ein heterozygot vorliegendes dominant wirkendes Allel so veränderbar ist, dass es vollständig inaktiviert ist und nur noch das andere, nicht mutierte Allel exprimiert wird. Weiterhin werden tierische Zellen, beispielsweise menschlichen Ursprungs, bereitgestellt, die *ex vivo* durch Kontaktieren mit erfindungsgemäßen Nukleinsäurekonstrukten ein inaktiviertes Zielgen enthalten, wobei ein verwendetes Resistenzmarker- oder Reportergen nach Selektion eliminiert wurde. Solche Zellen stehen zur Re-Transplantation auf den Spenderorganismus oder zur Transplantation auf einen anderen Organismus bereit. Zielzellen für die Gentherapie sind z.B. somatische Zellen, Vorläuferzellen oder adulte Stammzellen. Ein besonderes Anwendungsgebiet der Gentherapie sind dominante Erbkrankheiten wie beispielsweise Epidermolysen, z. B. dominant vererbte Formen der Epidermolysis Bullosa simplex. Besonders bevorzugt zur Gentherapie sind Konstrukte, die mit Bezug auf die Verwendung als Genfalle beschrieben sind.

In einer sechsten Ausführungsform dienen erfindungsgemäße Nukleinsäurekonstrukte als Substrat für prokaryontische Transposasen *in vitro* bzw. *in vivo* zur Herstellung einzelner Vektoren für die gezielte Mutagenese in Eukaryonten bzw. für die Herstellung von Bibliotheken solcher Vektoren.

In der ersten Variante der Verwendung erfindungsgemäßer Nukleinsäurekonstrukte als Substrat für prokaryontische Transposasen schließen Erkennungssequenzen für eine geeignete Transposase NMD-Kassetten sowie diese flankierende Erkennungssequenzen für Rekombinasen und optional in diesen enthaltenden Konstrukten beinhaltete Resistenzgene ein und bilden ein synthetisches Transposon. Durch Inkubation eines solchen Transposons mit der geeigneten Transposase bildet sich ein als Transposom bezeichneter DNA-Proteinkomplex. Nach Mischen eines so hergestellten Transposoms mit einer geeigneten Ziel-DNA, z.B. einem Vektor, der ein kloniertes genomisches Fragment des Mausgenoms enthält, erfolgt unter geeigneten Bedingungen die zufällige Insertion des erfindungsgemäßen Transposons in die Ziel-DNA. Nach Abstoppen der Reaktion können entsprechende Vektoren zur Vermehrung in kompetente *E. coli* Stämme transformiert werden, wobei durch das mit der NMD-Kassette verbundene Resistenzgen eine Selektion für Klone erfolgen kann, die Vektoren enthalten, in die das Tranposon inseriert wurde. Durch Sequenzierung können dann die Produkte der Transposition ermittelt werden, bei denen die Insertion der NMD-Kassette so erfolgte, dass sie als Vektoren für eine gezielte Geninaktivierung geeignet sind. Dies sind alle Vektoren, bei denen die Insertion in ein Intron eines in dem klonierten genomischen Fragment lokalisierten Gens erfolgte.

In einer zweiten Variante der Verwendung erfindungsgemäßer Nukleinsäurekonstrukte als Substrat für Transposasen wird ebenfalls *zunächst in vitro* ein Transposom aus einem erfindungsgemäßen Nukleinsäurekonstrukt und der geeigneten spezifischen Transposase gebildet. In Abwandlung des oben beschriebenen Verfahrens wird das Transposom jedoch in *E. coli* transformiert, die außerdem einen Vektor beinhalten, der ein kloniertes genomisches Fragment z.B. des Mausgenoms, enthält. Dabei erfolgt die Insertion des eine NMD-Kassette beinhaltenden Transposons *in vivo.* Durch das mit der NMD-Kassette verbundene Resistenzgen kann wiederum eine Selektion für Klone erfolgen, die Vektoren enthalten, in die das Transposon inseriert wurde. Durch Sequenzierung können dann die Vektoren identifiziert werden, bei denen die Insertion der NMD-Kassette so erfolgte, dass die Vektoren zur Verwendung für eine gezielte Geninaktivierung geeignet sind. Dies sind alle Vektoren, bei denen die Insertion in ein Intron eines in dem klonierten genomischen Fragment lokalisierten Gens erfolgte.

In Fortbildung der genannten Varianten zur Verwendung erfindungsgemäßer Nukleinsäurekonstrukte als Substrat für prokaryontische Transposasen können entsprechende Konstrukte mit einem prokaryontischen Replikationsursprung gekoppelt werden, der es erlaubt, aus einem größeren Fragment, in das eine erfindungsgemäßes Transposon inseriert wurde, direkt ein kleineres Fragment zu subklonieren, in dem die NMD-Kassette weiterhin von genomischen Fragmenten flankiert ist.

In einer bevorzugten Variante sind erfindungsgemäße, als Substrat für prokaryontische Transposasen dienende Nukleinsäurekonstrukte zur Herstellung von Bibliotheken von Vektoren vorgesehen, die zur gezielten Mutagenese eukaryontischer Gene verwendet werden können. Dabei kann die Transposition entsprechend der erstgenannten Variante der Ausführungsform erfindungsgemäßer Nukleinsäurekonstrukte als Substrat für Transposasen *in vitro* mit einem Gemisch von Vektoren, die subgenomische Fragmente des Zielorganismus enthalten, erfolgen. Alternativ kann die Transposition entsprechend der zweitgenannten Variante der Ausführungsform erfindungsgemäßer Nukleinsäurekonstrukte als Substrat prokaryontischer Transposasen *in vivo* in einer Bibliothek klonierter genomischer Fragmente des Zielorganismus, z.B. in einer Bibliothek von künstlicher bakterieller Chromosomen (BACs, "bacterial artificial chromosomes"), erfolgen. In beiden Fällen ist das Ergebnis eine Bibliothek von Vektoren, die zur gezielten Mutagenese mittels homologer Rekombination in dem Zielorganismus, z.B. einer murinen ES-Zelle, verwendet werden kann.

Erfindungsgemäß ist vorgesehen, dass die Nukleinsäurekonstrukte mindestens die folgenden funktionalen Elemente aufweisen:
- eine Spleißakzeptorstelle,
- in 5' Position von der Spleißakzeptorstelle einen synthetischen oder natürlichen ersten Intronabschnitt, der an seinem 3'-Ende alle zur Funktion eines 3'-Endes eines Introns notwendigen Elemente enthält, dessen 5'-Ende aber keine funktionalen Elemente eines 5'-Endes eines Introns aufweist
- in 3' von der Spleißakzeptorstelle eine Spleißdonorstelle,
- wobei zwischen Spleißakzeptorstelle und Spleißdonorstelle ein funktionales Exon gebildet wird, das mindestens ein Stopkodon enthält, bevorzugt drei Stopkodons, davon eins in jedem möglichen Leserahmen, vorzugsweise ausgewählt aus den Stopkodons UAA und UAG,
- wobei das mindestens eine, bevorzugt die drei Stopkodons vorzugsweise mehr als 50 bp, bevorzugter mehr als 55 bp von der Spleißdonorstelle beabstandet sind, und
- vorzugsweise das Exon eine nicht durch 3 teilbare Anzahl von Nukleotiden aufweist.
- in 3' an die Spleißdonorstelle anschließend einen zweiten synthetischen oder natürlichen Intronabschnitt, der der alle zur Funktion eines 5'-Endes eines Introns notwendigen Elemente enthält, dessen 3'-Ende aber keine funktionalen Elemente eines 3'-Endes eines Introns aufweist

Nachfolgend wird die Kombination dieser minimal notwendigen funktionalen Elemente entsprechend ihrer Funktion zur Vermittlung der Nonsense vermittelten RNA-Degradation als NMD-Kassette bezeichnet.

Die erfindungsgemäßen NMD-Kassetten enthalten bevorzugt zumindest ein funktionales Exon, das eine Spleißakzeptorstelle an seinem 5'-Ende und eine Spleißdonorstelle an seinem 3'-Ende aufweist, wobei die Länge dieses Exons bevorzugt mindestens 50 Nukleotide beträgt. Das Exon enthält bevorzugt mindestens ein Stopkodon, bevorzugter eins in jedem möglichen Leserahmen, bevorzugt mindestens 50 Nukleotide stromaufwärts von der Spleißdonorstelle des Exons. Dabei werden die Stopkodons UAA und UAG in Säugetierzellen bevorzugt, da das dritte (UGA) unter bestimmten Bedingungen nicht als Stopkodon wirkt, sondern ein Selenocystein kodiert.

Alternativ oder zusätzlich kann das in einer NMD-Kassette enthaltene Exon eine nicht glatt durch 3 teilbare Anzahl von Nukleotiden aufweisen, die also kein Vielfaches von 3 ist, sondern ein Vielfaches von 3 plus 1 Nukleotid oder plus 2 Nukleotide.

Da bei natürlich vorkommenden Genen die Intronphasen am 5'- und 3'- Ende eines Introns gleich sind, führt das Spleißen von prä-mRNAs natürlicher Gene auch dann zu funktionalen mRNAs, wenn einzelne Kodons aus Nukleotiden zusammengesetzt sind, die jeweils von benachbarten Exons stammen. Im Gegensatz ist in der alternativen NMD-Kassette mit einem Exon, das eine nicht glatt durch 3 teilbare Anzahl von Nukleotiden aufweist, das Kodon am 5' Ende dieses Exons an einer anderen Position von einem Intron unterbrochen, als das Kodon am 3'-Ende des Exons der NMD-Kassette. Die nicht glatt durch 3 teilbare Anzahl von Nukleotiden des Exons erzeugt eine Verschiebung der Intronphase, so dass in einem Transkript ein von einem stromabwärts gelegenen Exon kodiertes Stopkodon erzeugt werden kann, wenn in dem resultierenden Leserahmen ein entsprechendes Triplett (UAA, UAG oder UGA) vorliegt. Dieses wird aber nur wirksam, wenn das in der NMD-Kassette enthaltene Exon kein Stopkodon enthält.

Für die Zwecke der Erfindung werden die Spleißakzeptorstelle und die Spleißdonorstelle in ihren Positionen mit dem Nukleotid bezeichnet, das jeweils als erstes bzw. letztes Nukleotid einen Bestandteil des Exons bildet und daher in einer prozessierten mRNA enthalten ist.

In 5' angrenzend an die Spleißakzeptorstelle ist vorzugsweise ein flankierender erster Intronabschnitt angeordnet, der bevorzugt die funktionalen Elemente eines 3'-Endes eines Introns beinhaltet. Dies sind die intronischen Anteile der Spleißakzeptorstelle, die das 5'-Ende des NMD vermittelnden Exons definiert, ein für die Lariatbildung beim Spleißen notwendiger Verzweigungspunkt ("branch point"), der bevorzugt 10 bis 50 Nukleotide in 5' von der Spleißakzeptorstelle lokalisiert ist, sowie zwischen Verzweigungspunkt und Spleißakzeptor lokalisierter Polypyrimidinabschnitt von 10 bis 50 Nukleotiden Länge. 5' von dem Verzweigungspunkt sind weitere Sequenzen vorgesehen, die bevorzugt keine weiteren Spleißakzeptorstellen, Verzweigungspunkte oder Polypyrimidine und auch keine Spleißdonorstelle enthalten. Diese zusätzlichen Sequenzen weisen bevorzugt eine Länge auf, die bei Insertion der NMD-Kassette direkt stromabwärts benachbart zu einer Spleißdonorstelle eines Zielgens ausreicht, eine Lariatbildung zwischen dieser Spleißdonorstelle und dem in dem Nukleinsäurekonstrukt enthaltenen Verzweigungspunkt zu ermöglichen. Dabei wird eine Länge von 10 bis 200 Nukleotiden bevorzugt.

In 3' angrenzend an die Spleißdonorstelle ist vorzugsweise ein flankierender zweiter Intronabschnitt angeordnet, der bevorzugt die funktionalen Elemente eines 5'-Endes eines Introns beinhaltet. Dies sind im wesentlichen die intronischen Anteile der Spleißdonorstelle, die das 3'-Ende des NVD vermittelnden Exons definiert. In 3' davon sind bevorzugt keine weiteren Spleißdonorstellen und auch keine Elemente vom 3'-Ende eines Introns. Die zusätzlich in 3' von den intronischen Anteilen der Spleißdonorstelle vorgesehenen Sequenzen weisen bevorzugt eine Länge auf, die bei Insertion der NMD-Kassette direkt stromaufwärts benachbart zu einem Verzweigungspunkt eines Zielgens ausreicht, eine Lariatbildung zwischen der in der NMD-Kassette enthaltenen Spleißdonorstelle und dem in dem Zielgen benachbarten Verzweigungspunkt zu ermöglichen. Dabei wird eine Länge von 10 bis 200 Nukleotiden bevorzugt.

Diese flankierenden ersten und zweiten Intronabschnitte ersetzen oder ergänzen bei der Integration in das Intron eines Zielgens dessen Intronbereich, so dass das Konstrukt ein Exon des Zielgens ersetzt oder ein zusätzliches Exon einführt.

Bei Insertion einer NMD-Kassette in ein Intron zwischen den intronischen Anteilen der Spleißdonorstelle am 5'-Ende des Introns und dem Verzweigungspunktes am 3'-Ende des Introns bilde der erste und der zweite Intronabschnitt der NMD-Kassette mit den an sie angrenzenden Sequenzen des ursprünglichen Introns des Zielgens vollständige funktionale Introns. Dadurch gewinnt das in der NMD-Kassette enthaltene Exon seine Funktionalität beim Spleißen, indem es mit den in 5' und 3' zur Integrationsstelle angrenzenden Spleißdonor- bzw. Spleißakzeptorstellen des Zielgens jeweils Spleißapparate ausbilden kann. In der Folge ist das Exon der im kodogenen Strang angeordneten NMD-Kassette im gespleißten Transkript enthalten.

In bevorzugter Ausführung enthalten die NMD-Kassetten starke Spleißakzeptor- und Spleißdonorstellen, sowie einen stark wirksamen Verzweigungspunkt in 5' von der Spleißakzeptorstelle, besonders bevorzugt in 3' an diesen angrenzend einen Polypyrimidinabschnitt. Diese Kombination führt zu einer hohen Exonfunktionalität des in der NMD-Kassette enthaltenen Exons, da sie der Anordnung zum Spleißen notwendiger Elemente konstitutiver Exons entspricht, die im wesentlichen keinem alternativen Spleißen unterliegen.

Die vollständige Inaktivierung aller endogenen Spleißvarianten eines Gens kann dadurch erreicht werden, dass eine erfindungsgemäße NMD-Kassette in ein Intron oder Exon des Zielgens integriert wird, das in jedem der alternativen Transkripte enthalten ist.

Für die Erzeugung hypomorpher Mutanten werden erfindungsgemäße Nukleinsäurekonstrukte eingesetzt, in denen zumindest eines von Spleißakzeptor- und Spleißdonorstelle und dem Verzweigungspunkt in 5'von der Spleißakzeptorstelle eine niedrigere Aktivität haben, also schwächer sind. Zum gleichen Zweck kann auch der Polypyrimidinabschnitt weniger gut mit den im Spleißosom enthaltenen RNAs basenpaarende Nukleotide enthalten.

Diese am Spleißen beteiligten Elemente mit verminderter Funktionsfähigkeit führen dazu, dass das Spleißen unvollständig ist und eine NMD-Kassette in aktiver Orientierung, d.h. im kodogenen Strang angeordnet, anteilig mit den benachbarten Introns entfernt wird, und somit zur unvollständigen NMD und daher zu einem hypomorphen Phänotyp.

Weitere optionale funktionale Elemente der erfindungsgemäßen Nukleinsäurekonstrukte sind:
- intronische oder exonische Spleißverstärkersequenzen innerhalb der Intron- bzw. Exonsequenzen der NMD-Kassette, die zusätzlich zu den vorgenannten Sequenzen enthalten sein können, oder Abschnitte der NMD-Kassette mit gleicher Funktion ersetzen.

Die Erfindung bevorzugt die optionale Flankierung von NMD-Kassetten mit
- flankierenden Paare von Erkennungssequenzen für eine oder mehrere Rekombinasen oder Integrasen, wobei dies bevorzugt Erkennungssequenzen für die
- die Rekombinasen Cre und/oder Flp, Dre sowie für die Integrase phiC31 spezifisch sind,
- außerdem Mutanten von Erkennungssequenzen für Rekombinasen oder Integrasen, z.B. Mutanten von loxP und frt-Erkennungssequenzen sind, noch bevorzugter Mutanten von loxP und frt-Erkennungssequenzen, die eine irreversible Invertierung der dazwischen liegenden DNA-Abschnitte ermöglichen, beispielsweise 1ox66 und 1ox71, sowie frtLE und frtRE.
- wobei die Paare von Erkennungssequenzen für jeweils eine Rekombinase in gleichgerichteter oder entgegengesetzter Orientierung angeordnet sein können,
- und die Orientierung jeweils eines Paares von Erkennungssequenzen für eine spezifische Rekombinase oder Integrase unabhängig von der Orientierung der paarweisen Erkennungssequenzen anderer Rekombinasen oder Integrasen ist.

Die Erfindung sieht bevorzugt die Flankierung von NMD-Kassetten und weiterer in umfänglicheren Nukleinsäurekonstrukten enthaltener Bestandteile, z.B. ein Resistenzgen, mit Erkennungssequenzen für Rekombinasen und Integrasen vor. Dabei bevorzugt die Erfindung die Anwendung von Rekombinaseerkennungssequenzen zur konditionalen Manipulation von NMD-Kassetten, während Integraseerkennungssequenzen bevorzugt als Alternative für Rekombinaseerkennungssequenzen für die Manipulation von Resistenzgenen und anderer Elemente sind, die nicht Bestandteil der minimal notwendigen funktionalen Elemente der NMD-Kassette sind.

Vorzugsweise werden die minimal notwendigen funktionalen Elemente der NMD-Kassette beidseitig von unterschiedlich orientierten Erkennungssequenzen für eine Rekombinase flankiert, so dass die Wechselwirkung mit der für diese spezifischen Rekombinase zur Invertierung der Orientierung der NMD-Kassette innerhalb ihres Insertionsorts führt. Die Anwesenheit von flankierenden Erkennungssequenzen für eine Rekombinase stellt die konditionale Aktivierbarkeit der NMD-Kassette her, wenn diese in den nicht-kodogenen Strang des Zielgens integriert ist, bzw. die konditionale Inaktivierbarkeit der NMD-Kassette, wenn diese in den kodogenen Strang des Zielgens integriert ist. Die Inversion der NMD-Kassette wird dann erreicht, wenn die Erkennungssequenzen gegenläufig, d.h. in unterschiedlicher Orientierung, angeordnet sind, so dass sie eine Inversion des zwischen ihnen angeordneten Exons ermöglichen. Bei gleichgerichteter Anordnung der Erkennungssequenzen führt die Wechselwirkung mit der Rekombinase zur Deletion des Exons. Daher kann alternativ oder zusätzlich zu gegenläufig angeordneten Erkennungssequenzen das Exon der NMD-Kassette oder die gesamte NMD-Kassette zur Deletion von gleichgerichteten Erkennungssequenzen eingefaßt sein.

Im Rahmen der Erfindung bezeichnet der Begriff der Aktivierung der NMD-Kassette die Wechselwirkung einer spezifischen Rekombinase mit den die NMD-Kassette flankierenden Erkennungssequenzen, die zu einer Inversion der NMD-Kassette führt, so dass das in der NMD beinhaltete Exon in den kodogenen Strang des Zielgens gelangt.

Als Inaktivierung der NMD-Kassette wird die Wechselwirkung einer spezifischen Rekombinase bezeichnet, die das in der NMD-Kassette beinhaltete Exon aus dem kodogenen Strang des Zielgens entfernt, entweder durch Inversion, wodurch das Exon in den nicht-kodogenen Strang gelangt, oder durch Deletion des von den Erkennungssequenzen eingefaßten Fragmentes.

Die Wechselwirkung einer Rekombinase und/oder Integrase kann, abhängig von der Art und/oder Orientierung der Erkennungssequenzen, zur Inversion oder Deletion der dazwischen angeordneten DNA-Abschnitte, z.B. der NMD-Kassette und/oder eines Resistenzgens verwendet werden.

Die Inversion der NMD-Kassette bewirkt die Inaktivierung des Gens durch Degradation der davon synthetisierten RNA, wenn die funktionalen Elemente der NMD-Kassette nach der Inversion im kodogenen Strang des Zielgens angeordnet sind, bzw. die Re-Aktivierung des Zielgens, wenn die funktionalen Elemente der NMD-Kassette ursprünglich in den nicht-kodogenen Strang des Zielgens integriert waren.

Im Fall der konditionalen Inaktivierung des Zielgens sind die funktionalen Elemente der NMD-Kassette zunächst im nicht-kodogenen Strang des Zielgens lokalisiert. Die Orientierung der NMD-Kassette wird durch die Wechselwirkung mit der Rekombinase umgekehrt, d.h. invertiert, so dass die in der NMD-Kassette enthaltenen funktionalen Elemente in den kodogenen Strang des Zielgens angeordnet werden, so dass die Spleißakzeptorstelle und die Spleißdonorstelle des in der NMD-Kassette enthaltenen Exons mit den Spleißdonor- bzw. Spleißakzeptorstellen der angrenzenden Exons des Zielgens einen funktionalen Spleißapparat ausbilden können und die das NMD vermittelnde Exon flankierenden Introns aus dem Transkript gespleißt werden. In der Folge wirkt das mindestens eine Stopkodon, vorzugsweise eines der drei Stopkodons, in dem NMD vermittelnden Exon als prämatures Stopkodon, so dass das Transkript der NMD zugeführt wird.

Es wurde gefunden, dass durch Invertieren der NMD-Kassette zur Anordnung ihrer funktionalen Elemente im kodogenen Strang des Zielgens der Nonsense vermittelte RNA-Abbau für das spezifische Gen ausgelöst wird. In den Ausführungsformen, in der die NMD-Kassette mit oder ohne flankierende Erkennungssequenzen für eine Rekombinase unmittelbar in ihrer aktiven Orientierung in das Zielgen integriert ist, d.h. ihre funktionalen Bestandteile im kodogenen Strang angeordnet sind, werden entsprechende Transkripte degradiert.
Die Inversion der integrierten NMD-Kassette aus der aktiven Orientierung durch Wechselwirkung flankierender Erkennungssequenzen mit der spezifischen Rekombinase führt dann zur Anordnung der funktionalen Elemente der NMD-Kassette in den nicht kodogenen Strang, also zur Wirkungslosigkeit der NMD-Kassette und damit zur Reaktivierung des Zielgens.

Während der Transkription des eukaryontischen Gens, welches die im Intron integrierte NMD-Kassette im nicht-kodogenen Strang enthält, d.h. vor der Inversion in ihre aktive Orientierung, wird ein Transkript erzeugt, aus dem durch Spleißen zwischen den ursprünglichen eukaryontischen Spleißakzeptor- und Spleißdonorstellen des Zielgens die Bereiche des ursprünglichen eukaryontischen Introns einschließlich der darin integrierten NMD-Kassette entfernt werden. Im Ergebnis wird eine unveränderte mRNA erhalten, da die für das Spleißen erforderlichen Elemente der NMD-Kassette im nicht kodogenen Strang der Zielzelle vorliegen. Nach Inversion der NMD-Kassette, d.h. bei ihrer Orientierung im kodogenen Strang, wird das in ihr befindliche Exon erkannt und das darin enthaltene prämature Stopkodons führt zur NMD.

Bei allen konditionalen Ausführungsformen der Erfindung umrahmen bzw. flankieren Erkennungssequenzen die NMD-Kassette und wirken als das Element, das auf den Auslöser hin, nämlich die Anwesenheit der spezifischen Rekombinase, die NMD-Kassette aktiviert oder inaktiviert. Die Wechselwirkung der Erkennungssequenzen mit der für diese spezifischen Rekombinase führt zu einer mittels derselben Rekombinase irreversiblen Invertierung der NMD-Kassette oder zu deren Deletion. Die Inversion der NMD-Kassette führt abhängig von der Anordnung ihrer funktionalen Elemente vor der Wechselwirkung im kodogenen oder nicht kodogenen Strang des Zielgens zu deren Anordnung in den jeweils komplementären Strang und entsprechend zur Re-Aktivierung bzw. Inaktivierung des Zielgens. Die Deletion einer aktiven NMD-Kassette durch die Rekombinase führt zur Re-Aktivierung des vorher inaktivierten Zielgens.
In Fortbildung der Erfindung ist es möglich, die NMD-Kassette, die von einem Paar von Erkennungssequenzen für eine Rekombinase flankiert wird, zusätzlich mit Erkennungssequenzen eines zweiten Paars von für eine andere Rekombinase spezifischen Erkennungssequenzen zu flankieren. In dieser Ausführungsform führt die Anwesenheit der zweiten spezifischen Rekombinase gezielt zu einer weiteren mittels derselben Rekombinase irreversiblen Inversion oder zur Deletion der dazwischen liegenden NMD-Kassette. Solche zweiten Erkennungssequenzen, die die minimale NMD-Kassette beidseitig flankieren, können wiederum von dritten, vierten und weiteren Paaren anderer spezifischer Erkennungssequenzen für Rekombinasen flankiert werden, die unabhängig voneinander ebenfalls jeweils für eine irreversible Inversion der dazwischen liegenden Sequenzen bzw. zu deren Deletion eingesetzt werden können.

Anstelle von geschachtelten Paaren von Erkennungssequenzen für Rekombinasen, die die NMD-Kassette beidseitig umfassen, können auch paarweise ineinander geschachtelte Sequenzen verwendet werden, was auch als so genannter Flex-Switch bekannt geworden ist (Branda CS und Dymecki SM (2004): Talking about a revolution: The impact of site-specific recombinases on genetic analyses in mice. Dev Cell 6 (1):7-28). Die Verwendung solcher paarweise ineinander geschachtelten Paaren spezifischer Erkennungssequenzen für Rekombinasen führt dazu, dass erst die Wechselwirkung des einen Paares von Erkennungssequenzen mit einer ersten spezifischen Rekombinase das Paar von Erkennungssequenzen für die zweite spezifische Rekombinase so positioniert und orientiert, dass sie für die spezifische Rekombinase und damit für gewünschte Funktion, z.B. Inversion oder Deletion, funktional werden. Der genannte Flex-Switch lässt sich auch auf zweite und dritte, dritte und vierte bzw. jedes aufeinander folgende Paar sich jeweils beidseitig umfassender Paare von Erkennungssequenzen für Rekombinasen anwenden.

Falls die erfindungsgemäße NMD-Kassette von einem ersten Paar spezifischer Erkennungssequenzen für eine Rekombinase flankiert wird und zusätzlich noch von mindestens einem weiteren Paar Erkennungssequenzen für mindestens eine weitere spezifische Rekombinase beidseitig flankiert wird, lässt sich die NMD-Kassette durch die Gegenwart der jeweils spezifischen Rekombinase zumindest zweimal invertieren, bzw. zunächst einmal invertieren und bei entsprechender Orientierung des weiteren Paars von Erkennungssequenzen schließlich deletieren. Wenn die erfindungsgemäße NMD-Kassette nach Insertion in das Zielgen im nicht-kodogenen Strang vorliegt, kann ihre erste Invertierung mittels einer ersten Rekombinase zur Inaktivierung des Zielgens eingesetzt werden. Diese Inaktivierung des Zielgens kann rückgängig gemacht werden, indem mittels der zweiten Rekombinase die Kassette erneut invertiert wird und zurück in den nicht-kodogenen Strang gelangt oder indem die NMD-Kassette mittels der zweiten Rekombinase deletiert wird. Mit beiden Verfahren ist Aktivität des Zielgens gesteuert wiederherstellbar.

Alternativ kann die NMD-Kassette nach Insertion in das Zielgen im kodogenen Strang des Zielgens vorliegen und dieses inaktivieren. Durch Invertierung mittels einer ersten Rekombinase gelangt die NMD-Kassette in den nicht-kodogenen Strang, so dass im Ergebnis das Zielgen re-aktiviert wird. Ein weiteres Paar von Erkennungssequenzen für eine zweite Rekombinase kann dann zur erneuten Inaktivierung des Zielgens durch Invertierung der NMD-Kassette mittels der zweiten Rekombinase in ihre aktive Orientierung genutzt werden. Entsprechend läßt sich eine NMD-Kassette wiederholt invertieren, wenn sie von weiteren Paaren von Erkennungssequenzen für weitere Rekombinasen flankiert wird, die andere Erkennungssequenzen zur Rekombination benutzen als die erste und zweite Rekombinase. Im Ergebnis erhält man eine mehrfach invertierbare NMD-Kassette, die zur wiederholten Änderung des Aktivitätszustandes eines Gens benutzt werden kann, wobei die Anzahl der möglichen Inversionen gleich der Anzahl der Paare von Erkennungssequenzen ist.

Als Erkennungssequenzen für Rekombinase sind solche Sequenzen geeignet, die in Wechselwirkung mit der spezifischen Rekombinase zur reversiblen, vorzugsweise zur irreversiblen Invertierung der NMD-Kassette führen. Als Erkennungssequenzen für Rekombinasen sind gegeneinander gerichtete lox-, gleich gerichtete frt- und rox-Sequenzen sowie Erkennungssequenzen für phiC31-Integrase, bevorzugterweise irreversibel invertierbare Sequenzen einsetzbar, beispielsweise gegeneinander gerichtete mutierten loxP- oder frt-Sequenzen (lox66 in Kombination mit 1ox71, oder frtLE in Kombination frtRE, wie in Branda und Dymecki beschrieben (Branda CS und Dymecki SM (2004): Talking about a revolution: The impact of site-specific recombinases on genetic analyses in mice. Dev Cell 6(1) :7-28) . Alternativ ist die Herstellung der Irreversibilität einer Rekombinasereaktion durch Anordnung ihrer Erkennungssequenzen als Flex-Switch vorgesehen.

Die Erfindung sieht bevorzugterweise vor, NMD-Kassetten mit einem
- Resistenzgen oder optional mit
- einem Resistenz- und/oder Reportergen zu einem gemeinsamen Abschnitt zu verbinden.
Dabei sind
- bifunktionale Resistenzgene bevorzugt, die Resistenz in Eukaryonten und Prokaryonten vermitteln, und außerdem solche, die
- positiv und negativ selektiert werden können, wobei vorgesehen ist
- das Resistenzgen und das optional vorhandene Reportergen mit einem Paar von Erkennungssequenzen für eine Rekombinase oder Integrase zu flankieren, wobei
- dies bevorzugt andere Erkennungssequenzen sind als jene, die die NMD-Kassette flankieren.

In bevorzugter Ausführungsform ist die erfindungsgemäße NMD-Kassette mit einem Selektionsmarkergen kombiniert, das von anderen Erkennungssequenzen für Rekombinase flankiert wird, als sie in der NMD-Kassette enthalten sind, so dass das Resistenzgen mittels der spezifischen Rekombinase unabhängig deletiert werden kann. Das von Erkennungssequenzen für Rekombinase flankierte Resistenzgen kann innerhalb der NMD-Kassette oder angrenzend an diese zu einem gemeinsamen DNA-Abschnitt verbunden sein. Beispiele für Selektionsmarkergene sind Resistenzgene, die eine Antibiotikaresistenz in Eukaryonten vermitteln, vorzugsweise gleichzeitig mit einer Resistenz in Bakterien, wie z.B. das Neomycin-/Kanmycinresistenzgen. Weiter bevorzugt sind Selektionsmarkergene, gegen die positiv und negativ selektiert werden kann, wie z.B. Fusionsproteine von *Herpes simplex* Thymidinkinase mit einem Puromycin- oder einem Neomycinresistenzgen. Optional ist vorgesehen, ein mit der NMD-Kassette verbundenes eukaryontisch und prokaryontisch wirksames Resistenzgen mit
- einem prokaryontischen Replikationsursprung zu verbinden, der es ermöglicht DNA-Fragmente, die ein entsprechendes Nukleinsäurekonstrukt beinhalten, nach Zirkularisierung in Bakterien, z.B. *E. coli,* zu vermehren, wobei
- die Verbindung des Replikationsursprungs mit dem Selektionsmarkergen bevorzugt so erfolgt, dass er innerhalb des Paares von Erkennungssequenzen für eine Rekombinase oder Integrase liegt, die das Resistenzgen flankieren.

Die Möglichkeit, das Resistenzmarkergen und einen optional damit verbundenen prokaryontischen Replikationsursprung im Anschluss an die Schritte der Insertion in ein Intron des Zielgens und der Selektion durch die Aktivität einer spezifischen Rekombinase oder Integrase zu entfernen, bietet unabhängig von der NMD-Kassette den besonderen Vorteil, dass weitere Auswirkungen eines Selektionsgens und des Replikationsursprungs, beispielsweise transaktivierende Einflüsse, unterbunden werden.

Für die gezielte Insertion in Exons sieht die Erfindung vor, eine NMD-Kassette und ein mit ihr verbundenes Resistenzgen mit weiteren Sequenzen außerhalb der Erkennungsstellen für Rekombinasen und Integrasen zu flankieren. Dies sind bevorzugt
- ein synthetischer oder natürlicher zusätzlicher dritter Intronabschnitt mit den intronischen Anteilen einer Spleißdonorstelle an seinem 5'-Ende, angeordnet in 5' vom ersten Intronabschnitt, zusammen mit
- einem zusätzlichen synthetischen oder natürlicher vierten Intronabschnitt, angeordnet in 3' vom zweiten Intronabschnitt, mit den intronischen Anteilen einer Spleißakzeptorstelle an seinem 3'-Ende, sowie 5' davon den zur Wirkung eines Spleißakzeptors notwendigen Polypyrimidinabschnitt sowie einen 5' davon einen Verzweigungspunkt,
- wobei dieser dritte und vierte Intronabschnitt keine weiteren Spleißdonor- und Spleißakzeptorstellen und auch keinen weiteren Verzweigungspunkt aufweist und
- außerhalb von optional in entsprechenden Nukleisäurekonstrukten enthaltenen Rekombinaseerkennungssequenzen lokalisiert sind, die die minimal notwendigen funktionalen Elemente einer NMD-Kassette und das Resistenzgen flankieren.
- Optional können intronische Spleißverstärkersequenzen innerhalb der genannten zusätzlichen dritten und vierten Intronabschnitte lokalisiert sein.

Zusätzlich zu den in 5' und 3' das Exon flankierenden ersten und zweiten Intronabschnitten kann das Konstrukt zusätzliche Intronabschnitte aufweisen, nämlich einen dritten Intronabschnitt, der an seinem 5' Ende die intronischen Anteile einer Spleißdonorstelle aufweist, positioniert in 5' von dem ersten Intronabschnitt. Der andere zusätzliche Intronabschnitt ist der vierte Intronabschnitt, der am 3' Ende des zweiten Intronabschnitts positioniert ist. Dieser vierte Intronabschnitt weist an seinem 3' Ende die intronischen Anteile einer Spleißakzeptorstelle , sowie 5' davon den zur Wirkung eines Spleißakzeptors notwendigen Polypyrimidinabschnitt sowie einen Verzweigungspunkt auf. Bei Integration bilden die zusätzlichen dritten und vierten Intronabschnitte mit dem jeweils angrenzenden ersten bzw. zweiten Intronabschnitten vollständige funktionale Introns, die das NMD vermittelnde Exon flankieren. Dabei ist vorgesehen, entsprechende Konstrukte in sogenannte Protospleißstellen (Dibb NJ, Newman AJ. (1989): Evidence that introns arose at proto-splice sites. EMBO J 8(7):2015-2021) in Exons zu inserieren, die zusammen mit den intronischen Anteilen der in entsprechenden Konstrukten enthaltenen Spleißdonor- und -akzeptorstellen komplette funktionale Spleißstellen ergeben. Solche Protospleißstellen weisen bevorzugt die Nukleotidsequenz CMAGR auf, wobei die Angabe der Sequenz gemäß der IUPAC Konvention erfolgt (M steht dabei für das alternative Vorliegen der Nukleotide A oder C und R steht für G oder A). Innerhalb der Protospleißstelle ist die Insertion zwischen dem Guanosin (G) und dem 3' davon folgenden Purin vorgesehen (R).

Bevorzugt sind entsprechende Konstrukte dazu vorgesehen, in intronlose Ein-Exon-Gene inseriert zu werden, die durch die Aufspaltung des endogenen Exons und die Insertion des NMD vermittelnden Exons in ein Gen mit drei Exons umgewandelt wird. Der dritte und vierte Intronabschnitt sind notwendig, wenn das Konstrukt zur Integration in ein Exon eines Zielgens verwendet werden soll, um in 5' von der Spleißakzeptorstelle und in 3' von der Spleißdonorstelle der NMD-Kassette jeweils ein vollständiges Intron herzustellen. Dies wird erreicht, indem die Enden dieser Intronabschnitte in Kombination mit den Exonsequenzen, die den Insertionspunkt flankieren, eine funktionale Spleißdonor- bzw. eine funktionale Spleißakzeptorstelle bilden. Auf diese Weise wird ein Exon im Genom der Zielzelle durch Insertion der NMD-Kassette, die in beidseitig von jeweils einem vollständigen Intron flankiert wird, in zwei funktionale Exons unterteilt, welche die NMD-Kassette flankieren und von dem in der NMD-Kassette beinhalteten Exon durch vollständige, funktionelle Introns getrennt sind.

Für die gezielte Mutagenese von Zielgenen mittels homologer Rekombination ist es weiterhin vorgesehen, Nukleinsäurekonstrukte, die für die Insertion in Introns vorgesehen sind, und solche, die in Exons inseriert werden sollen, zusätzlich mit Sequenzen des jeweiligen Zielgens zu flankieren, die homolog zu den Sequenzen sind, die den gewählten Insertionspunkt in dem Zielgen flankieren.

Für die Verwendung als Genfalle ist vorgesehen, NMD-Kassetten, die mit einem Selektionsmarkergen, mit einem mit einem prokaryontischen Replikationsursprung gekoppelten Selektionsmarkergen, mit einem Reportergen oder den oben beschriebenen Kombinationen von Selektionsmarker- und Reportergenen gekoppelt sind, mit synthetischen oder natürlichen Sequenzen, die keine Spleißsignale enthalten, zu flankieren, um einen Verlust funktional wichtiger Elemente bei zufälliger Integration entsprechender Konstrukte in das Genom der Zielzelle zu verhindern.

Weiterhin sieht die Erfindung für Genfallen vor,
- in entsprechenden Konstrukten enthaltene Reportergene optional als terminale Exons auszuführen, bei denen der offene Leserahmen des Reportergens am 5'-Ende von einem Polypyrimidinabschnitt, einem Verzweigungspunkt und einem Spleißakzeptor begrenzt wird und am 3'-Ende von einem funktionalen Polyadenylierungssignal gefolgt wird, auf das eine erfindungsgemäße NMD-Kassette folgt,
- wobei drei unterschiedliche Konstrukte vorgesehen sind, die sich darin unterscheiden, dass der offene Leserahmen des Reportergens in jeweils einer anderen Intronphase an die 5' angrenzende Spleißakzeptorstelle angrenzt.

Außerdem ist vorgesehen in solchen Konstrukten beinhaltete Resistenz- und Reportergene sowie den optional mit diesen gekoppelten Replikationsursprung so mit Erkennungssequenzen für eine Rekombinase oder Integrase zu flankieren, dass diese Sequenzen mittels der geeigneten Rekombinase / Integrase nach erfolgter Integration im Genom deletiert werden können und die NMD-Kassette in eine wirksame Position gerät.

Reportergene , die bevorzugt mit der NMD-Kassette gekoppelt sind, sind z.B. die Gene für β-Galactosidase, GFP, eGFP und andere fluoreszierende Genprodukte.

In einer Ausführungsform kann ein Selektionsmarker- und ein Reportergen gekoppelt sein, insbesondere indem sie in einer bicistronischen Expressionkassette verbunden sind, bei der das 3' gelegene Cistron von einer IRES (interne Ribosomeneintrittsstelle) translatierbar ist. Weiterhin können Fusionsproteine eines Selektionsmarker- und eines Reportergens mit einer NMD-Kassette gekoppelt sein.

Außerdem sieht die Erfindung NMD-Kassetten beinhaltende Nukleinsäurkonstrukte für den retroviralen Gentransfer vor. Dazu ist bevorzugt vorgesehen, ein- oder mehrfach invertierbare NMD-Kassetten, die mit einem bevorzugt deletierbaren Selektions- und/oder Reportergen verbunden sind, mit
- einem Verpackungssignal (Ψ) eines Retrovirus und
- einer "primer binding site" für eine tRNA und einem
- Polypurinabschnitt zu koppeln und
- die NMD-Kassette zusammen mit dem Verpackungssignal (Ψ), der PBS und dem Polypurinabschnitt mit "long terminal repeats" (LTRs) eines Retrovirus zu flankieren.

Dabei sind dem Fachmann bekannte Ausführungsformen als selbst-inaktivierende retrovirale Vektoren bevorzugt, die zum Verlust der U3 Region der LTRs und des Verpackungssignals (Ψ) nach der Integration ins Genom führt, so dass die inserierte Sequenz nicht erneut mobilisiert werden kann (Logan AC (2002): Advances in lentiviral vector design for genemodification of hematopoietic stem cells. Curr Opin Biotechnol 13(5):429-436)

Retroviraler Gentransfer bietet gegenüber der Transfektion von Genfallen den Vorteil, dass die dabei erzielten Effizienzen wesentlich höher sind, als die von Transfektionsmethoden.

Zur Herstellung von Viruspartikeln, die RNA erfindungsgemäßer Nukleinsäurekonstrukte enthalten, werden zunächst die entsprechenden Nukleinsäurekonstrukte in Verpackungszellinien transfiziert, die die für die Herstellung der Viruspartikel notwendigen Proteine exprimieren. Die von dem erfindungsgemäßen Konstrukt transkribierte RNA wird zusammen mit den für die reverse Transkription und Insertion notwendigen Proteinen in Virushüllproteine verpackt und von den Verpackungszellen als Viruspartikel freigesetzt.

Die Viruspartikel können dann zur Transduktion der Nukleinsäuren in eukaryontische Zellen benutzt werden. In diesen erfolgt die reverse Transkription in DNA und die Herstellung einer doppelsträngigen DNA, die dann zufällig in das Genom der Zielzelle inseriert. Wenn die Insertion in ein Intron eines Gens erfolgt, kann die NMD-Kassette nach rekombinasevermittelter Deletion des Selektionsmarkergens zur konditionalen Steuerung des getroffenen Gens genutzt werden.

Für den retroviralen Gentransfer sind erfindungsgemäße Nukleinsäurekonstrukte vorgesehen, in denen das Selektionsmarkergen als Exon ausgeführt ist und eine Resistenz nur vermittelt, wenn es im korrekten Leserahmen an ein Exon eines endogenen Gens der Zielzelle gespleißt wird. Bevorzugt sind für den retroviralen Gentransfer erfindungsgemäße Nukleinsäurekonstrukte vorgesehen, in denen die Transkription des Selektionsmarkergens von einem eigenen Promotor erfolgt und die NMD-Kassette selbst das einzige geninaktivierende Element ist. In diesem Fall muß der genaue Insertionsort mittels Plasmid Rescue oder geeigneten PCR-Methoden bestimmt werden, um die Klone zu ermitteln, in denen die Insertion in ein Intron erfolgte.

Für die Verwendung von NMD-Kassetten als Bestandteil von eukaryontischen Transposons ist vorgesehen, Nukleinsäurekonstrukte, die bevorzugt eine ein- oder mehrfach invertierbare NMD-Kassette enthalten und bevorzugt mit einem deletierbaren Selektionsmarkergen verbunden sind, mit
- endständigen Erkennungssequenzen für eine eukaryontische Transposase zu flankieren, wobei
- die Erkennungssequenzen für die Transposasen "sleeping beauty", und "Piggybac" (Bestor TH (2005): Transposons reanimated in mice. Cell 122 (3):322-325) sowie "frog prince" (Miskey C(2003): The Frog Prince: a reconstructed transposon from Rana pipiens with high transpositional activity in vertebrate cells. Nucleic Acids Res 31 (23):6873-81.) bevorzugt sind.

Dies ermöglicht der jeweiligen Transposase, NMD-Kassetten, die *in vivo* in der DNA einer Zelle integriert vorliegen, aus dem Genom auszuschneiden und an anderer Stelle wieder zufällig ins Genom zu integrieren, wobei ein neues mutiertes Allel eines anderen Gens erzeugt wird, wenn die erneute Insertion in einem anderen Gen erfolgt.

Für die Gentherapie dominanter Erbkrankheiten sieht die Erfindung in Fällen, in denen das dominante krankheitsverursachende Allel heterozygot vorliegt, einerseits die gleichen Nukleinsäurekonstrukte vor, die zur homologen Rekombination in ein Intron oder ein Exon eines Zielgens vorgesehen sind. Alternativ können für Verfahren, die auf der ungerichteten Mutagenese beruhen, erfindungsgemäße Nukleinsäurekonstrukte, die als Genfallen oder retrovirale Vektoren ausgeführt sind, zur Therapie benutzt werden. Bei den auf zufälliger Insertion beruhenden Verfahren sind NMD-Kassetten bevorzugt, die mindestens einmal invertiert werden können, damit auch solche Zellen für die vorgesehene Re-Transplantation genutzt werden können, in denen die Insertion in das mutierte Allel zunächst in der unwirksamen Orientierung erfolgte.

Für die Herstellung von Konstrukten zur gezielten Mutagenese sieht die Erfindung neben der herkömmlichen Klonierung von Restriktionsfragmenten und dem auf homologer Rekombination in *E .coli* beruhenden ET-Klonieren die Herstellung von für die homologe Rekombination in eukaryontische Zielgene geeigneten Vektoren durch ungerichtete Transposon-Mutagenese *in vitro* und *in vivo* vor.

Dazu werden bevorzugt ein- oder mehrfach invertierbare NMD-Kassetten, die
- mit einem deletierbaren, in Eukaryonten und Prokaryonten selektierbaren Resistenzgen verbunden sind,
- an den Enden mit Erkennungssequenzen für eine prokaryontische Transposase flankiert,
- so dass diese mit der NMD-Kassette und dem damit verbundenen Resistenzgen ein synthetisches prokaryontisches Transposon bilden,
- wobei Erkennungssequenzen für die Transposasen Mu, Tn5 und besonders EZ-Tn5 bevorzugt sind.
   Optional ist dabei die Verbindung des in solchen Nukleinsäurekonstrukten enthaltenen Resistenzgens mit einem prokaryontischen Replikationsursprung vorgesehen, wobei dieser unabhängig von zusätzlichen Faktoren in *E. coli* aktiv sein kann, wie z.B. der ColE1 Replikationsursprung, oder nur in Anwesenheit eines zusätzlichen Faktors aktiv ist, wie z.B. der R6K Replikationsursprung.
   Die Erfindung sieht außerdem *die in vitro* Erzeugung von DNA-Protein-Komplexen vor, sogenannte Transposomen, bestehend aus einem erfindungsgemäßen prokaryontischen Transposon und der für die darin enthaltenen Erkennungssequenzen spezifischen Transposase. Diese Transposomen sind geeignet, das die NMD-Kassette beinhaltende Transposon *in vitro* oder in *E. coli* in klonierte genomische Fragmente eines Zielorganismus zu inserieren.
   Weiterhin sieht die Erfindung Nukleinsäurekonstrukte vor, die genomische Fragmente eines Zielorganismus enthalten, in denen sich eine durch herkömmliche Klonierung, homologe Rekombination in *E. coli* oder durch Transposition inserierte erfindungsgemäße NMD-Kassette befindet.
   Da in der bevorzugten Ausführungsform die Nonsense vermittelte Degradation der RNA nur abhängig von einem genügenden Abstand (>50 Nukleotide) des prämaturen Stopkodons von der nächsten stromabwärts folgenden Spleißdonorstelle und ansonsten unabhängig von der Lokalisation des prämaturen Stopkodons in der mRNA sowie von der jeweiligen Intronphase ist, ist die Wirkung der NMD-Kassette davon unabhängig, in welches Intron des Zielgens die Insertion stattgefunden hat. Dies ermöglicht wesentlich größere Spielräume bei der Planung von Konstrukten zur gezielten Mutagenese als herkömmliche Methoden. Außerdem erlaubt dies, erfindungsgemäße Konstrukte für Anwendungen zu nutzen, die auf zufälliger Integration in ein Zielgen beruhen, z.B. als Teil von umfänglicheren Nukleinsäurekonstrukten, die als Teil von Genfallen oder Transposons fungieren. Um erfindungsgemäße Nukleinsäurekonstrukte auch für Gene ohne Introns und intronhaltige Gene mit nur einem kodierenden Exon nutzbar zu machen, sieht die Erfindung außerdem Konstrukte vor, in denen die NMD-Kassette von zusätzlichen intronischen Bereichen flankiert wird, die zusammen mit Protospleißstellen am Insertionspunkt im Exon funktionale Spleißstellen ergeben. Dies führt dazu, dass das Zielgen anschließend aus mehreren kodierenden Exons besteht und entsprechende Transkripte dem Spleißen unterliegen und bei Lokalisation des NMD vermittelnden Exons im kodogenen Strang zur Degradation der entsprechenden RNA führen.
   Die vorliegende Erfindung bietet den Vorteil, die Inaktivierung eines Zielgens durch Insertion eines erfindungsgemäßen Nukleinsäurekonstrukts in ein Intron oder Exon einer Zelle im wesentlichen unabhängig von der individuellen Herkunft dieser Zelle zu ermöglichen. Im Unterschied dazu hat die Unterdrückung der Expression eines Gens durch spezifische RNA-Interferenz mit z.B. shRNAs den Nachteil, dass möglicherweise individuelle Punktmutationen bei einigen Individuen die Hybridisierung und Unterdrückung durch nicht hybridisierende Nukleotide abschwächen.
   Die Wirkung einer NMD-Kassette ist im wesentlichen unabhängig von der Methode, mit der sie in ein Zielgen inseriert wird. Die Erfindung sieht dazu die gezielte Mutagenese von Zielgenen mittels homologer Rekombination von NMD-Kassetten beinhaltenden Targetingkonstrukten sowie die zufällige Mutagenese von Genen durch die Insertion von NMD-Kassetten beinhaltenden Genfallen, retroviraler Vektoren oder Transposons vor.
   Der Einsatz erfindungsgemäßer NMD-Kassetten in Genfallen, retroviralen Vektoren und Transposons bietet den Vorteil, dass die Anwendung der Erfindung nicht auf Zellen, z.B. murine ES-Zellen, beschränkt ist, in denen ein gezielte Mutagenese möglich ist, und die es außerdem ermöglichen, aus solchen Zellen einen kompletten Organismus, z.B. eine Maus, zu generieren. Deshalb sieht die Erfindung eine Nutzung in allen eukaryontischen Zellen vor, die Spleißen von prä-mRNA und NMD aufweisen, also auch z.B. pflanzliche Zellen.
   Außerdem sieht die Erfindung vor, aus erfindungsgemäß hergestellten nicht-menschlichen Zellen vollständige Organismen, also z.B. Tiere und Pflanzen oder andere Eukaryonten herzustellen.
   Mit einer der genannten Methoden transformierte Zellen können durch Selektion auf das in allen Ausführungsformen mit der NMD-Kassette verbundene Selektionsmarkergen, z.B. ein Resistenzmarkergen, selektiert werden. Nach Selektion transformierter Zellen lässt sich das Selektionsmarkergen entfernen, beispielsweise durch Expression, Einbringen und/oder Aktivieren einer Rekombinase, die mit Erkennungsstellen für die Rekombinase, die das Resistenzmarkergen flankieren, in Wechselwirkung tritt und ausschließlich das Resistenzmarkergen entfernt. Vorzugsweise ist daher das Resistenzmarkergen von Erkennungssequenzen für eine andere Rekombinase flankiert, als sie die NMD-Kassette aufweist.
   Unabhängig von der Methode mit denen eine NMD-Kassette in Zielgene inseriert wurde sieht die Erfindung optional eine Flankierung der NMD-Kassette mit einem oder mehreren Paaren von Erkennungssequenzen für Erkennungssequenzen für eine oder mehrere unterschiedliche Rekombinasen vor. Dies bietet den erfindungsgemäßen Vorteil, eine einfache oder mehrfache konditionale Änderung des Aktivitätszustandes des Gens zu erlauben, in das eine NMD-Kassette inseriert wurde. Dabei kann die NMD-Kassette zunächst in den kodogenen Strang eines Zielgens inseriert werden und dieses zunächst inaktivieren. Alternativ kann die initiale Orientierung der inserierten Kassette so sein, dass die NMD-Kassette im nicht-kodogenen Strang lokalisiert ist und die Expression des betroffenen Gens zunächst unbeeinflußt bleibt. Durch Inversion mittels einer der Rekombinasen, deren Erkennungsequenzen die NMD-Kassette flankieren, kann der jeweilige Aktivitätszustand geändert werden, indem die NMD-Kassette in den jeweils anderen Strang des Zielgens überführt wird. In einer Ausführungsvariante zur Reaktivierung eines durch eine NMD-Kassette zunächst inaktivierten Gens kann alternativ die im kodogenen Strang lokalisierte NMD-Kassette mittels einer geeigneten Rekombinase deletiert werden.
   Die Herstellung von Targetingkonstrukten mit erfindungsgemäßen NMD-Kassetten für die gezielte Mutagenese bietet den Vorteil, dass diese Konstrukte mittels eines Klonierungsschrittes herstellbar sind und ein Nukleinsäurekonstrukt dafür ausreicht. Demgegenüber sind für die Herstellung von Targetingkonstrukten nach den bekannten Verfahren immer mehrere Klonierungsschritte und mehrere unterschiedliche Nukleinsäurekassetten notwendig. Als Klonierungsverfahren für die Verwendung von NMD-Kassetten werden herkömmliche Klonierungstechniken mittels Restriktionsverdau, Ligation und Transformation in geeignete *E*. *coli* Stämme sowie Klonierungen mittels homologer Rekombination in *E*. *coli* (ET-Klonierung, Recombineering) bevorzugt.
   Im Fall einer gezielten Mutagenese von Zielgenen, die kein Intron aufweisen, oder von intronhaltigen Genen, die zwar mehrere Exons aufweisen, von denen aber nur eins kodierend ist, sieht die Erfindung die Insertion erfindungsgemäßer Nukleinsäurekonstrukte in das jeweils kodierende Exon vor. Wenn das NMD vermittelnde Exon eines erfindungsgemäßen Nukleinsäurekonstrukts, in dem die NMD-Kassette von zusätzlichen intronischen Bereichen flankiert wird, nach der Insertion im kodogenen Strang des Gens lokalisiert ist und die im dritten und vierten Intronabschnitt enthaltenen endständigen intronischen Anteile einer Spleißdonorstelle bzw. einer Spleißakzeptorstelle zusammen mit einer Protospleißstelle am Insertionspunkt im Exon des Zielgens funktionale Spleißstellen ergeben, besteht das Zielgen anschließend aus mehreren kodierenden Exons. Entsprechende Transkripte unterliegen dem Spleißen und der Pionierrunde der Translation, was wiederum zur Degradation der RNA führt. Dies bietet gegenüber anderen Verfahren den erfindungsgemäßen Vorteil, dass bei der Herstellung konditionaler Mutanten regulatorische Sequenzen, wie Promotoren, 5'- und 3'-UTRs sowie Polyadenylierungssignale, die die kodierenden Sequenzen von Ein-Exon-Genen flankieren, nicht manipuliert werden müssen und somit ungünstige Effekte solcher Manipulationen vermieden werden.
   Das gezielte Einbringen einer NMD-Kassette in ein bestimmtes Zielgen in einer eukaryontischen Zelle durch den Mechanismus der homologen Rekombination wird durch zusätzliche DNA-Abschnitte erreicht, die mit der NMD-Kassette verbunden sind, diese flankieren und eine für die homologe Rekombination hinreichende Homologie zu den den gewünschten Insertionsort flankierenden Sequenzen des Zielgens sowie eine für die homologe Rekombination hinreichende Länge aufweisen.
   In einer bevorzugten Ausführungsform für die gezielte Mutagenese werden mit der NMD-Kassette verbundene Sequenzen, die zum Zielgen homolog sind, so gewählt, dass die Insertion in ein Intron des gewünschten Zielgens erfolgt, das nicht von alternativem Spleißen betroffen ist. Denn das so genannte alternative Spleißen kann dazu führen, dass die erfindungsgemäße Wirkung der NMD-Kassette, einen Nonsense vermittelten Abbau der prä-mRNA zu verursachen, verhindert oder abgeschwächt wird.
   In einer Variante der Ausführungsformen erfindungsgemäßer Nukleinsäurekonstrukte zur gezielten Mutagenese sieht die Erfindung die Verwendung von NMD-Kassetten vor, in denen zumindest eines von Spleißakzeptor- und Spleißdonorstelle, dem Polypyrimidinabschnitt und dem Verzweigungspunkt in 5' von der Spleißakzeptorstelle eine verringerte Aktivität aufweisen. Die Verwendung solcher am Spleißen beteiligter Elemente mit verminderter Funktionsfähigkeit erlaubt es, ein unvollständiges Spleißen zu erzeugen, wenn eine NMD-Kassette in aktiver Orientierung, d.h. im kodogenen Strang angeordnet ist, indem das NMD vermittelnde Exon anteilig mit den benachbarten Introns entfernt wird, und somit zur unvollständigen NMD und daher zu einem hypomorphen Phänotyp führt. Diese Option, die Expression eines Zielgens anteilig zu verringern, ist vorteilhaft, da sie mit herkömmlichen Methoden nicht geplant werden kann.
   Die Erfindung sieht für die gezielte Mutagenese von Zielgenen Nukleinsäurekonstrukte vor, die die NMD-Kassette zunächst gezielt entweder in den kodogenen oder den in den nicht-kodogenen Strang des Zielgens inserieren. Im erstgenannten Fall ist das Allel, in das die NMD-Kassette homolog rekombiniert wurde, inaktiviert, da entsprechende Transkripte das NMD vermittelnde Exon enthalten. Bei der Lokalisation der NMD-Kassette im nicht-kodogenen Strang bleibt die Expression des betroffenen Allels zunächst unverändert. In Verbindung mit der optional vorgesehenen Flankierung der NMD-Kassette mit Rekombinaseerkennungssequenzen sieht die Erfindung eine anschließende Änderung des Aktivitätszustandes entsprechender Allele mittels rekombinasevermittelter Invertierung oder Deletion der NMD-Kassette bzw. eine mehrfache Invertierung mittels mehrerer Rekombinasen vor. Dies bietet gegenüber herkömmlichen Verfahren den wesentlichen Vorteil, dass nicht nur konditional inaktivierbare Allele erzeugt werden können, sondern auch konditional reaktivierbare Allele sowie Allele mit mehrfach konditional veränderbarem Aktivitätszustand hergestellt werden können.
   Als Zielzellen für die gezielte Mutagenese sind besonders kultivierte eukaryontische Zellen bevorzugt, die endogen eine vergleichsweise hohe Frequenz homologer Rekombination aufweisen und direkt zur Herstellung ganzer Organismen mit der eingeführten Mutation geeignet sind, wie z.B. murine ES-Zellen. Eine gezielte Mutagenese mit erfindungsgemäßen Nukleinsäurekonstrukten kann aber auch in Zellen erfolgen, in denen die Frequenz homologer Rekombination künstlich erhöht wurde, und aus denen ganze Organismen nur durch Klonierung, z.B. mittels Kerntransfer in eine entkernte Eizelle, erzeugt werden können. Außerdem sieht die Erfindung die gezielte Mutagenese mittels erfindungsgemäßer Nukleinsäurekonstrukte in allen eukaryontischen Zellen vor, die Spleißen und NMD aufweisen.
   Als Alternative zur Herstellung von Targetingvektoren für die gezielte Mutagenese, die durch homologe Rekombination in eukaryontischen Zellen erreicht wird, sieht die Erfindung neben herkömmlichen Klonierungsverfahren und Verfahren, die auf homologer Rekombination in E. *coli* beruhen (ET-Klonierung), die Herstellung einzelner Targetingvektoren und die Herstellung von Bibliotheken von Targetingvektoren mittels Transposition *in vitro* oder *in vivo* in geeigneten Bakterien (z.B. *E. coli)* vor.
   Da ein einziger Klonierungsschritt für die Herstellung von Targetingkonstrukten mit erfindungsgemäßen NMD-Kassetten ausreichend und die Wirkung der NMD-Kassette unabhängig von der jeweiligen Intronphase ist und davon, in welchem Intron des Zielgens sie inseriert wird, bieten erfindungsgemäße Nukleinsäurekonstrukte, in denen die NMD-Kassette von Erkennungssequenzen für eine prokaryontische Transposase flankiert wird, den wesentlichen Vorteil, dass sie mittels Transposase-vermittelter zufälliger Insertion in klonierte genomische Fragmente des Zielgens zur Herstellung von Targetingvektoren genutzt werden können. Ausführungsvarianten von mit Transposaseerkennungssequenzen flankierten Nukleinsäurekonstrukten, in denen die NMD-Kassette mit Erkennungsequenzen für mindestens eine Rekombinase flankiert ist, bieten zusätzlich den Vorteil, dass die Orientierung der NMD-Kassette nachträglich geändert werden kann, wenn die ursprüngliche Orientierung nicht den experimentellen Anforderungen genügt. Z.B. kann eine NMD-Kassette, die durch die Transposition zunächst in den kodogenen Strang eines Gens in einem klonierten genomischen Fragment inseriert wurde, vor der Nutzung des Vektors zur Transfektion einer eukaryontischen Zelle *in vitro* invertiert werden, indem das Konstrukt unter geeigneten Bedingungen mit der entsprechenden Rekombinase kontaktiert wird. Alternativ kann die benötigte Inversion erreicht werden, indem das Konstrukt in einen Bakterienstamm transformiert wird, der die benötigte Rekombinase induziert exprimiert, z.B. in die *E. coli* Stämme 294Cre oder 294flp.
   Zur Herstellung von Targetingvektoren mittels Transposition bevorzugt die Erfindung besonders in herkömmliche Klonierungsvektoren, wie z.B. pBluescript, und in bacterial artificial chromosomes (BACs) klonierte genomische Fragmente. Alternativ sind aber auch Gemische nicht-klonierter genomischer Fragmente zum Einsatz vorgesehen. Dabei sieht die Erfindung vor genomische Fragmente aus dem Organismus zu benutzen, in dem die gezielte Mutagenese erfolgen soll.
   Zur Herstellung von Targetingvektoren mittels Transposition bevorzugt die Erfindung die Verwendung sogenannter Transposomen. Ein Transposom besteht aus einem als Transposon ausgeführten DNA-Fragment, das an den Enden von Erkennungssequenzen für eine Transposase flankiert ist, z.B. die "19 bp mosaic end sequences" (19ME), an die die geeignete Transposase, im Falle von 19ME Sequenzen die Transposase EZ-Tn5 , *in vitro* gebunden wurde.
   Die Erfindung sieht vor, dass das in einem Transposom enthaltene DNA-Fragment eine erfindungsgemäße NMD-Kassette enthält, bevorzugt flankiert von einem oder mehreren Paaren von Erkennungssequenzen für eine oder mehrere Rekombinase, und die NMD-Kassette an ein Resistenzgen gekoppelt vorliegt, welches von anderen Erkennungsequenzen für eine Rekombinase oder Integrase flankiert wird und optional einen prokaryontischen Replikationsursprung enthalten kann. Dabei sieht die Erfindung zum einen die Verwendung eines Replikationsursprungs vor, z.B. ColE1, für dessen Aktivität alle notwendigen Faktoren in herkömmlichen *E*. *coli*-Stämmen vorhanden sind und deshalb die Replikation eines Plasmids, in dem er enthalten ist, unabhängig von zusätzlichen Faktoren vermittelt. Alternativ ist die Verwendung eines Replikationsursprungs vorgesehen, der nur in Zellen aktiv ist, die einen für die Aktivität des Replikationsursprungs notwendigen Faktor exprimieren, wie es z.B. im Fall des R6K Replikationsursprungs beschrieben wurde, der in *E*. *coli* von der Expression des Faktors pir abhängig ist.
   Für die Verwendung von genomischen Fragmenten in herkömmliche Klonierungsvektoren, die einen eigenen Replikationsursprung besitzen, und bei der Verwendung vollständiger BACs als Targetingvektoren, wird die Verwendung von Transposomen ohne Replikationsursprung bevorzugt. Die Insertion der mit einem Resistenzgen gekoppelten und zusammen mit diesem von Transposaseerkennungssequenzen flankierten NMD-Kassette erfolgt, indem das die NMD-Kassette beinhaltende DNA-Fragment zunächst mit der Transposase inkubiert wird, so dass diese an die endständigen Transposaseerkennungssequenzen des DNA-Fragment binden kann. Die so erzeugten Transposomen werden dann mit einem Gemisch isolierter Vektoren gemischt und weiter inkubiert, wobei die Transposase die in den Transposomen enthaltenen DNA-Fragmente in die Vektoren inseriert. Nach Beendigung der Transposasereaktion wird das DNA-Gemisch in geeignete *E*. *coli* Stämme transformiert. Die Transformanten werden dann mit für das in dem Transposom enthaltene Resistenzgen geeigneten Antibiotikum selektiert. Überlebende Klone enthalten nur ursprünglich in dem verwendeten Vektorgemisch enthaltene Vektoren, in die das in dem Transposom enthaltene DNA-Fragment inseriert wurde, da Vektoren ohne Insertion nicht die notwendige Resistenz vermitteln können und nicht inserierte Transposons wegen des Fehlens eines eigenen Replikationsursprungs und aufgrund ihrer linearen Form nicht repliziert werden können.
   Das Vorhandensein eines prokaryontischen Replikationsursprungs in erfindungsgemäßen prokaryontischen Transposons bietet den weiteren wesentlichen Vorteil, dass diese nach erfolgter Insertion in eine klonierte genomische DNA oder in ein lineares, nicht kloniertes genomisches Fragment, direkt zusammen mit den genomischen Sequenzen, die den jeweiligen Insertionspunkt des Transposons flankieren, subkloniert werden können. Dazu sieht die Erfindung vor, das ein Gemisch von Vektoren, die genomische Fragmente enthalten oder ein Gemisch, das lineare genomische Fragmente des Organismus, in dem später die gezielte Mutagenese durchgeführt werden soll, zunächst mit erfindungsgemäßen Transposomen inkubiert werden, die als Transposons ausgeführte DNA-Fragmente enthalten, die neben der NMD-Kassette und einem Resistenzgen einen mit diesen verbundenen prokaryontischen Replikationsursprung besitzen. Nach erfolgter Insertion sieht die Erfindung vor, die DNA aus der Reaktion aufzureinigen und anschließend mit einem Restriktionsenzym zu verdauen, das nicht innerhalb des in dem Transposom verwendeten DNA-Fragmentes schneidet. Dies bietet den Vorteil, dass Fragmente entstehen, die an beiden Enden die gleichen Restriktionsschnittstellen in ihrer geschnittenen Form aufweisen. Diese Fragmente können durch Ligation zirkularisiert werden und bilden so in Bakterien replizierbare und selektierbare Plasmide, in denen eine NMD-Kassette sowie das mit ihr verbundene Resistenzgen und der Replikationsursprung von zwei genomischen Fragmenten flankiert sind, die durch eine Restriktionsschnittstelle verbunden sind, die durch die Ligation der ursprünglich an den Enden lokalisierten geschnittenen Restriktionsschnittstellen entstanden ist.
   Durch Schneiden der Plasmide an dieser Restriktionsschnittstelle lassen sich die Plasmide vor der Transfektion für die gezielte Mutagenese wieder linearisieren, wobei das eine Ende des entstehenden linearen DNA-Fragmentes dem 5'-Endes eines genomischen DNA-Fragmentes entspricht, dass bei dem Verdau genomischer DNA mit demselben Restriktionsenzym entstehen würde, und das andere Ende dem 3'-Ende des genannten genomischen Restriktionsfragmentes.
   In Fortbildung sieht die Erfindung die Nutzung erfindungsgemäßer Transposons und Transposomen *in vivo* in Bakterien zur Herstellung von Targetingvektoren für die gezielte Mutagenese vor. Dazu werden bevorzugt erfindungsgemäße Transposomen in Bakterien elektroporiert, die klonierte genomische Fragmente des Organismus enthalten, in dem später die gezielte Mutagenese erfolgen soll. Durch Selektion für das in den Transposomen enthaltenene Resistenzgen werden Klone angereichert, die die NMD-Kassette zusammen mit dem Resistenzgen in das Plasmid bzw. das BAC oder die bakterielle DNA inseriert haben. Um die bakterielle DNA zu eliminieren, ist anschließend eine Aufreinigung der Plasmid- bzw. BAC-DNA und optional eine Re-Transformation derselben in ein frisches Aliquot von Bakterien vorgesehen, die vorher nicht mit Transposomen in Kontakt waren. Soll die *in vivo* Verwendung von Transposomen mit einer anschließenden Subklonierung gekoppelt werden, so sieht die Erfindung die Verwendung eines nicht-autonomen Replikationsursprungs, z.B. R6K, vor, um eine Interferenz mit dem jeweiligen in Klonierungsvektoren und BACs vorhandenen Replikationsursprung zu verhindern. Die Subklonierung erfolgt dann wie oben beschrieben durch Restriktionsverdau mit einem Enzym, das nicht in den Sequenzen des Transposons schneidet, sowie anschließender Zirkularisierung durch Ligation. Die anschließende Transformation erfolgt dann bevorzugt in Bakterienstämme, die den für die Aktivität des in dem Transposon beinhalteten Replikationsursprungs notwendigen Faktor exprimieren, z.B. im Fall von R6K den Faktor pir.
   In einer Abwandlung des beschriebenen Verfahrens zur Herstellung von Targetingvektoren für die gezielte Mutagenese mittels Transposition *in vivo* sieht die Erfindung die Elektroporation von Transposons, die vorher nicht mit der Transposase kontaktiert wurden, in Bakterien vor, die klonierte genomische Fragmente enthalten und die Transposase von einem Plasmid oder von einer stabil ins Genom integrierten Kopie konstitutiv oder induziert exprimieren. Bei diesem Verfahren erfolgt die Reaktion des Transposons mit der Transposase sowie die Bildung eines Transposoms also erst *in vivo.* Die weiteren Reaktionen und Verfahren entsprechen dem für die Elektroporation von Transposomen beschriebenen Vorgehen.
   Da die Insertion erfindungsgemäßer Transposons zur Herstellung von Targetingvektoren für die gezielte Mutagenese zufällig und ungerichtet erfolgt, sieht die Erfindung vorzugsweise vor, den Insertionspunkt und die Orientierung eines Transposons in dem klonierten genomischen Fragment mittels Sequenzierung festzustellen.
   In Fällen, in denen die Orientierung des inserierten Transposons nicht der geplanten Mutagenese entspricht, ist vorgesehen, die Orientierung mittels der Reaktion einer Rekombinase mit ihren Erkennungssequenzen, die die NMD-Kassette flankieren, nachträglich zu ändern. Dies kann zum einen geschehen, indem das betreffende Targetingkonstrukt in Bakterien transformiert wird, die die Rekombinase exprimieren. Alternativ kann diese Reaktion auch *in vitro* durchgeführt werden, indem das Targetingkonstrukt mit der Rekombinase inkubiert und anschließend in geeignete Bakterien transformiert wird.
   Als weitere Alternative sieht die Erfindung vor, die Orientierung der NMD-Kassette nach erfolgter homologer Rekombination in dem Zielorganismus zu ändern, indem in diesem die Rekombinase exprimiert wird.
   Als Alternative zur gezielten Mutagenese sieht die Erfindung die Verwendung erfindungsgemäßer Nukleinsäurekonstrukte zur zufälligen Mutagenese vor. Bevorzugt ist dabei die Ausführung erfindungsgemäßer Nukleinsäurekonstrukte als Genfallen. Dies bietet den Vorteil, dass die Anwendung erfindungsgemäßer Nukleinsäurekonstrukte auch in Zellen erfolgen kann, in denen eine gezielte Mutagenese aufgrund des Fehlens oder eines nur geringen Maßes an homologer Rekombination nicht möglich ist. Außerdem bietet die Ausführung erfindungsgemäßer Nukleinsäuren als Genfallen den Vorteil, dass mit ihnen Bibliotheken mutierter Zellen hergestellt werden können, aus denen diejenigen ausgewählt werden können, in denen das jeweils interessierende Gen mutiert wurde.
   Im Unterschied zu Nukleinsäurekonstrukten zur gezielten Mutagenese weisen solche zur Verwendung als Genfalle im wesentlichen keine Bereiche großer Homologie mit Bereichen des Zielgenoms auf, so dass deren Integration zufällig erfolgt. Um bei der Insertion ins Genom einen Verlust der für die inaktivierende Wirkung der NMD-Kassette und für die Konditionalität entsprechender Konstrukte notwendigen Sequenzen zu verhindern, weisen als Genfallen ausgeführte erfindungsgemäße Konstrukte an den Enden zusätzliche Sequenzen auf, die keine Spleißsignale enthalten.
   In der bevorzugten Ausführungsform erfindungsgemäßer Genfallen wirkt die NMD-Kassette selbst als inaktivierendes Element und die Selektion für die Integration des Konstruktes wird durch ein positiv selektierbares Resistenzgen mit eigenem eukaryontischem Promoter und einer eigenen funktionalen Polyadenylierungsequenz vermittelt. In dieser Ausführungsform ist die Verwendung von Resistenzgenen bevorzugt, die sowohl in Eukaryonten als auch in Prokaryonten selektiert werden können. Alternativ können ein prokaryontisch und ein eukaryontisch selektierbares Resistenzgen auf einem gemeinsamen DNA-Abschnitt verbunden werden. Weiterhin ist bevorzugt, das Resistenzgen zusätzlich mit einem prokaryontischen Replikationsursprung (ori) zu verbinden und beide mit Erkennungssequenzen für eine Rekombinase oder Integrase zu flankieren, die eine Deletion des Resistenzgens zusammen mit dem Replikationsursprung im Genom der Zielzelle ermöglichen. Derartige Nukleinsäurekonstrukte ermöglichen es, aus genetisch manipulierten Zielzellen genomische Fragmente zu isolieren, die eine erfindungsgemäße Genfalle beinhalten. Dies geschieht, indem die genomische DNA der mutierten Zellen mittels Restriktionsenzymen, die nicht innerhalb des verwendeten erfindungsgemäßen Nukleinsäurekonstrukts schneiden, fragmentiert und anschließend durch Ligation zirkularisiert werden. Diese so erzeugten zirkularisierten Fragmente verhalten sich wie Plasmide und können durch Transformation in geeignete Bakterien, z.B. *E*. *coli,* und geeignete Selektion vermehrt werden, da sie die zur Amplifikation und Selektion in Prokaryonten erforderlichen funktionalen Elemente eines Plasmids aufweisen. Durch dieses auch als Plasmid Rescue bezeichnete Verfahren steht genügend DNA zur Verfügung, um den Insertionspunkt und damit ein gegebenenfalls getroffenes Gen durch Sequenzierung sequenzgenau zu bestimmen. Dazu werden aus der inserierten NMD-Kassette mit Primern, die innerhalb der NMD-Kassette hybridisieren, in die den Insertionspunkt flankierenden unbekannten Sequenzen hineinsequenziert.
   Alternativ können klonierungsfreie PCR-Techniken, die dem Fachmann bekannt sind, zur Bestimmung des jeweiligen Insertionspunktes benutzt werden, z.B. das als "genome walking" bekannte Verfahren.
   Gegenüber herkömmlichen als Genfallen verwendeten Nukleinsäurekonstrukten bietet das bevorzugte Verfahren, bei dem eine erfindungsgemäße NMD-Kassette als inaktivierendes Element wirkt, den Vorteil, dass für ihre Integration unabhängig von der Expression des Gens, in das sie inserierte, selektiert werden kann. Außerdem bieten erfindungsgemäße Konstrukte in dieser Ausführungsform gegenüber herkömmlichen Genfallen den Vorteil, dass sie unabhängig von der Intronphase und unabhängig davon funktionieren, in welches der Introns eines Gens sie inseriert wurden. Deshalb reicht ein erfindungsgemäßes Nukleinsäurekonstrukt in der bevorzugten Ausführungsform als Genfalle für alle intronhaltigen Gene eines Organismus aus, während bei der Verwendung herkömmlicher Genfallen insgesamt drei Konstrukte, jeweils eins für jede Intronphase, benötigt werden
   Weiterhin bietet die bevorzugte Ausführungsform mit zumindest einmal mittels einer Rekombinase invertierbarer NMD-Kassette den Vorteil, dass diese in die wirksame Orientierung überführt werden kann, wenn sie sich ursprünglich im nicht-kodogenen Strang des getroffenen Gens befand. Die Verwendung mehrfach mittels verschiedener Rekombinasen invertierbarer NMD-Kassetten in erfindungsgemäßen Genfallen bietet darüber hinaus den Vorteil, dass ein durch die Genfalle zunächst inaktiviertes Gen zunächst konditional reaktiviert und später wieder konditional inaktiviert, bzw. ein zunächst nicht inaktiviertes Gen zunächst konditional inaktiviert und nachträglich wieder konditional reaktiviert werden kann. Die mehrfache Invertierbarkeit bietet außerdem den Vorteil, dass jede Zelle, in der eine erfindungsgemäße Genfalle der bevorzugten Ausführungsform in ein Gen inseriert wurde, für weitere Arbeiten benutzt werden kann. Da in Mäusen der Anteil von Introns 20% des gesamten Genoms ausmacht, bietet die Erfindung den Vorteil, dass jede fünfte resistente Zelle nach der Transfektion einer Genfalle der bevorzugten Ausführungsform eine Kopie der Genfalle in einem funktionalen Gen enthält.
   In alternativer Ausführungsform weisen zur Verwendung als Genfalle vorgesehene erfindungsgemäße Nukleinsäurekonstrukte neben der NMD-Kassette, die vorzugsweise von mindestens einem Paar Erkennungssequenzen für Rekombinasen flankiert ist, ein Selektionsmarkergen auf, das von Erkennungssequenzen für eine andere Rekombinase flankiert ist als die, deren Erkennungssequenzen die NMD-Kassette flankieren. Die Erfindung sieht in dieser Ausführungsform vor, dass das Resistenzgen unvollständig ist und erst durch die Insertion in Gen durch endogene Bestandteile des Gens funktionsfähig wird. Z.B. kann ein unvollständiges Resistenzgen als terminales Exon ausgeführt sein. D.h. dass das 5'-Ende der kodierenden Sequenz des Resistenzgens von einem Spleißakzeptor mit 5' davon gelegenem Polypyrimidinabschnitt und Verzweigungspunkt gebildet wird und das stromabwärts vom Stopkodon der kodierenden Sequenz ein funktionales Polyadenylierungssignal angeordnet ist. Als Resistenzgene können dabei positiv und/oder negativ selektierbare Selektionsmarkergene mit der NMD-Kassette gekoppelt werden.
   Zusätzlich ist auch eine Kopplung des Resistenzgens mit einem Reportergen möglich, z.B. in Form einer ein Fusionsprotein aus Reportergen- und Resistenzgenprodukt kodierenden Sequenz oder in Form einer bicistronischen Kassette, bei der die Translation des 3' gelegenen Cistrons durch eine IRES vermittelt wird. Als Reportergene sind dabei kodierende Sequenzen für ein fluoreszierendes Protein (GFP, eGFP, RFP, etc.) oder solche, die ein Substrat umwandeln (z.B. β-Galactosidase) vorgesehen. Vorzugsweise ist das Resistenzmarkergen und ein optional damit gekoppeltes Reportergen gemeinsam von einem anderen Paar Erkennungssequenzen flankiert als die NMD-Kassette.
   Da die Funktionalität solcher Konstrukte wie bei der Verwendung herkömmlicher Genfallen von der Insertion in der korrekten Intronphase abhängig ist, sieht die Erfindung drei entsprechende Konstrukte mit Anordnung des Resistenzgens in jeweils einer der möglichen Intronphasen vor.
   Bei Integration erfindungsgemäßer Genfallen mit unvollständigem Resistenzgen stromabwärts eines endogenen Promotors der Zielzelle in ein Intron in der richtigen Orientierung und in der dem Konstrukt entsprechenden Intronphase wird das Resistenzgen komplettiert, indem es als terminales Exon in den Leserahmen des Zielgens gelangt. Durch das in der Genfalle beinhaltete Polyadenylierungssignal enthalten Transkripte des Zielgens keine stromabwärts der Genfalle lokalisierten Exons des getroffenen Gens, so dass zumindest die von diesen Exons vermittelten Funktionen des Produktes des Zielgens eliminiert sind. Bei der Verwendung von an das Resistenzgen gekoppelten Reportergenen können so hergestellte Zellen direkt zur Herstellung von Organismen benutzt werden, in denen die Expression des getroffenen Zielgens anhand des Expressionsmusters des Reportergens ermittelt werden kann. Für den Fall, dass ein positiv und negativ selektierbarer Selektionsmarker verwendet wird, kann durch negative Selektion zunächst auch gegen Zellen selektiert werden, die ein erfindungsgemäßes Nukleinsäurekonstrukt in passender Anordnung stromabwärts von in ES-Zellen aktiven endogenen Promotorelementen aufweisen. Eine anschließende Differenzierung von Aliquots von Transformanten zusammen mit der geeigneten positiven Selektion, kann dann zur Identifikation von Klonen genutzt werden, bei denen die Integration in einen Locus erfolgt ist, der im Laufe der Differenzierung aktiviert wird. Die nicht differenzierten Aliquots können dann zur Herstellung ganzer Organismen aus den identifizierten Klonen benutzt werden.
   Die Geninaktivierung mittels Genfallen, die auf der Komplettierung eines Resistenzgens beruhen, ist häufig nicht vollständig, wenn die Genfalle in eines der weiter 3' gelegenen Introns eines Gens inseriert wurde, da dann die entsprechenden Genprodukte noch einen Großteil der von 5' von der Genfalle lokalisierten Exons kodierten Anteile enthalten, die noch in der Lage sind, wenigstens einen Teil der Genfunktionen zu erfüllen. Deshalb sieht die Erfindung vor, dass das in den Nukleinsäurekonstrukten beinhaltete Resistenzgen bzw. das Resistenzgen und ein optional damit gekoppeltes Reportergen mittels der für die die genannten Elemente flankierenden Erkennungssequenzen spezifischen Rekombinase oder Integrase zu deletieren und somit aus dem Zielgen zu entfernen. Im Ergebnis gelangt so die NMD-Kassette in eine Position innerhalb des Gens, in der sie, abhängig von der jeweiligen Orientierung, in der sie sich in Bezug auf das Resistenzgen befand, sofort inaktivierend wirkt oder zunächst unwirksam im nicht-kodogenen Strang des Zielgens verbleibt. Bevorzugt ist dabei die gleiche Orientierung von Resistenzgen und dem NMD vermittelnden Exon, d.h. beide befinden sich im gleichen Strang des als Genfalle verwendeten Konstrukts. Dieses wird bei der Insertion mit dem kodogenen Strang des Zielgens verbunden, da andernfalls keine Selektion für die Insertion möglich wäre. Durch die optional vorgesehene Möglichkeit zur Deletion des Resistenzgens bzw. der gemeinsamen Deletion des Resistenzgens und eines damit verbundenen Reportergens ergibt sich so gegenüber herkömmlichen Genfallen der Vorteil, dass durch das Resistenzgen bzw. das Reportergen eventuell nicht vollständig inaktivierte Gene einer kompletten Inaktivierung durch die NMD-Kassette zugeführt werden können. Da die Erfindung in entsprechenden Konstrukten außerdem eine Flankierung der NMD-Kassette mit einem oder mehreren Paaren von Erkennungssequenzen für eine oder mehrere Rekombinasen vorsieht, kann ein zunächst durch ein Resistenzgen oder Reportergen vollständig oder unvollständig inaktiviertes Allel in ein konditionales Allel umgewandelt werden. Abhängig von der Orientierung der NMD-Kassette, die mit dem Selektionsmarkergen gekoppelt ist, kann das Zielgen, in das die NMD-Kassette integriert ist, konditional inaktiviert oder reaktiviert werden. Bei Vorliegen mehrerer Paare von Erkennungssequenzen, die die NMD-Kassette flankieren, kann der Aktivitätszustand des Gens auch mehrfach konditional geändert werden.
   Zielzellen für alle als Genfallen ausgeführte erfindungsgemäße Nukleinsäurekonstrukte sind in erster Linie murine ES-Zellen, aber auch ES-Zellen anderer nicht-menschlicher Säugetiere, die zur Herstellung kompletter Tiere mit den eingeführten Mutationen geeignet sind. Außerdem ist der Einsatz erfindungsgemäßer Genfallen in allen nicht-menschlichen tierischen Zellen, z.B. somatischen Zellen, vorgesehen, aus denen sich z.B. mittels Kerntransfer in entkernte Eizellen komplette nicht-menschliche Tiere herstellen lassen. Weiterhin sieht die Erfindung den Einsatz erfindungsgemäßer Genfallen in allen eukaryontischen Zellen vor, die Spleißen und NMD aufweisen, z.B. in kultivierten pflanzlichen Zellen, aus denen sich komplette Pflanzen herstellen lassen.
   In einer weiteren Ausführungsform sieht die Erfindung zur zufälligen Mutagenese die Verwendung von NMD-Kassetten als Bestandteile von Transposons in eukaryontischen Zellen vor. Dazu werden die NMD-Kassette und ein mit ihr verbundenes Selektionsmarkergen mit Erkennungssequenzen für eine eukaryontische Transposase (z.B. "sleeping beauty", "frog prince" oder "Piggybac" Transposase) an den Enden flankiert. Bevorzugt ist dabei die Verwendung einer NMD-Kassette, die mit Erkennungssequenzen für mindestens eine Rekombinase, bevorzugt für mehrere Rekombinasen flankiert ist, sowie ein Resistenzgen, das von Erkennungssequenzen für eine andere Rekombinase flankiert ist als die, die mit den die NMD-Kassette flankierenden Erkennungssequenzen reagieren. Weiterhin ist vorgesehen, dass das Resistenzgen so mit einem prokaryontischen Replikationsursprung verbunden ist, dass dieser innerhalb der das Resistenzgen flankierenden Erkennungssequenzen lokalisiert ist und zusammen mit dem Resistenzgen deletiert werden kann.
   Entsprechende Konstrukte können gezielt mittels homologer Rekombination oder mittels zufälliger Integration in das Genom einer Zielzelle inseriert werden. Der jeweilige Insertionsort kann dann mittels des oben beschriebenen Plasmid Rescues oder durch geeignete klonierungsfreie PCR-Methoden erfolgen. Dabei ist es unerheblich, ob die initiale Insertion in ein Gen oder in intergenische Bereiche erfolgt ist, da die von den Erkennungssequenzen für die Transposase flankierten Segmente in Anwesenheit der Transposase mobilisiert werden und an anderer Stelle wieder ins Genom inseriert werden (Transposition). Die Loci, an denen die erneute Insertion von NMD-Kassetten als Teil von Transposons erfolgte, können wiederum mittels Plasmid Rescues oder mit PCR-Techniken identifiziert werden. Erfolgte die Insertion in ein Intron eines Gens, kann das Resistenzgen zusammen mit dem prokaryontischen Replikationsursprung mittels der geeigneten Rekombinase entfernt werden. Abhängig von der Orientierung, in der die NMD-Kassette nach der Transposition in einem Allel vorliegt, d.h. abhängig davon, ob das NMD-vermittelnde Exon im kodogenen oder im nicht-kodogenen Strange des Allels lokalisiert ist, ist das betroffene Allel direkt vollständig inaktiviert oder verbleibt in seiner aktiven Form. Da die Erfindung eine Flankierung der NMD-Kassette mit Erkennungssequenzen für eine oder mehrere Rekombinasen vorsieht, kann der Aktivitätszustand eines durch Transposition erfindungsgemäßer Nukleinsäurekonstrukte mutiertes Allel mittels der geeigneten Rekombinasen einfach oder mehrfach in den jeweils anderen Aktivitätszustand überführt werden. Dies ist ein wesentlicher Vorteil gegenüber herkömmlichen Transposons, die bisher nur die komplette Inaktivierung getroffener Allele erlauben.
   Als Zielzellen für mit Transposaseerkennungssequenzen flankierte NMD-Konstrukte sind murine ES-Zellen bevorzugt, aus denen man komplette nicht-menschliche Tiere herstellen kann, die das NMD-Transposon stabil in ihr Genom integriert haben. Außerdem sieht die Erfindung die Insertion entsprechender Konstrukte in allen anderen nicht-menschlichen eukaryontischen Zellen vor, die zur Herstellung kompletter Organismen genutzt werden können. Dies betrifft insbesondere auch befruchtete Eizellen, in die entsprechende Konstrukte z.B. mittels der Pronukleusinjektion, d.h. Injektion in den männlichen Vorkern der befruchteten Eizelle eingebracht werden können, um diese dann mittels bekannter Methoden zu transgenen Tieren auszudifferenzieren. Weiterhin betrifft dies alle anderen nicht-menschlichen eukaryontischen Zellen, die Spleißen von prä-mRNA und NMD aufweisen und für die geeignete Transposasesysteme zur Verfügung stehen, z.B. auch kultivierte pflanzliche Zellen.
   Die Transposition von einem stabil in das Genom einer Zielzelle integrierten Konstrukt, dass eine NMD-Kassette enthält und von Erkennungssequenzen für eine Transposase flankiert ist, kann durch die transiente Expression der spezifischen Transposase in den Zellen erfolgen, in deren Genom das Konstrukt ursprünglich inseriert wurde. Alternativ können Zellen, in denen das Konstrukt einmal durch Transposition in einen anderen Locus überführt wurde, als neue Substrate für eine erneute transiente Expression der Transposase genutzt werden. Gleiches gilt für alle Tochterzellen dieser zweiten und aller weiteren Transpositionsreaktionen. Dies bietet den erfindungsgemäßen Vorteil, dass sich Banken von Zellen herstellen lassen, in denen ein erfindungsgemäßes Nukleinsäurekonstrukt in vielen verschiedenen Genen inseriert vorliegt.
   Alternativ zur Transposition in kultivierten Zellen kann die Transposition bevorzugt auch *in vivo* durchgeführt werden, indem ein Organismus, z.B. eine transgene Maus, die eine NMD-Kassette als Bestandteil eines Transposons enthält, mit einer zweiten, unabhängig erzeugten Maus verpaart wird, die die geeignete Transposase in der Keimbahn exprimiert. Die Transposition findet dann entweder in der befruchteten Eizelle oder bei der Bildung von Keimzellen in Individuen der Tochtergeneration statt. Der Locus, in dem die NMD-Kassette durch die Transposition inseriert ist, kann anhand genomischer DNA, die aus einer dem betreffenden Individuum entnommenen Biopsie entnommen wurde, mittels des erwähnten "Plasmid Rescues" oder durch geeignete bekannte PCR-Methoden ermittelt werden. Aufgrund des im Fall von Mäusen ca. 20%igen Anteils der Introns am Gesamtgenom weist bei diesen jeder fünfte Nachkomme, in dem eine Transposition stattgefunden hat, eine Insertion der NMD-Kassette in ein Intron eines anderen Gens auf. Dabei ist bekannt, dass die Transposition überrepräsentativ häufig in benachbarte Loci auf dem gleichen Chromosom erfolgt. Deshalb ist diese Ausführungsform der Erfindung besonders zur Untersuchung benachbarter Gene geeignet, z.B. wenn mit anderen Methoden ein bestimmter Bereich des Genoms, der mehrere oder eine Vielzahl von Genen enthält, mit bestimmten Phänotypen korreliert werden kann.
   Bevorzugt werden für die Anwendung von zur Transposition geeigneten NMD-Kassetten nicht-menschliche Säugetiere, v.a. Mäuse und andere Nager, z.B. Ratten, eingesetzt, aber auch andere Tiere sowie Pflanzen und andere Eukaryonten, die Spleißen von prä-mRNA und NMD aufweisen und für die geeignete Transposasesysteme zur Verfügung stehen.
   Zellen, die mit der erfindungsgemäßen NMD-Kassette genetisch manipuliert werden, sind insbesondere ES-Zellen nicht-menschlicher Säuger, vor allem der Maus, adulte Stammzellen oder noch teilungsfähige somatische Zellen, insbesondere von Menschen, Tieren oder Pflanzen aber auch von allen anderen Eukaryonten, die Spleißen von prä-mRNA und NMD aufweisen.
   Für gentherapeutische Zwecke ist weiterhin eine Insertion der NMD-Kassette in heterozygot dominant wirkende Allele bevorzugt, wie sie bei dominant vererbten Erbkrankheiten des Menschen auftreten. Aufgabe erfindungsgemäßer NMD-Kassetten dabei ist, das mutierte Allel zu inaktivieren und somit seinen dominanten Phänotyp zu eliminieren. Dazu können adulte Stammzellen oder noch teilungsfähige somatische Zellen eines Patienten zum einen mit einer eine NMD-Kassette beinhaltenden Genfalle transfiziert und anschließend Zellen identifiziert werden, bei denen die Genfalle ein Intron des mutierten Allels getroffen hat. Dabei sieht die bevorzugte Ausführungsform die Verwendung einer mittels einer Rekombinase invertierbaren NMD-Kassette vor. Dies bietet den erfindungsgemäßen Vorteil, dass auch solche Zellen genutzt werden können, in denen die NMD-Kassette zunächst in ihrer inaktiven Orientierung inseriert wurde, bei der sich also das NMD vermittelnde Exon im nicht-kodogenen Strang des mutierten Allels befindet. Die Überführung der NMD-Kassette in die korrekte, also das mutierte Allel inaktivierende Orientierung, kann dann durch transiente Expression der geeigneten Rekombinase erfolgen. Nach Deletion des Resistenzgens mittels der dafür vorgesehenen Rekombinase können die so gentherapierten Zellen in der Zellkultur expandiert werden, um schließlich auf den Patienten replantiert zu werden.
   Unabhängig davon, ob die genetische Manipulation mittels gezielter Mutagenese, durch Genfallen oder durch Transposition erfolgt, werden Zellen, die durch Einbringen der NMD-Kassette genetisch verändert worden sind, vorzugsweise selektiert, bevor die Zellen vermehrt oder zur Differenzierung gebracht werden. Bevorzugterweise ist die NMD-Kassette deshalb mit einem Selektionsmarkergen gekoppelt, so dass die Selektion durch Aufbringen des Selektionsdrucks, beispielsweise eines Antibiotikums im Falle einer Antibiotikaresistenz als Selektionsmarker, erfolgen kann.
   Im Anschluss an die Selektion resistenter Transformanten kann im Fall einer gezielten Mutagenese die homologe Rekombination der NMD-Kassette in den gewünschten Locus verifiziert werden bzw. die Identität des Insertionsorts von in Genfallen oder Transposons beinhalteten NMD-Kassetten bestimmt werden.
   Zellen, bei denen eine NMD-Kassette in geeigneter Weise in einem Zielgen integriert ist, können dann *in vitro* weiter kultiviert und zur Erzeugung von *in vitro* Zell- oder Gewebskulturen genutzt werden.
   Alternativ können im Falle von nicht-menschlichen ES-Zellen diese zu einem nicht-menschlichen Säuger ausdifferenzieren gelassen werden, wobei die Verwendung muriner ES-Zellen bevorzugt ist.
   Weiterhin sieht die Erfindung vor Zellen anderer nicht-menschlicher Tiere, Pflanzen und Eukaryonten, die eine erfindungsgemäße NMD-Kassette beinhalten, zur Herstellung vollständiger Organismen zu nutzen, die dann die eingeführte Mutation an ihre Nachkommen weiter vererben können.
   Zur Erzeugung nicht-menschlicher Säuger können bekannte Verfahren eingesetzt werden, bevorzugt die Injektion mutierter muriner ES-Zellen in Blastozysten, aus denen sich chimäre Tiere entwickeln, die bei Vorliegen von Keimbahntransmission der verwendeten ES-Zellen zur Generierung von Mauslinien mit der gewünschten Mutation genutzt werden können. Besonders bevorzugt bei der Verwendung von murinen ES-Zellen werden bekannte Verfahren, bei denen sich aus den mutierten ES-Zellen direkt ein nicht-chimäres Individuum entwickelt, das in allen Zellen, also auch den Keimzellen, die gewünschte Mutation aufweist, und direkt zur Etablierung einer entsprechenden Mauslinie genutzt werden kann. Dies kann z.B. durch die Verwendung von F1 ES-Zellen zur Injektion in tetraploide Blastozysten oder durch die Injektion in 8-Zell-Embryos erfolgen.
   Andere Tiere können z.B. durch Klonierung mittels Nukleustransfers aus einer mit einem erfindungsgemäßen Nukleinsäurekonstrukt manipulierten somatischen Zelle in eine entkernte Eizelle erzeugt werden. Auch bei dieser Ausführungsform der Erfindung haben die erfindungsgemäßen Nukleinsäurekonstrukte den Vorteil, dass aus transformierten Zellen unmittelbar Tiere erzeugt werden können, die ein Allel tragen, das unmittelbar inaktiviert ist, oder zunächst ohne Beeinträchtigung aktiv und konditional inaktivierbar ist, oder inaktiviert und konditional reaktivierbar ist.
   Im Fall der Nutzung von NMD-Kassetten als Bestandteil von Transposons in eukaryontischen Zellen sieht die Erfindung außerdem vor, erfindungsgemäße Transposons mittels Injektion in befruchtete Eizellen nicht-menschlicher Säugetiere zur Erzeugung transgener Tiere zu nutzen. Dies ist möglich, da diese Nutzung den Vorteil bietet, dass die NMD-Kassette in ihrem initialen Insertionsort nicht funktional sein muß, da sie anschließend durch Gegenwart der Transposase mobilisiert und an anderer Stelle reintegriert werden kann.
   Im Ergebnis führen alle Methoden zur Herstellung von Tieren oder anderen nicht-menschlichen eukaryontischen Organismen, die transgen bezüglich einer inserierten NMD-Kassette sind, zu Individuen, die ein eine NMD-Kassette beinhaltendes Allel an ihre Nachkommen weitervererben können, das unmittelbar inaktiviert ist, oder zunächst ohne Beeinträchtigung aktiv verbleibt und konditional inaktivierbar ist, oder zunächst inaktiviert vorliegt und konditional reaktivierbar ist. Gleiches gilt für andere Organismen wie Pflanzen und andere Eukaryonten, die entsprechende Allele tragen. Entsprechende Individuen können dann genutzt werden um durch Verpaaren Nachkommen zu erzeugen, die homozygot für die jeweilige Mutation sind.
   Unabhängig davon, ob eine mit Erkennungssequenzen für mindestens eine, bevorzugt zwei oder mehrere Rekombinasen flankierte NMD-Kassette gezielt mittels homologer Rekombination, zufällig durch Insertion einer Genfalle oder als Ergebnis der Transposition einer mit Erkennungssequenzen für eine Transposase flankierten erfindungsgemäßen Nukleinsäurekassette in ein Allel inseriert ist, sieht die Erfindung vor, dass die Aktivität eines betroffenen Zielgens durch die Wirkung einer der Rekombinasen für die die NMD-Kassette flankierenden Erkennungssequenzen konditional verändert werden kann, indem das NMD-vermittelnde Exon in den jeweils anderen Strang des Gens überführt wird oder indem die NMD-Kassette rekombinasevermittelt deletiert wird. Inversionen, die jede für sich aufgrund der bevorzugt verwendeten mutierten Rekombinaseerkennungssequenzen irreversibel sind, können durch Verwendung einer zweiten Rekombinase, deren Erkennungssequenzen ebenfalls die NMD-Kassette flankieren, rückgängig gemacht werden. Demgegenüber entfernt die rekombinasevermittelte Deletion die NMD-Kassette unwiederbringlich aus dem Genom, so dass ein betreffendes Allel dadurch irreversibel aktiviert wird.
   Die Rekombinasen bzw. Integrasen können von einem transient eingebrachten oder genomisch integrierten und vorzugsweise induzierbaren Nukleinsäurekonstrukt synthetisiert werden, das das Rekombinasegen z.B. unter der Kontrolle eines induzierbaren Promotors, beispielsweise eines Tetracyclin-induzierbaren Promotors enthält. Alternativ kann die Induktion der Rekombinasewirkung posttranslational erfolgen, wenn z.B. dem Fachmann bekannte Fusionsproteine einer Rekombinase und der ligandenbindenden Domäne eines Steroidrezeptors exprimiert werden, die erst in Anwesenheit jeweiligen Liganden in den Zellkern transloziert werden und dann ihre Wirkung entfalten. Beispielsweise wirkt das CreERT2-Fusionsprotein, dass aus der Peptidkette der Cre-Rekombinase und einer Mutante des humanen Östrogenrezeptors besteht, nur in Anwesenheit seines Liganden Tamoxifen, bleibt aber von natürlichen Östrogenen unbeeinflußt. Als weitere Alternative kann die Rekombinase in Proteinform in Zielzellen eingebracht werden.
   Bevorzugt wird ein Kontaktieren der Erkennungssequenzen mit den jeweils dazugehörigen Rekombinasen *in vivo.* Dies kann im Fall nicht-menschlicher Säuger und anderer Tiere sowie anderen sich sexuell fortpflanzenden Eukaryonten durch Verpaaren von Individuen, die die mit Erkennungssequenzen für die jeweiligen Rekombinasen flankierten NMD-Kassetten tragen, mit Individuen geschehen, die die jeweils benötigte Rekombinase gewebs- und/oder entwicklungsspezifisch bzw. ubiquitär induzierbar oder gewebespezifisch induzierbar exprimieren.
   Dabei kann die Expression einer Rekombinase, z.B. flp, bereits in der Zygote stattfinden, so dass alle Zellen des sich aus der Zygote entwickelnden Tieres die NMD-Kassette in der gleichen, von der Rekombinase in der Zygote erzeugten Orientierung aufweisen. Dies kann unter anderem dazu benutzt werden, ein ursprünglich für die konditionale Inaktivierung vorgesehenes Allel, bei dem das NMD vermittelnde Exon im nicht-kodogenen Strang lokalisiert ist, durch Wirkung der flp-Rekombinase in den kodogenen Strang überführt wird, wodurch das Allel in der Zygote und allen sich daraus entwickelnden Zellen zunächst inaktiviert vorliegt. Ein auf diese Weise erzeugtes "knock out first" Allel kann dann durch die Wirkung einer zweiten Rekombinase, z.B. bevorzugt Cre, gewebespezifisch, oder ubiquitär bzw. gewebespezifisch induzierbar reaktiviert werden. Die Erfindung sieht dies vor allem für Gene vor, die ubiquitär oder von vielen verschiedenen Zelltypen exprimiert werden und die bei konditional gewebespezifischer Inaktivierung keinen Phänotyp zeigen, da die fehlende Expression des Genproduktes von Zellen, in denen das Allel inaktiviert vorliegt, durch das gleiche, von anderen Zellen exprimierte Produkt kompensiert wird.
   Die Erfindung sieht außerdem die Expression der Rekombinase bevorzugt gewebsspezifisch, z.B. gewebsspezifisch induzierbar oder wird während der Entwicklung gewebsspezifisch induziert.
   In einer Ausführungsform der Erfindung kann die Invertierung einer von Erkennungssequenzen flankierten, in der inaktiven Orientierung im Zielgen angeordneten NMD-Kassette auslösbar sein, indem die Rekombinase von einem gewebsspezifischen und/oder induzierbaren Promotor exprimierbar ist. Auf diese Weise können die Transkripte eines Zielgens gewebsspezifisch bzw. induzierbar der Degradation zugeführt werden, so dass eine entsprechend gewebsspezifische und/oder induzierbare Inhibierung der Expression des betreffenden Zielgens bzw. Allels erreicht wird.
   Das Gen für eine spezifische Rekombinase kann außerdem per viraler Transduktion in Zielzellen eingebracht werden, entweder in kultivierten ES-Zellen oder *in vivo.*
   Ein weiterer Vorteil der Erfindung liegt darin, dass durch die konditionale Inaktivierung von Zielgenen auch komplexe, d.h. von mehreren Genen beeinflusste Phänotypen analysiert werden können. Dies kann durch konditionale Inaktivierung einer Mehrzahl von Genen mit jeweils spezifisch integrierenden Nukleinsäurekonstrukten erfolgen, wobei die NMD-Kassetten jeweils gleich oder verschieden sein können, vorzugsweise jeweils mit Erkennungssequenzen für jeweils unterschiedliche Rekombinasen zur selektiven Inaktivierung der einzelnen Gene durch die jeweilige spezifische Rekombinase. Weiterhin kann bei diesem Verfahren jede nicht-menschliche Zelle mit einem konditional inaktivierbaren Allel ausgestattet werden, und daraus nicht-menschliche Individuum erzeugt werden, selbst wenn das Zielgen oder die Zielgene während der embryonalen Entwicklung essentiell sind oder von diesen mitbeeinflusste Phänotypen nur vor einem bestimmten genetischen Hintergrund auftreten.

### Genaue Beschreibung der Erfindung

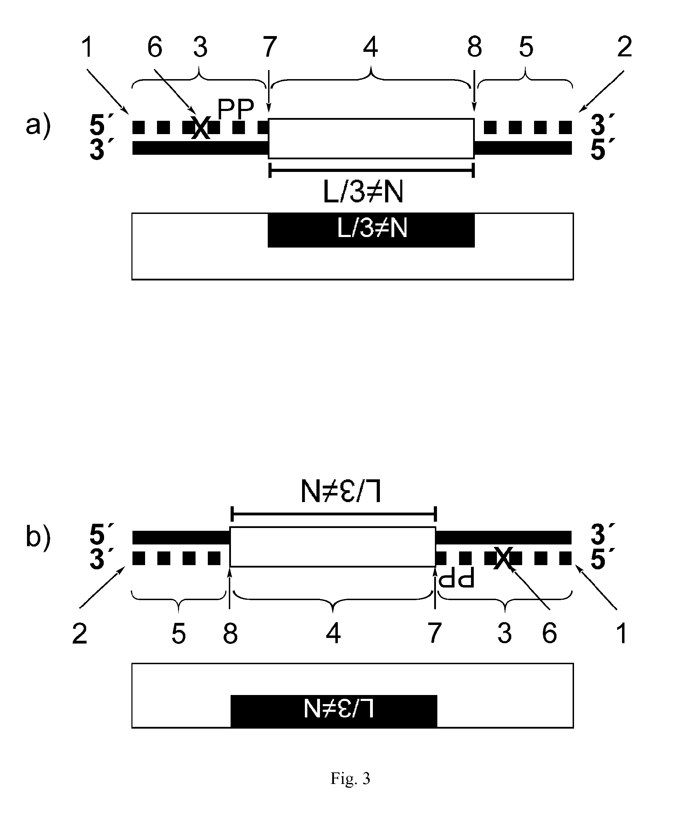

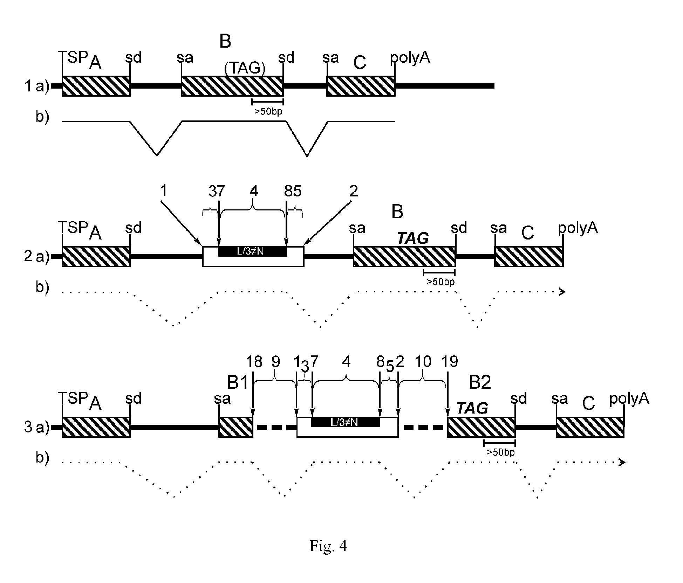

Die Erfindung wird nun näher anhand von Beispielen und Figuren beschrieben, in denen
- Figur 1 eine schematische Darstellung einer erfindungsgemäßen NMD-Kassette zeigt, nämlich unter a) in aktiver Orientierung und unter b) in inaktiver Orientierung,
- Figur 2 schematisch die Wirkungsweise der erfindungsgemäßen Nukleinsäurekonstrukte am Beispiel des Ersetzens eines genomischen Exons, der Insertion in ein Intron bzw. der Insertion in ein Exon eines Zielgens zeigt,
- Figur 3 schematisch ein erfindungsgemäßes Nukleinsäurekonstrukt zeigt, das zusätzlich oder in Alternative zu mindestens einem Stopkodon einen exonischen Bereich aufweist, der eine nicht glatt durch 3 teilbare Anzahl von Nukleotiden aufweist,
- Figur 4 schematisch die Inaktivierung eines Zielgens durch ein erfindungsgemäßes Nukleinsäurekonstrukt nach Figur 3 am Beispiel der Insertion in ein Intron bzw. Exon eines Zielgens zeigt,
- Figur 5 schematisch die anteilige Inaktivierung eines Zielgens durch ein erfindungsgemäßes Nukleinsäurekonstrukt zeigt,
- Figur 6 schematisch die Inaktivierung einer Spleißvariante eines Gens mittels eines erfindungsgemäßen Nukleinsäurekonstrukts zeigt,
- Figur 7 schematisch die Erzeugung einer NMD-Kassette im Genom einer Zielzelle durch ortsgerichtete Mutagenese zeigt,
- Figur 8 schematisch die Insertion eines erfindungsgemäßen Nukleinsäurekonstrukts in ein Intron eines Zielgens zeigt,
- Figur 9 schematisch die Inaktivierung eines Gens durch ein erfindungsgemäßes Nukleinsäurekonstrukt durch Insertion in ein Exon des Zielgens zeigt,
- Figur 10 die Insertion eines erfindungsgemäßen Nukleinsäurekonstrukts in ein Zielgen und dessen anschließende konditionale Inaktivierung zeigt,
- Figur 11 die unmittelbare Inaktivierung des Zielgens durch ein erfindungsgemäßes Nukleinsäurekonstrukt mit anschließender konditionaler Reaktivierung des Zielgens zeigt,
- Figur 12 die Reaktivierung eines durch ein erfindungsgemäßes Nukleinsäurekonstrukt inaktiviertes Zielgen durch Deletion des Konstrukts zeigt,
- Figur 13 die konditionale Aufhebung der durchgeführten konditionalen Inaktivierung eines Zielgens durch eine erneute Invertierung eines erfindungsgemäßen Nukleinsäurekonstrukts zu dessen Reaktivierung zeigt,
- Figur 14 die konditionale Reaktivierung eines Zielgens durch Inversion eines erfindungsgemäßen Nukleinsäurekonstrukts und seine anschließende konditionale Inaktivierung durch Deletion des Konstrukts,
- Figur 15 die konditionale Reaktivierung eines Zielgens durch Inversion eines erfindungsgemäßen Nukleinsäurekonstrukts sowie seine konditionale Inaktivierung durch erneute Inversion des Konstrukts,
- Figur 16 schematisch zwei Genfallen mit einem erfindungsgemäßen
   Nukleinsäurekonstrukt und ihre Anwendung zeigt, wobei zunächst ein Reporter-/Selektionsgen inaktivierend wirkt,
- Figur 17 schematisch eine Genfalle zeigt, bei der direkt ein erfindungsgemäßes NMD vermittelndes Exon als Genfalle wirkt,
- Figur 18 schematisch ein erfindungsgemäßes Nukleinsäurekonstrukt als Bestandteil eines retroviralen Vektors darstellt,
- Figur 19 ein als eukaryontisches Transposon ausgeführtes erfindungsgemäßes Nukleinsäurekonstrukt zeigt,
- Figur 20 ein als prokaryontisches Transposon ausgeführtes erfindungsgemäßes Nukleinsäurekonstrukt und die Herstellung eines Vektors für die gezielte Geninaktivierung zeigt,
- Figur 21 die Herstellung von Banken für die gezielte Geninaktivierung geeigneter Vektoren aus Gemischen genomischer Fragmente mittels erfindungsgemäßer Nukleinsäurekonstrukte zeigt,
- Figur 22 schematisch die Insertion eines erfindungsgemäßen Nukleinsäurekonstrukts in das β7-Integringen der Maus zeigt,
- Figur 23 die mit erfindungsgemäßen Nukleinsäurekonstrukten erzielte Inaktivierung des murinen β7-Integringens auf Protein- und RNA-Ebene im Vergleich mit Kontrollen zeigt,
- Figur 24 eine NMD-Kassette und ihre Wirkung in beiden Orientierungen auf ein Reportergen zeigt,
- Figur25 eine optimierte NMD-Kassette und ihre Wirkung auf das selbe Reportergen zeigt, und
- Figur 26 die Ergebnisse einer Insertion einer als Transposon ausgeführten NMD-Kassette in ein kloniertes genomisches DNA-Fragment zeigt.
   In den Figuren werden nur NMD-Kassetten mit einer nicht durch 3 teilbaren Anzahl von Nukleotiden (Figur 3) und Figur 4)) sowie in den übrigen Figuren NMD-Kassetten mit mindestens einem Stopkodon bevorzugt mindestens 50 bp stromaufwärts der Spleißdonorstelle des NMD vermittelnden Exons dargestellt. Ebenfalls bevorzugte NMD-Kassetten, die ein NMD vermittelndes Exon mit einem geringeren Abstand zwischen dem mindestens einen Stopkodon und der Spleißdonorstelle des Exons enthalten, z.B. von einem Exon eines T-Zellrezeptorgens abgeleitete, sind nicht dargestellt.

Die Darstellung aller Figuren entspricht der Konvention, dass sich bezogen auf die Transkriptionsrichtung eines dargestellten Gens oder eines Teiles davon, das jeweilige 5'-Ende links befindet, das 3'-Ende rechts. Bei der Darstellung von Genen ist, abgesehen von darin beinhalteten NMD-Kassetten, jeweils nur der kodogene Strang dargestellt.

In den Figuren bezeichnen gleiche Bezugsziffern, einzelne Buchstaben oder Buchstabenfolgen gleiche Elemente. Diese Bezugsziffern, Buchstaben und Buchstabenfolgen werden in der Beschreibung in Klammern angegeben. Von Zielgenen wird jeweils nur der kodogene Strang dargestellt. Dabei stellen schraffierte Rechtecke Exons eines Zielgens dar, während Intronsequenzen des Zielgens als durchgezogene schwarze Linien gezeigt sind.

Primärtranskripte, deren Prozessierung durch Spleißen zu translatierbarer mRNA führt, sind als durchgezogene Linie dargestellt, während Transkripte, die der Degradation zugeführt werden, als dünne Punktlinien dargestellt sind. Die Pfeilspitze am Ende der Transkripte gibt die Transkriptionsrichtung an. Introns, die aus einem Primärtranskript gespleißt werden sind in den Primärtranskripten nach unten abgewinkelt dargestellt. Die waagerecht dargestellten Bereiche der Primärtranskripte stellen die Exons dar, die die reife mRNA bilden.

Erkennungssequenzen für Rekombinasen und Integrasen sind in den Figuren als Pfeilspitzen innerhalb von DNA dargestellt, wobei gegeneinander gerichtete Pfeilspitzen die Möglichkeit Inversion dazwischen angeordneter DNA-Abschnitte andeuten und gleichgerichtete Pfeilspitzen die Möglichkeit zur Deletion bei Wechselwirkung mit der spezifischen Rekombinase oder Integrase. Anteilig schwarz und weiß gezeichnete Pfeilspitzen deuten an, dass die Erkennungssequenzen Mutanten sind, für die die Wechselwirkung mit der spezifischen Rekombinase nur einmal, d.h. irreversibel möglich ist und das Paar von Erkennungssequenzen anschließend nicht mehr mit der Rekombinase oder Integrase reagiert. Nach irreversibler Inversion sind von dem Paar Pfeilspitzen für die betreffenden Erkennungssequenzen eine vollständig schwarz und die andere weiß dargestellt.

Die Mehrzahl der hier schematisch dargestellten Verfahren werden zur Erzeugung genetisch manipulierter Tiere vorzugsweise so durchgeführt, dass die Insertion des Nukleinsäurekonstrukts in ES-Zellen vorgenommen wird, wobei Transformanten mittels eines Selektionsmarkergens identifiziert bzw. selektiert werden. Anschließend kann das Selektionsmarkergen noch in Zellkultur, z.B. im Stadium der ES-Zellen, durch Anwesenheit der für die das Selektionsmarkergen umrahmenden Erkennungssequenzen spezifischen Rekombinase deletiert werden. Das Ergebnis der Deletion dieses Selektionsmarkergens ist in den Beispielen dargestellt. Alternativ kann das Selektionsmarkergen *in vivo* deletiert werden, indem Tiere, die ein entsprechendes Nukleinsäurekonstrukt beinhalten, mit Tieren verpaart werden, die die spezifische Rekombinase in ihren Keimzellen exprimieren, so dass es in der befruchteten Eizelle zur Deletion kommt.

Aus erfindungsgemäß veränderten Zellen kann ein Gewebe differenzieren gelassen oder ein Tier erzeugt werden. Im Anschluß daran kann die Orientierung der NMD-Kassette im Zielgen mittels der Rekombinaseaktivität verändert werden oder dieselbe anschließend deletiert und aus dem Zielgen entfernt werden. Die Aktivität der für die Erkennungssequenzen spezifische Rekombinase kann z.B. durch Verpaaren mit Tierstämmen, die diese Rekombinasen, vorzugsweise gewebsspezifisch und/oder induzierbar, exprimieren, oder durch viralen Gentransfer erzeugt werden.

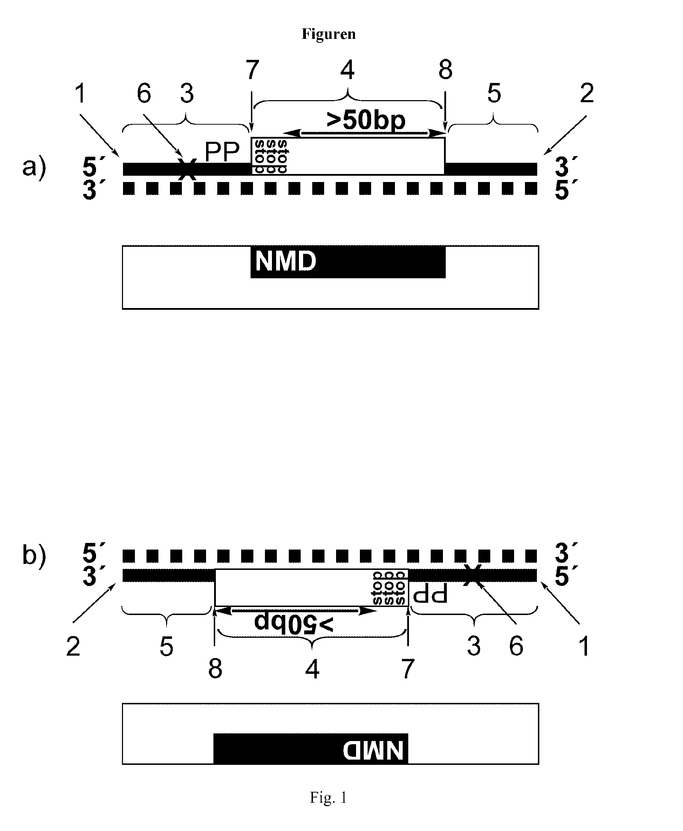

In den Figuren 1a) und 1c) sowie 3a) und 3c) ist der Aufbau von erfindungsgemäßen NMD-Kassetten detailliert als doppelsträngige DNA dargestellt. In den übrigen Abbildungen finden die in Figuren 1b) und 1d) sowie 3b) und 3d) eingeführten vereinfachten Schemadarstellungen von NMD-Kassetten Verwendung.

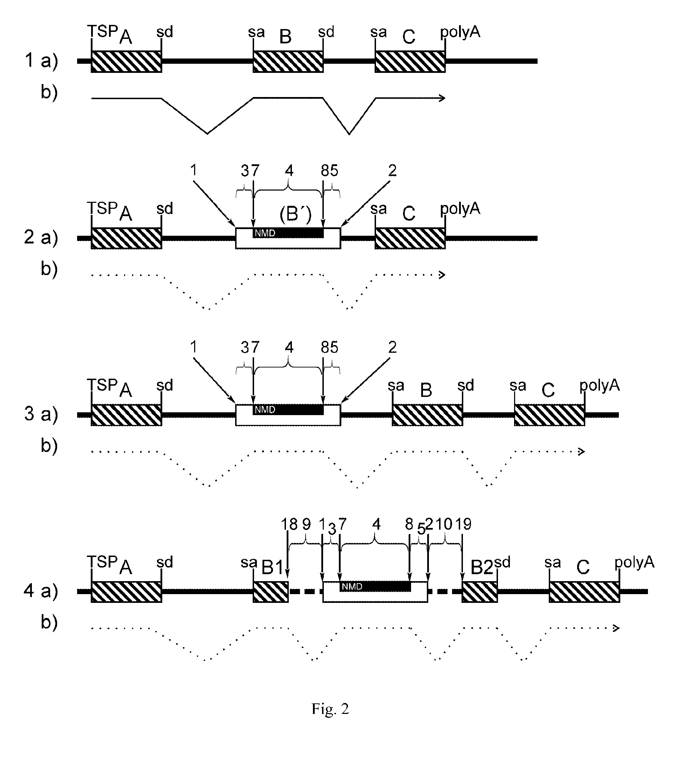

In der Figur 2) sowie in den Figuren 4) bis 7) sind jeweils nur die für die Wirkungsweise von NMD-Kassetten wesentlichen Elemente dargestellt. Optional damit verbundene Elemente wie Erkennungssequenzen für Rekombinasen, Transposasen, Resistenz- oder Reportergene und deren Funktion in erfindungsgemäßen Nukleinsäurekonstrrukten werden in den darauf folgenden Figuren erläutert.

In den Figuren 1a) und 1c) sowie 3a) und 3c) stellt der jeweils obere Strang den kodogenen Strang dar und ist als durchgezogene Linie dargestellt. Der jeweils komplementäre nichtkodogene Gegenstrang wird als unterbrochene Linie dargestellt.

Die Figuren 1b) und 1d) sowie 3b) und 3d) zeigen eine vereinfachte Darstellung einer NMD-Kassette, die in den übrigen Figuren zur Anwendung kommt. Dabei wird die NMD-Kassette als weißes Rechteck dargestellt. Der darin enthaltene mit "NMD" gekennzeichnete Bereich stellt das Exon (4) dar, die links und recht angrenzenden weißen Flächen die Intronabschnitte (3) und (5). Der durchgehend weiße Bereich in der anderen Hälfte stellt den Gegenstrang dar. Ist der schwarze Bereich in der oberen Hälfte angeordnet und lesbar als "NMD" markiert ist, befindet sich die NMD-Kassette in ihrer aktiven Orientierung, d.h. im kodogenen Strang eines Zielgens angeordnet (Figuren 1b) und 3b)), während die inaktive Orientierung der NMD-Kassette die Bezeichnung NMD auf dem Kopf und von rechts nach links zu lesen zeigt und die untere Hälfte, d.h. den nicht kodogenen Strang schwärzt (Figuren 1d) und 3d)).

In Figur 1 ist schematisch der Aufbau einer erfindungsgemäßen NMD-Kassette gezeigt, die nicht von Erkennungssequenzen für Rekombinasen flankiert ist. Das Konstrukt weist von 5' nach 3' einen ersten Intronabschnitt (3), ein Exon (4), gefolgt von einem zweiten Intronabschnitt (5) auf. Das Exon (4) wird von einer Spleißakzeptorstelle (7) und einer Spleißdonorstelle (8) begrenzt

Der in 5' vom Exon (4) angeordnete erste Intronabschnitt (3) weist einen schematisch dargestellten Verzweigungspunkt (6) sowie einen zwischen dem Verzweigungspunkt (6) und dem Exon (4) lokalisierten Polypyrimidinabschnitt (PP) auf, die beide an der Spleißreaktion während der Reifung zur mRNA teilnehmen. Die Erfindung sieht vor, dass der Intronabschnitt (3) an seinem 5'-Ende (1) keine Spleißdonorstelle auf weist. Es ist außerdem bevorzugt, dass er zwischen dem 5'-Ende (1) und dem Verzweigungspunkt (6) keine funktionalen Sequenzen zum Spleißen, z.B. keine funktionalen Spleißakzeptor-, Spleißdonorstellen, Verzweigungspunkt und Polypyrimidinsequenz aufweist, oder zumindest im Falle ihres Vorhandenseins, z.B. in Form kryptischer Spleißdonor- und - akzeptorstellen, so angeordnet sind, dass sie nicht zum Spleißen benutzt werden können. Der Intronabschnitt (3) enthält also nur die funktionalen Anteile des 3'-Endes eines Introns, und stellt somit kein vollständiges Intron dar. Optional kann der Intronabschnitt (3) aber intronische Spleiß-"enhancer" (-verstärker) oder -"silencer" (-dämpfer) enthalten, die selbst kein Spleißen vermitteln, sondern das Spleißen in der Nähe befindlicher Exons beeinflussen. Solche Elemente sind erfindungsgemäß zum Optimieren von NMD-Kassetten vorgesehen.

Der Intronabschnitt (3) kann natürlichen Ursprungs oder synthetisch sein und enthält am 3'-Ende mindestens die intronischen Anteile der Spleißakzeptorstelle (7), in 5' von der Spleißakzeptorstelle (7) eine bevorzugt 10 bis 50 bp lange Pyrimidin-reiche Sequenz (PP), sowie in 5' Richtung angrenzend den Verzweigungspunkt (6). Der Verzweigungspunkt (6) kann bevorzugt 10 bis 40 bp oder weiter in 5' von der Spleißakzeptorstelle (7) beabstandet sein, bevorzugter 30 bp. In 5' vom Verzweigungspunkt (6) umfasst der Intronabschnitt (3) bevorzugt mindestens 10 bis 100 bp, so dass die Lariatbildung beim Spleißvorgang von der Spleißdonorstelle eines stromaufwärts lokalisierten Exons eines Zielgens auf die Spleißakzeptorstelle (7) erfolgen kann.

Der in 3' an das Exon (4) angrenzende zweite Intronabschnitt (5) umfasst mindestens die intronischen Anteile einer Spleißdonorstelle (8), bevorzugt mindestens 10 bis 100 bp eines Introns, das synthetischen oder natürlichen Ursprungs sein kann, damit die Sequenz des Intronabschnitts (5) eine für die Bildung eines Lariats beim Spleißen von der Spleißdonorstelle (8) auf die Spleißakzeptorstelle eines stromabwärts folgenden Exons hinreichende Länge aufweist.

Der zweite Intronabschnitt (5) weist an seinem 3'-Ende (2) weder einen Verzweigungspunkt, noch einen Polypyrimidinabschnitt oder eine Spleißakzeptorstelle auf, enthält also nur die funktionalen Anteile des 5'-Endes eines Introns und stellt ebenfalls kein vollständiges Intron dar. Es ist dabei außerdem bevorzugt, dass er zwischen der Spleißdonorstelle (8) am 5'-Ende und dem seinem 3'-Ende (2) keine weiteren funktionalen Sequenzen zum Spleißen, z.B. keine funktionalen Spleißakzeptor-, Spleißdonorstellen, Verzweigungspunkt und Polypyrimidinsequenz aufweist, oder solche im Falle ihres Vorhandenseins, z.B. in Form kryptischer Spleißdonor- und -akzeptorstellen, so angeordnet sind, dass sie nicht zum Spleißen benutzt werden können. Optional kann der Intronabschnitt (5) aber intronische Spleiß-"enhancer" oder -"silencer" enthalten, die selbst kein Spleißen vermitteln, sondern das Spleißen in der Nähe befindlicher Exons beeinflussen. Solche Elemente sind erfindungsgemäß zum Optimieren von NMD-Kassetten vorgesehen. In 3' von der Spleißdonorstelle (8) umfasst der Intronabschnitt (5) bevorzugt mindestens 10 bis 100 bp, so dass die Lariatbildung beim Spleißvorgang von der Spleißdonorstelle (8) des NMD vermittelnden Exons eines Zielgens auf die Spleißakzeptorstelle eines in 3' benachbarten Zielgens erfolgen kann wenn die Insertion des erfindungsgemäßen Konstrukts in unmittelbarer Nachbarschaft stromaufwärts des Verzweigungspunktes des in 3' benachbarten Exons erfolgt.

Der erste und/oder zweite Intronabschnitt (3) bzw. (5) können mit Bereichen aus dem Zielgen identisch sein, z.B. um homologe Rekombination zu ermöglichen, wie im Fall, dass nur ein Exon eines Zielgens durch ein NMD vermittelndes Exon (4) ersetzt werden soll, die übrigen Sequenzen des Gens aber unverändert bleiben sollen.

Das Exon (4) wird von der Spleißakzeptorstelle (7) und der Spleißdonorstelle (8) begrenzt. Das Exon (4) weist mindestens ein Stopkodon (STOP) auf, hier dargestellt als die bevorzugte Ausführungsform mit drei Stopkodons, davon eins in jedem Leserahmen, wobei die Stopkodons vorzugsweise in der Nähe der Spleißakzeptorstelle (7) des Exons (4) angeordnet sind und das am weitesten in 3' gelegene Stopkodon bevorzugt mindestens 50 bp von der Spleißdonorstelle (8) beabstandet ist. Der Abstand zwischen der Spleißakzeptorstelle (7) und dem am weitesten in 5' Richtung angeordneten Stopkodon ist nicht beschränkt. Das Exon (4) hat bevorzugt eine Mindestgröße von 50 bis 55 bp, bevorzugter 60 bis 100 bp, bevorzugter eine größere Anzahl von bp.

Da die erfindungsgemäße Nutzung eines NMD vermittelnden Exons (4) das Spleißen voraussetzt, wird die bevorzugte Form einer NMD-Kassette so definiert, dass der erste (3) und zweite Intronabschnitt (5) mit den beschriebenen zum Spleißen notwendigen Sequenzelementen enthalten sind. Zur Herstellung einer minimalen NMD-Kassette in einem Zielgen können jedoch auch Nukleinsäurekonstrukte verwendet werden, die nur einen Bereich des Exons (4) aufweisen und zumindest ein Stopkodon in ein Exon des Zielgens einführen. Auf diese Weise läßt sich die erfindungsgemäße NMD-Kassette in einem Zielgen herstellen, deren erster und zweiter Intronabschnitt dem Zielgen selbst entstammen. Daher werden auch solche NMD-Kassetten als erfindungsgemäß angesehen, die in einem Zielgen enthalten sind, selbst wenn das Konstrukt zu ihrer Herstellung nicht die bevorzugte vollständige NMD-Kassette umfaßte.

Das NMD vermittelnde Exon (4) kann im wesentlichen von einem natürlichen Exon stammen, daraus abgeleitet sein oder rein synthetischen Ursprungs sein. Optional kann das Exon (4) Spleiß-"enhancer" oder -"silencer" beinhalten, um seine Funktionalität zu optimieren.

Erfindungsgemäß ist bevorzugt, dass NMD-Kassetten beinhaltende Nukleinsäurekonstrukte außer dem Exon (4) weder im gleichen Strang noch im Gegenstrang weitere Exons enthalten.

Das NMD vermittelnde Exon (4) wird in einem Zielgen nur wirksam, wenn die NMD-Kassette so in die genomische DNA der Zielzelle integriert ist, dass es als funktionales Exon erkannt und Teil der reifen mRNA des Zielgens wird. Dies wird als die aktive Orientierung einer NMD-Kassette bezeichnet, in der ihre funktionalen Elemente im kodogenen Strang der DNA der Zielzelle angeordnet sind. Die aktive Orientierung von NMD-Kassetten ist schematisch in Figuren 1a) und 1b) sowie 3a) und 3b) gezeigt. Die inaktive Orientierung ist in den Figuren 1c) und 1d) sowie 3c) und 3d) dargestellt.

Allgemein ist es erfindungsgemäß bevorzugt, dass der nicht-kodierende Strang (in Figuren 1a) und 1c) sowie 3a) und 3c) als unterbrochene Linie dargestellt, in Figuren 1b) und 1d) sowie 3b) und 3d) anteilig als durchgehender weißer Anteil gegenüber dem schwarz dargestellten und mit "NMD" gekennzeichneten Exon (4) keine funktionalen Sequenzen zum Spleißen, z.B. keine funktionalen Spleißakzeptor-, Spleißdonorstellen, Verzweigungspunkte und Polypyrimidinsequenzen aufweist, oder solche, im Falle ihres Vorhandenseins, z.B. in Form kryptischer Spleißdonor- und -akzeptorstellen, so angeordnet sind, dass sie nicht zum Spleißen benutzt werden können. Dies bietet den erfindungsgemäßen Vorteil, dass in einem Zielgen enthaltene Sequenzen einer NMD-Kassette das Spleißen seiner Transkripte nicht beeinflussen, wenn die NMD-Kassette in ihrer inaktiven Orientierung beinhaltet und das NMD-vermittelnde Exon im nicht-kodogenen Strang des Zielgens lokalisiert ist.

In der inaktiven Anordnung des Exons (4), die in Figuren1c) und 1d) sowie in Figuren 3c) und 3d) gezeigt ist, gelangen keine der Spleißsignale der NMD-Kassette in das Transkript, da dieses die Sequenzen der NMD-Kassette nur in der umgekehrten, nicht wirksamen Orientierung enthält. Die Prozessierung der prä-mRNA (nicht dargestellt) führt dann dazu, dass die Sequenzen als Bestandteil eines umgebenden Introns entfernt werden und in dem prozessierten Transkript nicht mehr enthalten sind.

Die jeweils in den Figuren 1b) und 1d) sowie 3b) und 3d) gezeigten Kästen geben schematisch die darüber gezeigten Elemente der doppelsträngigen Darstellung deckungsgleich wieder, wobei in Figur 1b) das Exon (4) als schwarzer Balken in der aktiven Orientierung, im kodogenen Strang eines Zielgens, in der oberen Hälfte und lesbar als "NMD" gekennzeichnet dargestellt ist. Figur 1d) zeigt die inaktive Orientierung von Exon (4) in der unteren Hälfte des Kastens und die "NMD"-Kennzeichnung auf dem Kopf von rechts nach links zu lesen. Die Spleißakzeptorstelle (7) und die Spleißdonorstelle (8) sind in den Figuren 1b) und 1d) sowie den Figuren 3b) und 3d) nicht explizit dargestellt, sondern bilden die Enden des Balkens der Exon (4) dargestellt; der erste und zweite Intronabschnitt (3) und (5) sind als weiße Bereiche gezeigt, die das Exon (4) beidseitig umrahmen. Das 5'-Ende (1) und das 3'-Ende (2) der NMD-Kassette sind durch die seitlichen Begrenzungen des Kastens dargestellt, der die gesamte NMD-Kassette repräsentiert. Der nicht kodierende Gegenstrang ist wegen seiner Funktionslosigkeit als paralleler weißer Teilbereich in der unteren Hälfte des Kastens dargestellt. Abgesehen von dem Verzweigungspunkt (6), werden die genannten Elemente ((1) bis (5) sowie (7) und (8)) in den folgenden Figuren zusammen mit der in den Figuren 1b) und 1d) sowie 3b) und 3d) schematischen Darstellung von NMD-Kassetten dargestellt.

Figur 2) zeigt die verschiedenen Möglichkeiten, wie eine NMD-Kassette in der bevorzugten Ausführung mit drei Stopkodons in ein Gen zu dessen Inaktivierung inseriert werden kann und stellt die Wirkungsweise des NMD vermittelnden Exons dar.

In Figur 2.1.a) ist dargestellt, dass die von einem natürlichen Gen mit drei Exons (A), (B), (C) transkribierte prä-mRNA (Figur 2.1.b)) vom Transkriptionsstartpunkt (TSP) bis zum polyA-Signal (polyA) sowohl die Exons (waagerecht), als auch die Intronabschnitte (abgewinkelt) enthält, wobei Spleißdonorstellen mit (sd) und Spleißakzeptorstellen mit (sa) gekennzeichnet sind. Die reife mRNA besteht nur noch aus den waagerecht dargestellten Exons einschließlich eines polyA-Schwanzes (nicht dargestellt), der post-transkriptionell angefügt wird.

In Figur 2.2.a) ist schematisch dargestellt, wie das Exon (B) durch Insertion einer erfindungsgemäßen NMD-Kassette durch das in der NMD-Kassette beinhaltete Exon (4) ersetzt wird. Dabei kann das Exon (4) von (B) abgeleitet sein (B') und auch die Intronabschnitte (3) und (5) identisch mit den entsprechenden Bereichen des Gens sein. In 2.2.a) ist die erfindungsgemäße NMD-Kassette im kodogenen Strang des Gens angeordnet. Das primäre Transkript, unter 2.2b) dargestellt, enthält das waagerecht dargestellte Exon (4) mit den damit verbundenen Spleißakzeptor- und Spleißdonorstellen (7) und (8), so dass das Spleißen der prä-mRNA dazu führt, dass die reife mRNA das mindestens eine Stopkodon des Exons (4) im Leserahmen aufweist, das als prämatures Terminationskodon erkannt wird und zur Degradation der mRNA führt.

Figur 2.3.a) zeigt die bevorzugte Ausführungsform, in der das inserierte Konstrukt aus erfindungsgemäßer NMD-Kassette aus Exon (4) mit flankierendem ersten und zweiten Intronabschnitt (3) und (5) in ein Intron des Zielgens integriert ist, wobei der erste und zweite Intronabschnitt (3) und (5) homologe Abschnitte des Introns des Zielgens ersetzen oder als zusätzliche intronische Sequenzen in das Intron des Zielgens eingeführt werden können. Im Ergebnis enthält das Zielgen nach der Insertion der NMD-Kassette das Exon (4) als zusätzliches Exon. Entsprechend enthält auch die davon transkribierte prä-mRNA in Figur 2.3b) das Exon (4) mit seiner Spleißakzeptor- (7) und Spleißdonorstelle (8), was beim Spleißen zu einem Transkript führt, in dem das Exon (4) vollständig enthalten ist und daher der NMD unterliegt.

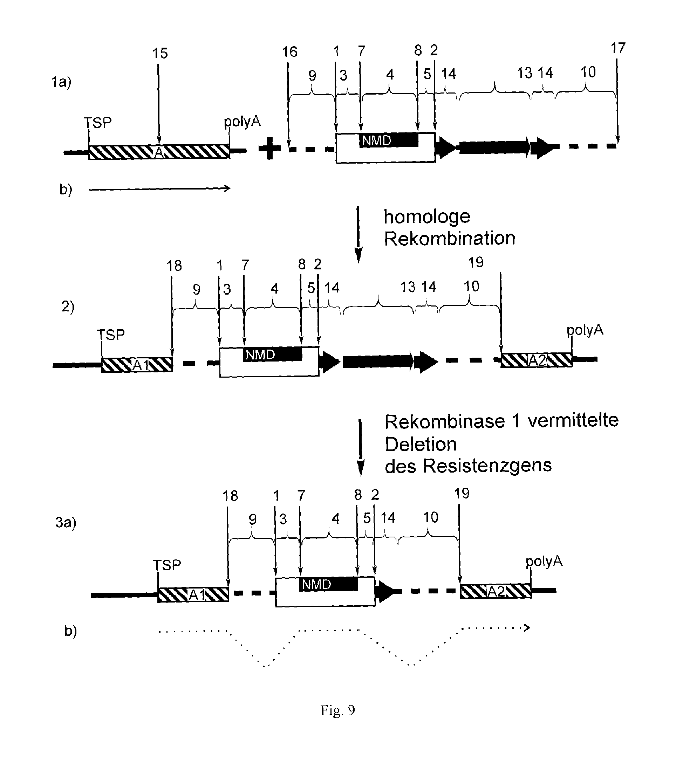

Figur 2.4a) zeigt die Situation, dass ein erfindungsgemäßes Nukleinsäurekonstrukt in ein Exon (B) eines Zielgens integriert ist. Erfindungsgemäße Nukleinsäurekonstrukte beinhalten für diesen Zweck neben dem in der der NMD-Kassette beinhalteten Exon (4) und erstem und zweitem Intronabschnitt (3), (5) zusätzliche, als unterbrochene Linie dargestellte, flankierende Intronabschnitte (9) und (10). Der zusätzliche dritte Intronabschnitt (9), in 5' vom ersten Intronabschnitt bzw. der minimalen NMD-Kassette gelegen, weist am 5' Ende die intronischen Anteile einer Spleißdonorstelle (hier nicht dargestellt, vergl. (16) in Figur 9)) auf. Der zusätzliche vierte Intronabschnitt (10), in 3' vom zweiten Intronabschnitt bzw. der minimalen NMD-Kassette gelegen, besitzt an seinem 3' Ende die intronischen Anteile einer Spleißakzeptorstelle (hier nicht dargestellt, vergl. (17) in Figur 9)) sowie in 5' von der Spleißakzeptorstelle einen funktionalen Polypyrimidinabschnitt und 5' davon einen funktionalen Verzweigungspunkt. Zusammen mit den Insertionspunkt flankierenden Exonsequenzen, die bevorzugt einer Protospleißstelle (hier nicht dargestellt, vergl. (15) in Figur 9)) entstammen, bilden die intronischen Anteile der Spleißstellen am 5'-Ende des Intronabschnitts (9) und am 3'-Ende des Intronabschnitte (10) eine funktionale Spleißdonorstelle (18) bzw. eine Spleißakzeptorstelle (19). Der erste Intronabschnitt (3) und der dritte Intronabschnitt (9) bzw. der zweite Intronabschnitt (5) und der vierte Intronabschnitt (10) bilden so jeweils vollständige funktionale Introns, die das Exon (4) in 5' bzw. 3' flankieren und das ursprüngliche Exon (B) des Zielgens in zwei funktionale Exons (B1) und (B2) teilen.

Das primäre Transkript (Figur 2.4b)) eines solchermaßen veränderten Zielgens enthält als zusätzliche Sequenz die vollständige NMD-Kassette, wobei deren erster und zweiter Intronbereich (3) und (5) jeweils vom zusätzlichen dritten bzw. vierten Intronabschnitt (9) bzw. (10) flankiert ist. Dies führt dazu, dass die Intronabschnitte (3) und (9) durch Spleißen zwischen der am 3'-Ende des durch die Insertion erzeugten Exons (B1) lokalisierten Spleißdonorstelle (18) und der Spleißakzeptorstelle (7) des Exon (4) als ein zusammenhängendes Intron entfernt werden. Die Spleißdonorstelle (8) am 3'-Ende des Exons (4) reagiert mit der Spleißakzeptorstelle (19) am 5'-Ende des durch die Insertion erzeugten Exons (B2), so dass auch die Intronabschnitte (5) und (10) als ein zusammenhängendes Intron aus dem Primärtranskript gespleißt werden. Dadurch gelangt das mindestens eine in Exon (4) beinhaltete Stopkodon in den Leserahmen, wird als prämatures Stopkodon erkannt und verursacht die NMD des Transkripts.

Figur 3) zeigt entsprechend Figur 1) schematisch die Ausführungsform der NMD-Kassette, in der das Exon (4) zusätzlich oder anstelle des mindestens eines Stopkodons eine nicht glatt durch 3 teilbare Anzahl von Nukleotiden, gekennzeichnet durch (L/3≠N), zur Verschiebung der Intronphase des Zielgens aufweist. Unter 3a) ist die aktive Orientierung im kodogenen Strang in doppelsträngiger Darstellungsweise gezeigt, unter 3c) die inaktive Orientierung. Die Figuren 3b) und 3d) zeigen dieselbe Kassette in ihrer aktiven bzw. inaktiven Orientierung in der schematischen Form.

In Figur 4) ist schematisch die Wirkungsweise der erfindungsgemäßen NMD-Kassette in der Ausführungsform gemäß Figur 3) dargestellt, in der das Exon (4) in Alternative zu der bevorzugten Ausführungsform kein Stopkodon enthält, aber eine Anzahl von Nukleotiden aufweist, die ein Vielfaches von 3 plus 1 oder plus 2 zusätzliche Nukleotide ist. Voraussetzung für die Anwendung dieser Ausführungsform ist, dass ein stromabwärts eines in das Zielgen eingeführten Exons (4) mit einer nicht durch 3 teilbaren Anzahl von Nukleotiden und ohne das bevorzugt vorgesehene mindestens eine Stopkodon ein Triplett in einem der stromabwärts folgenden Exons des Zielgens, ausgewählt aus TAA, TAG oder TGA, mehr als 50 bp 5' von der nächsten Spleißdonorstelle enthalten ist, das durch die durch Exon (4) verursachte Verschiebung der Intronphase in den Leserahmen gerät. Dabei ist es unerheblich, in welchem der stromabwärts auf Exon (4) folgenden Exons dies Triplett lokalisiert ist, so lange es sich dabei nicht um das 3'-terminale Exon des Zielgens handelt, da ein in einem 3'-terminalen Exon lokalisiertes Stopkodon wegen des Fehlens einer stromabwärts folgenden Spleißdonorstelle nicht in ein prämatures Stopkodon umgewandelt werden kann.

In Figur 4.1a) ist schematisch ein unverändertes Zielgen mit den Exons (A), (B) und (C) dargestellt, dessen primäres Transkript unter 4.1b) gezeigt ist. Die reife mRNA enthält nur die waagerecht dargestellten im Primärtranskript enthaltenen Exons. Das Exon (B) enthält die Basenfolge TAG, in Klammern angedeutet bevorzugt mehr als 50 bp beabstandet von der Spleißdonorstelle (sd) des Exons (B). Die Sequenz TAG liegt jedoch nicht im Leserahmen und wirkt daher nicht als Stopkodon. Ein in einer NMD-Kassette befindliches Exon (4) mit einer nicht durch 3 teilbaren Anzahl von Nukleotiden und ohne das erfindungsgemäße bevorzugte mindestens eine Stopkodon kann ein endogenes Exon des Zielgens ersetzen (nicht dargestellt) oder wie in Figur 4.2a) dargestellt als zusätzliches Exon in ein Intron des Zielgens eingeführt werden, oder in Verbindung mit den zusätzlichen Intronabschnitten (9) und (10) zur Insertion in ein Exon des Zielgens genutzt werden (Figur 4.3a)).

In Figur 4.2) ist gezeigt, wie eine integrierte erfindungsgemäße NMD-Kassette, die ein Exon (4) mit einer nicht durch drei teilbaren Anzahl von Nukleotiden und in der dargestellten Ausführungsform kein erfindungsgemäß bevorzugtes Stopkodon enthält, in das Intron zwischen den Exons (A) und (B) integriert ist, wobei der mit dem Exon (4) gekoppelte erste (3) und zweite Intronabschnitt, (5) der NMD-Kassette mit den aus dem Intron zwischen Exon (A) und (B) stammenden Intronbereichen des Zielgens verbunden sind

Die prä-mRNA, die unter 4.2b) dargestellt ist, wird durch Spleißen zu einer mRNA prozessiert, die nur noch die waagerecht gezeichneten Exons aufweist, und darin die Basenfolge TAG des genomischen Exons (B), die auf Grund der durch die nicht durch 3 teilbare Anzahl der Nukleotide in Exon (4) verursachten Verschiebung der Intronphase nun im Leserahmen liegt und als prämatures Stopkodon wirkt (fett und kursiv angedeutet). Da diese prämature Stopkodon vorzugsweise mehr als 50 bp von der Spleißdonorstelle (sd) des Exons (B) entfernt ist. Durch Spleißen entsteht hier ein Transkript, das durch Verschiebung der Intronphase ein prämatures Stopkodon in einem Exon enthält, hier am Beispiel des Exons B dargestellt, das nicht das 3'-terminale Exon des Gens ist. Dadurch unterliegt eine solche mRNA der NMD.

In Figur 4.3) ist der Fall dargestellt, dass das in der NMD-Kassette beinhaltete Exon (4) in der Ausführungsform mit einer nicht durch 3 teilbaren Anzahl von Nukleotiden und ohne das bevorzugte mindestens eine Stopkodon in ein Exon des Zielgens inseriert ist. Voraussetzung dafür ist wieder, dass das in 3' von Exon (4) folgende Exon nicht das 3'-terminale Exon des Gens ist und ein 50 bp 5' von der Spleißdonorstelle (sd) beabstandetes Triplett (TAG) enthält, das durch die von Exon (4) bedingte Verschiebung der Intronphase in ein prämatures Stopkodon umgewandelt wird.

Wie generell bei der Insertion in Exons vorgesehen, wird das NMD vermittelnde Exon (4) zusätzlich zu dem ersten (3) und zweiten Intronabschnitt (5) von einem dritten (9) und einem vierten (10) als unterbrochene Linie dargestellten Intronabschnitt flankiert, wie mit Bezug auf Figur 2 erläutert. Die am 5'-Ende von Intronabschnitt (9) und am 3'-Ende von Intronabschnitt (10) lokalisierten intronischen Anteile einer Spleißdonor- bzw. Spleißakzeptoranteile (nicht dargestellt) ergeben zusammen mit den den Insertionspunkt im Exon (B) flankierenden Sequenzen eine funktionale Spleißdonorstelle (18) und eine funktionale Spleißakzeptorstelle (19), wodurch das Exon (B) in die Exons (B1) und (B2) geteilt wird. Die Intronbereiche (9) und (3) sowie (5) und (10) wirken damit als vollständige Introns, die das NMD vermittelnde Exon (4) mit den neu erzeugten Exons (B1) und (B2) verbinden. Das in 3' von dem in der inserierten NMD-Kassette beinhalteten Exon (4) gelegene Exon (B2) mit der vorhandenen Basefolge TAG, die vorzugsweise mehr als 50 bp von der in 3' gelegenen Spleißdonorstelle des Exons entfernt ist, führt durch die Verschiebung der Intronphase wegen der nicht glatt durch 3 teilbaren Anzahl von Nukleotiden des Exons (4) ebenfalls zu einem Transkript, das beim Spleißen der Degradation unterliegt.

In den folgenden Figuren wird jeweils nur noch die in den Figuren 1) und 2) abgebildete bevorzugte Ausführungsform der Erfindung mit einem NMD vermittelnden Exon (4) dargestellt, das mindestens ein Stopkodon, bevorzugter je ein Stopkodon in jedem der drei für das Exon (4) möglichen Leserahmen aufweist, bevorzugt mindestens 50 bp beabstandet von der Spleißdonorstelle des Exons (4). Alle im folgenden dargestellten Ausführungsformen können jedoch auch mit einem Exon (4) ohne Stopkodons in Exon (4) hergestellt werden, wenn das Exon (4) in dieser Ausführungsform eine nicht durch 3 teilbare Anzahl von Nukleotiden aufweist, und die in Figur 3) und 4) beschriebenen Bedingungen zur Verwendung solcher Konstrukte erfüllt sind.

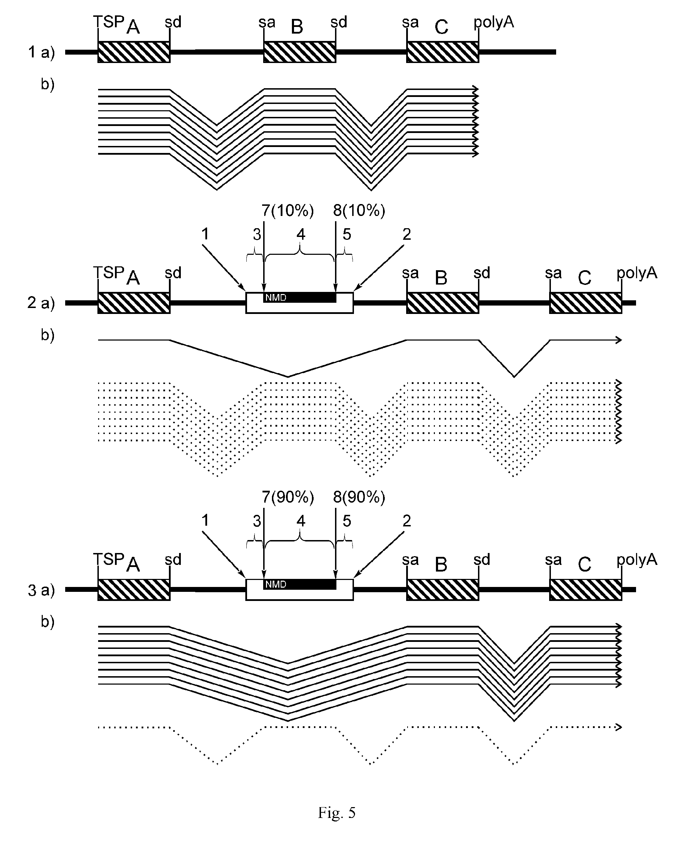

Figur 5) zeigt die Ausführungsform der vorliegenden Erfindung, in der die durch die NMD-Kassette vermittelte Degradation des Transkripts, nicht wie in den Figuren 2.2a) bis 2.4b) sowie in den Figuren 4.2a) bis 4.3b dargestellt, vollständig ist, sondern nur anteilig erfolgt. Während in den bevorzugten Ausführungsformen das Exon (4) als konstitutives Exon ausgelegt ist, kommen in den in Figur 5) dargestellten Fällen alternativ gespleißte Formen von Exon (4) zum Einsatz. Die durch alternativ gespleißte Formen von Exon (4) verursachte anteilige Degradation der mRNA führt dann in Bezug auf das Translationsprodukt zu hypomorphen Mutanten, wenn nicht andere posttranskriptionelle Prozesse den durch die anteilige Degradation der mRNA ausgelösten Effekt aufheben.

Zur anteiligen Degradation der mRNA eines Gens wird die erfindungsgemäße NMD-Kassette bevorzugt so verändert, dass die in ihr beinhaltete Spleißakzeptorstelle (7) und/oder die Spleißdonorstelle (8) funktional schwächer ausgewählt werden, so dass Exon (4) anteilig nicht als funktionales Exon wirkt und daher während des Spleißens zusammen mit den es flankierenden Introns aus dem Primärtranskript geschnitten wird, so dass das Exon (4) in einem Anteil der mRNA nicht mehr enthalten ist. Diese Situation ist schematisch in den Figuren 5.2) und 5.3) dargestellt.

Figur 5.1a) zeigt ein Gen, das keinem alternativen Spleißen unterliegt, weshalb alle seine Transkripte zu reifer mRNA prozessiert werden (Figur 5.1b)). In Figur 5.2a) sind die Spleißakzeptorstelle (7) und die Spleißdonorstelle (8) mit (10%) kennzeichnet, um die Reduktion der durch die NMD-Kassette vermittelten Degradation der mRNA auf ca. 10% anzuzeigen. Dies bedeutet, dass die Funktionalität der Spleißakzeptor- und Spleißdonorstellen (7(10%)) und (8(10%)) um ca. 90% gegenüber einer vollständig funktionalen Spleißakzeptor- und/oder Spleißdonorstelle vermindert ist, was zum alternativen Spleißen der Transkripte führt, wie schematisch unter 5.2b) dargestellt. Dabei zeigt Fig. 5.2b) als punktierte Linien die 90% der primären Transkripte, in denen das in der der NMD-Kassette beinhaltete Exon (4) als Exon erkannt wird, so dass das darin beinhaltete mindestens eine Stopkodon als prämatures Stopkodon wirkt, so dass es schließlich zur Nonsense vermittelten Degradation dieser Transkripte kommt. Die durchgezogene Linie zeigt die Transkripte, in denen die eingesetzten Spleißakzeptor- und Spleißdonorstellen (7(10%)) bzw. (8(10%)) nicht reagieren. In diesem Fall wird das Exon (4) der NMD-Kassette nicht als solches erkannt und zusammen mit den es flankierenden Introns vollständig aus dem Transkript entfernt wird, so dass dieses nicht dem NMD unterliegt und zur Translation des Genproduktes genutzt werden kann.

Figur 5.3a) zeigt die Situation, in der funktionale Elemente der NMD-Kassette, die am Spleißvorgang teilnehmen, weniger in ihrer Funktionalität eingeschränkt sind als in Figur 5.2a). Am Beispiel der Spleißakzeptor- und Spleißdonorstellen (7(90%)) bzw. (8(90%)) wird gezeigt, dass eine Verminderung der Funktionalität von Sequenzelementen der NMD-Kassette, die am Spleißvorgang beteiligt sind, um 10% dazu führt, dass nur 10% der primären Transkripte das NMD vermittelnde Exon (4) enthalten und daher der Degradation unterliegen, während die verbleibenden 90% der primären Transkripte auf Grund der reduzierten Funktionalität der am Spleißvorgang beteiligten Elemente der NMD-Kassette so prozessiert werden, dass das Exon (4) zusammen mit den es flankierenden Introns aus den Transkript herausgespleißt wird. Transkripte, aus denen Exon (4) herausgespleißt wird enthalten kein erfindungsgemäßes prämatures Stopkodon und können deshalb zur maturen mRNA prozessiert werden, die zur Translation zur Verfügung steht (Figur 5.3b).

Für die Zwecke der Erfindung können funktionale Elemente der NMD-Kassette mit verminderter Funktionalität beim Spleißen aus natürlichen Genen gewonnen werden, die alternativem Spleißen unterliegen, beispielsweise Spleißakzeptor- und Spleißdonorstellen verminderter Funktionalität aus Genen, in denen ein nicht-terminales Exon aus einem Anteil der primären Transkripte durch Prozessieren entfernt wird und in einem Anteil der reifen mRNA nicht enthalten ist. Alternativ können auch Verzweigungspunkte und Polypyrimidinabschnitte mit verminderter Funktionalität für die Herstellung alternativ erfindungsgemäßer NMD vermittelnder Exons benutzt werden. Als weitere Alternative sieht die Erfindung zur Herstellung alternativ gespleißter NMD vermittelnder Exons die Verwendung sogenannter Spleiß-"silencer" vor, die als exonische Spleiß-"silencer" Bestandteil eines erfindungsgemäßen NMD vermittelnden Exons sein können, oder als intronische Spleiß-"silencer" in Intronbereichen erfindungsgemäßer Nukleinsäurekonstrukte lokalisiert sein können.

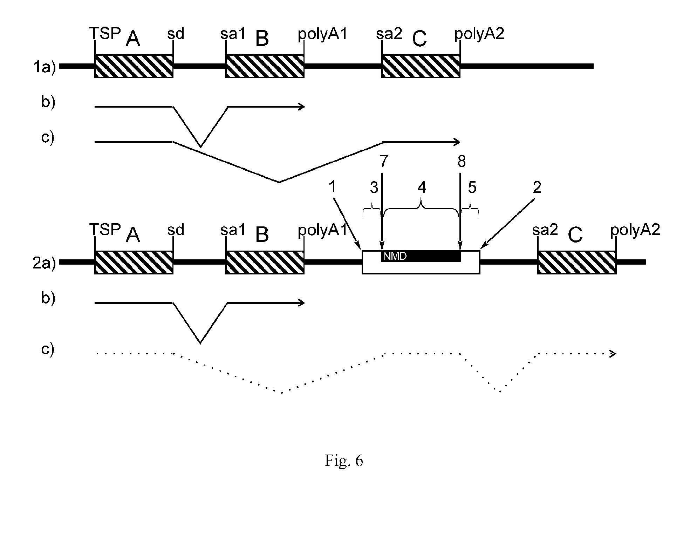

Als eine Ausführungsform der vorliegenden Erfindung zur vollständigen oder anteiligen Inaktivierung der von Genen transkribierten und prozessierten mRNAs, im Rahmen der Erfindung auch als Inaktivierung von Genen bezeichnet, können die Konstrukte zur anteiligen oder vollständigen Inaktivierung einzelner Spleißvarianten eines Zielgens verwendet werden. Dies ist schematisch in Figur 6) am Beispiel der vollständigen Inaktivierung einer Spleißvariante eines Gens schematisch gezeigt.

Figur 6.1a) zeigt am Beispiel eines Gens mit alternativ genutzten 3'-terminalen Exons (B) und (C), die jeweils durch eine spezifische Spleißakzeptorstelle (sa1) bzw. (sa2) und ein eigenes Polyadenylierungssignal (polyA1) bzw. (polyA2) definiert werden. Als Spleißdonorstelle wirkt jeweils die Spleißdonorstelle (sd) des Exons (A). Die alternativen Transkripte des unveränderten Gens sind schematisch in Figur 6.1b) und 6.1c) dargestellt, die jeweils das gemeinsame Exon (A) und eines der Exons (B) oder (C) enthalten.

In Figur 6.2a) dargestellt, wie ein erfindungsgemäßes Exon (4) einschließlich flankierender erster (3) und zweiter Intronabschnitte (5) einer NMD-Kassette in das Intron des Zielgens integriert ist, das zwischen den alternativ gespleißten Exons (B) und (C) angeordnet ist.

Unter 6.2b) ist mit der durchgezogenen Linie schematisch das primäre Transkript gezeichnet, das das in 5' gelegene alternative Exon (B), enthält. Dieses primäre Transkript wird durch Herausschneiden der abgewinkelt dargestellten Intronabschnitte zu einer mRNA prozessiert, die nur die Exons (A) und (B) enthält, letzteres mit seiner spezifischen polyA-Sequenz. Unter 6.2c) ist das alternative primäre Transkript dargestellt, in welchem das erfindungsgemäße NMD vermittelnde Exon (4) enthalten ist. Beim Spleißen wird das in Exon (4) enthaltene prämature Stopkodon erkannt und entsprechende Transkripte der NMD zugeführt. Im Ergebnis wird nur noch das von der die Exons (A) und (B) beinhaltenden mRNA kodierte Genprodukt synthetisiert.

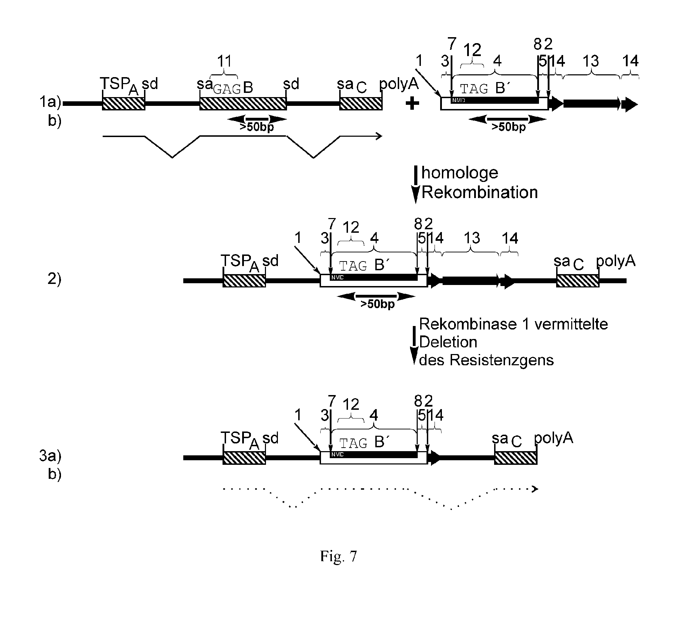

Die Figuren 7) bis 9) zeigen die Möglichkeiten, wie erfindungsgemäße NMD-Kassetten in Zielgene eines eukaryontischen Organismus eingebracht werden können. Es handelt sich dabei um das Ersetzen eines endogenen Exons durch ein erfindungsgemäßes NMD vermittelndes Exon, die Insertion einer erfindungsgemäßen NMD-Kassette in ein Intron sowie die Insertion einer erfindungsgemäßen NMD-Kassette in ein Exon eines Zielgens. Während die Insertion in ein Intron gezielt mittels homologer Rekombination oder zufällig in Form einer Genfalle oder eines Transposons erfolgen kann, ist für das Ersetzen eines endogenen Exons durch ein NMD vermittelndes Exon sowie für die Insertion einer NMD-Kassette in ein Exon die gezielte Mutagenese mittels homologer Rekombination bevorzugt. In den Figuren 7) bis 9) sind zunächst nur Verfahren zur direkten Herstellung kompletter Geninaktivierung dargestellt. Die gleichen Verfahren können aber mit Konstrukten, die erfindungsgemäße Erkennungssequenzen für Rekombinasen beinhalten, auch zur Herstellung konditionaler Mutanten angewendet werden.

Figur 7) zeigt eine mögliche Vorgehensweise zur Erzeugung eines NMD vermittelnden Exons in einem Gen einer Zielzelle, wobei das erfindungsgemäße Exon (4) ein natürlicherweise in dem Zielgen vorhandenes Exon (B) ersetzt. Gemäß der bevorzugten Ausführungsform weist das zur genetischen Manipulation eingesetzte Nukleinsäurekonstrukt ein Selektionsmarkergen (13) auf, beispielsweise ein Resistenzgen, das weiter bevorzugt von gleichgerichteten Erkennungssequenzen für eine Rekombinase oder Integrase (14) flankiert ist, mit der das Selektionsmarkergen selektiv aus dem Genom entfernt werden kann.

Die Erzeugung der erfindungsgemäßen NMD-Kassette im Zielgen erfolgt hier durch die Erstellung eines prämaturen Stopkodons (12) innerhalb eines natürlichen Exons durch homologe Rekombination eines DNA-Fragments, das eine zur Rekombination mit dem natürlichen Gen hinreichende Homologie und Länge aufweist, sowie einen veränderten, in diesem Fall von dem Kodon GAG (11) des Exons (B) abgeleitete Sequenzabschnitt TAG (12), im Abstand von mindestens 50 bp von der Spleißdonorstelle (8). Alternativ können prämature Stopkodons auch durch Austausch von zwei, drei oder mehr Nukleotiden bzw. durch Insertion von ein bis drei zusätzlichen Nukleotiden erzeugt werden. Zur Selektion genetisch manipulierter Zielzellen, d.h. von Transformanten, ist es bevorzugt, dass das in die Zielzelle einzubringende DNA-Fragment mit einem Selektionsmarkergen gekoppelt ist, wie in Figur 7.2) als Resistenzgen (13) beispielhaft dargestellt. Durch Aufbringen des spezifischen Selektionsdrucks ist es damit möglich, Mutanten zu selektieren und zu isolieren. Das Resistenzmarkergen (13) läßt sich mittels der flankierenden Erkennungssequenzen für eine Integrase oder Rekombinase (14) selektiv aus dem Insertionsort entfernen, wonach eine einzelne Erkennungssequenz (14) im Lokus verbleibt. Das in dem so erzeugten Exon (B') enthaltene prämature Stopkodon wird bei der Prozessierung entsprechender Transkripte erkannt, was zur Degradation derselben mittels NMD führt. Dies ist schematisch in Figur 7.3b) dargestellt.

In Fortbildung des in Figur 7) dargestellten Verfahrens zur Erzeugung eines prämaturen Stopkodons durch Ersetzen eines Exons (B) des Zielgens mit einem von (B) abgeleiteten Exon (B') sieht die Erfindung die Nutzung nicht mit dem zu ersetzenden Exon (B) homologer Exons vor, die ein prämatures Stopkodon enthalten. Diese können von anderen Exons des Zielgens, von Exons anderer Gene des Zielorganismus oder von Exons aus einer anderen Spezies abgeleitet oder rein synthetischer Natur sein.

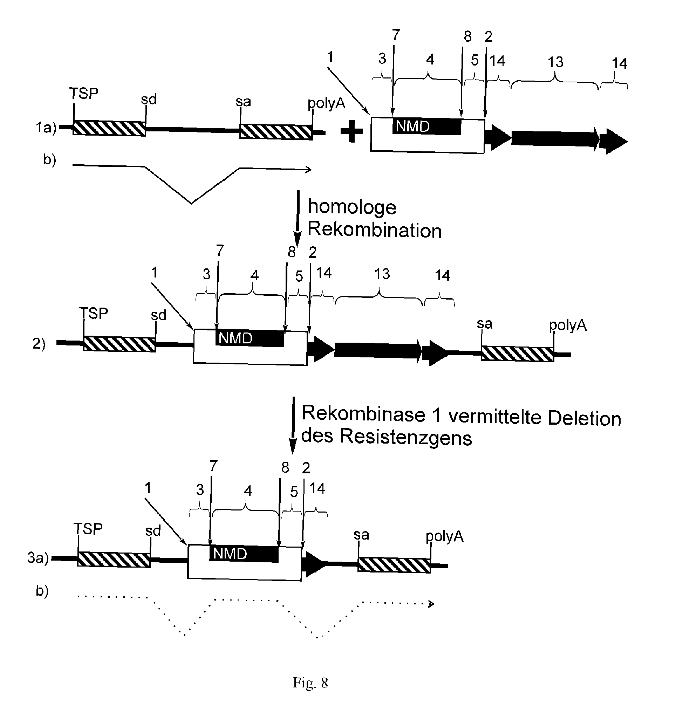

In Figur 8) ist schematisch die erfinderisch bevorzugte Insertion einer erfindungsgemäßen NMD-Kassette in ein Intron eines Gens einer Zielzelle dargestellt. Dabei kann die Insertion gezielt mittels homologer Rekombination erfolgen oder das Ergebnis einer zufälligen Insertion sein, wie sie bei der Verwendung von Genfallen und Transposons auftritt.

Vorzugsweise wird die Insertion in ein Intron eines Zielgens in ES-Zellen der Maus zur vollständigen Geninaktivierung durchgeführt wird. Zur Insertion der NMD-Kassette, die gemäß der bevorzugten Ausführungsform der Erfindung mit einem Resistenzgen (13) verbunden ist, das beidseitig von Erkennungssequenzen (14) für eine Rekombinase oder Integrase flankiert ist, sind diese Bereiche mit DNA-Fragmenten (nicht dargestellt) flankiert, die homolog zu den stromaufwärts bzw. stromabwärts des gewünschten Insertionspunkts vorhandenen Sequenzen des Zielgens sind. Vermittelt durch die homologe Rekombination erfolgt die Insertion des erfindungsgemäßen Nukleinsäurekonstrukts aus NMD-Kassette und angrenzendem Selektionsmarkergen (13) in ein Intron des Zielgens.

Als Selektionsmarkergene (13) können statt eines Resistenzgens, wie beispielsweise das Neomycin-Resistenzgen, auch solche eingesetzt werden, die eine positive und/oder negative Selektion erlauben, wie beispielsweise Fusionsproteine eines Puromycin-Resistenzgens mit dem Thymidinkinasegen aus Herpes simplex (Positivselektion mit Puromycin, Negativselektion mit Ganciclovir) oder eine Expressionskassette für Hypoxanthin-Guanosin-Phosphoribosyl-Transferase (HPRT) für die in ES-Zellen ohne funktionales endogenes HPRT-Gen positiv mit 6-Thioguanosin und negativ mit HAT-Medium selektiert werden.

Mit dem für das Selektionsmarkergen spezifischen Selektionsmedium können Transformanten selektiert und angereichert werden, so dass es möglich ist, einzelne Klone zu isolieren und bezüglich des Vorhandenseins der gewünschten Mutation untersucht werden. Figur 8.2) zeigt die Organisation eines mutierten Allels des Zielgens nach erfolgter Insertion der NMD-Kassette.

In Gegenwart der Rekombinase oder Integrase, die für die Erkennungssequenzen (14) spezifisch ist, die das Selektionsmarkergen (13) flankieren, wird das Selektionsmarkergen (13) deletiert und ist anschließend nicht mehr im Genom der Zielzelle vorhanden. Figur 9.3b) zeigt, dass die reife mRNA des Zielgens mit im Intron integrierter NMD-Kassette enthält das NMD vermittelnde Exon (4) während die vollständigen Introns, die von den Intronabschnitten (3) und (4) der NMD-Kassette und von den die Intronabschnitte (3) und (4) flankierenden Bereichen des Introns, in das die Insertion erfolgte, gebildet werden, beim Spleißen aus dem Transkript entfernt werden. In der Folge enthält die prozessierte RNA ein prämatures Stopkodon, vorzugsweise drei, eins für jeden Leserahmen, und/oder weist eine Verschiebung der Intronphase auf, was jeweils zur Degradation der mRNA mittels NMD führt.

Figur 9) zeigt, wie eine erfindungsgemäße NMD-Kassette zur vollständigen Inaktivierung eines Zielgens durch Insertion in ein Exon verwendet wird. Die Darstellung bezieht sich auf die bevorzugt für diese Anwendung vorgesehenen Ein-Exon-Gene, die durch das Fehlen von Introns ausgezeichnet sind. Eine Insertion in ein Exon kann aber auch in Genen erfolgen, die zwei oder mehrere Exons und somit auch Introns aufweisen, z.B. in Fällen, in denen Introns essentielle regulatorische Elemente beinhalten und eine Insertion einer NMD-Kassette zur Inaktivierung dieser Elemente führen würde.

In Figur 9) ist unter 1a) dargestellt, wie das Gen vom Transkriptionsstartpunkt (TSP) zu seinem Polyadenylierungssignal transkribiert wird, um das primäre Transkript, dargestellt unter b), zu erhalten. Die Insertion des Konstrukts erfolgt bevorzugt in eine in der kodierenden Sequenz des Gens befindliche Protospleißstelle (15), die die Sequenz CMAGR aufweist.

Das Konstrukt enthält neben der erfindungsgemäßen NMD-Kassette mit erstem Intronabschnitt (3), Exon (4) und Intronabschnitt (5) in 5' einen dritten flankierenden Intronabschnitt (9), sowie in 3' einen flankierenden vierten Intronabschnitt (10). Zwischen zweitem Intronabschnitt (5) und viertem Intronabschnitt (10) ist ein Resistenzmarkergen (13), eingefaßt von gleichgerichteten Erkennungssequenzen (14) angeordnet. Das 5'-Ende des Konstrukts wird von den intronischen Anteilen einer Spleißdonorstelle (16) des dritten Intronabschnitts (9) gebildet, das 3'-Ende von den intronischen Anteilen einer Spleißakzeptorstelle (17) des vierten Intronabschnitts (10).

Durch die in Figur 9.2b) dargestellte Insertion des Konstruktes in die Protospleißstelle des Zielgens (15), vermittelt durch nicht dargestellte die genannten funktionalen Elemente des Konstruktes flankierende homologe Bereiche, wird das Exon (A) des Zielgens in zwei funktionale Exons (A1) und (A2) geteilt. Im dargestellten Beispiel handelt es sich um zwei terminale Exons von denen das eine (A1) von einem Transkriptionsstartpunkt (TSP) und einer Spleißdonorstelle (sd) und das andere von einer Spleißakzeptorstelle (sd) und dem polyA-Signal (polyA) begrenzt werden. Im nicht dargestellten Fall der Insertion einer erfindungsgemäßen NMD-Kassette in ein nicht-terminales Exon würden statt dessen zwei nicht-terminale Exons das NMD vermittelnde Exon flankieren.

Durch Anwesenheit der Rekombinase, die für die Erkennungssequenzen (14) spezifisch ist, die das Selektionsmarkergen (13) flankieren, wird letzteres aus dem betroffenen Allel entfernt. Das Ergebnis ist unter 3a) dargestellt. Das entsprechende primäre Transkript (Figur 9.3b)) enthält die Sequenzen der beiden aus dem ursprünglichen Exon (A) hervorgegangenen Exons (A1) und (A2), das NMD vermittelnde Exon (4) und die flankierenden, vollständige Introns bildenden, ersten und dritten (3, 9) bzw. zweiten und vierten (5, 10) Intronabschnitte sowie die verbleibende Erkennungssequenz (14) der Rekombinase, mittels derer das Selektionsmarkergen (13) eliminiert wurde. Beim Spleißen wird das prämature Stopkodon im Exon (4) von der NMD-Maschinerie erkannt und das Transkript der Degradation zugeführt.

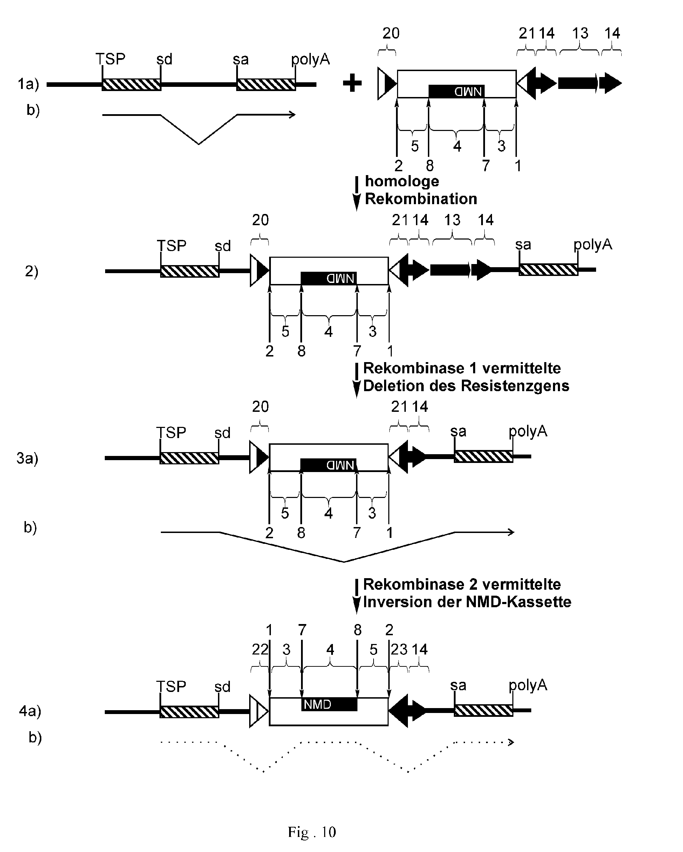

In Fortbildung sieht die Erfindung die konditionale Inaktivierung von Genen, die konditionale Reaktivierung zuvor inaktivierter Gene sowie die mehrfache konditionale Änderung des Aktivitätszustandes von Genen mittels erfindungsgemäßer NMD-Kassetten vor. Die dafür notwendigen Verfahren sind in den nachfolgenden Figuren 10) bis 15) schematisch dargestellt. Dabei wird jeweils nur der Fall einer in ein Intron inserierten NMD-Kassette gezeigt. Die Erfindung sieht diese Anwendungen aber auch für die Fälle vor, in denen ein endogenes Exon durch ein erfindungsgemäßes Exon ersetzt wird, oder eine NMD-Kassette in ein Exon eines Genes inseriert wird, da beide Verfahren wie die in den Figuren 7) und 9) beschrieben ebenfalls darauf beruhen, dass ein erfindungsgemäßes NMD vermittelndes Exon von vollständigen funktionalen Introns flankiert wird.

Die Verfahren zur konditionalen Änderung des Aktivitätszustandes von Genen werden, wie in den Figuren 10) bis 15) dargestellt, für die Anwendung in den endogen Gene eines Zielorganismus bevorzugt. Die Erfindung sieht aber auch die Anwendung in nicht-endogenen Genen vor, die als Transgen in das Genom des Zielorganismus inseriert wurden.

Weiterhin ist die in den Figuren 10) bis 15) nur die bevorzugte Insertion erfindungsgemäßer Kassetten in Introns von Genen mittels homologer Rekombination dargestellt. Die in Figuren (10) bis (15) dargestellten Verfahren zur konditionalen Änderung des Aktivitätszustandes eukaryontischer Gene mittels Verwendung von Rekombinasen sind aber auch für Gene anwendbar, in die erfindungsgemäße NMD-Kassetten zufällig inseriert wurden, z.B. in Form Genfallen, retroviraler Vektoren und Transposons, die NMD-Kassetten beinhalten.

Die in den Figuren 10) bis 15) jeweils unter Punkt 2) dargestellte Orientierung des Konstrukts in den kodierenden oder nicht-kodierenden Strang des Zielgens nach erfolgter homolger Rekombination wird erfindungsgemäß durch die Anordnung zum Zielgen homologer DNA-Abschnitte vorgegeben, die die NMD-Kassette und das damit verbundene Resistenzgen flankieren. Die Orientierung der NMD-Kassette in den entsprechenden Konstrukten ist in den Figuren 10) bis 15) jeweils rechts unter Punkt 1) dargestellt. In Abhängigkeit von der Orientierung in der die NMD-Kassette in ein Gen inseriert wird, ist das unmittelbare Ergebnis ein inaktiviertes oder nicht beeinflußtes Zielgen. Zur Vermeidung von Einflüssen des Resistenzmarkergens (13) wird dieses nach Selektion manipulierter Zielzellen durch Aktivität der Rekombinase 1, die für die flankierenden Erkennungssequenzen (14) spezifisch ist, deletiert.

Für die Änderung des Aktivitätszustandes sieht die Erfindung zum einen die rekombinasevermittelte Inversion einer erfindungsgemäßen NMD-Kassette vor, wodurch deren funktionalen Elemente in den jeweils komplementären Strang des Zielgens gelangen. Im Fall, dass sich die funktionalen Elemente der NMD-Kassette zunächst im kodogenen Strang des Zielgens befinden, sieht die Erfindung als Alternative die rekombinasevermittelte Deletion der NMD-Kassette zur Reaktivierung des Zielgens vor.

Die Erfindung bevorzugt die Inversion und Deletion erfindungsgemäßer NMD-Kassetten *in vivo* in Genen, in die sie inseriert wurden. Die Erfindung sieht aber auch die Inversion in die betreffende Rekombinase exprimierenden Bakterien und *in vitro* durch Kontaktieren mit der spezifischen Rekombinase vor. Dies ist z.B. vorteilhaft, wenn ein Nukleinsäurekonstrukt zum Einführen einer NMD-Kassette in einen genomischen Lokus mittels homologer Rekombination die Kassette nicht in der benötigten Orientierung enthält

Die Darstellung der konditionaler Ausführungsformen der Erfindung in den Figuren 10) bis 15 erfolgt jeweils an einem aus zwei Exons bestehenden Gen, das jeweils unter Punkt 1a) links dargestellt ist. Das entsprechende Transkript findet sich jeweils unter Punkt 1b). Rechts daneben ist das jeweils zur Mutagenese verwendete Konstrukt dargestellt. Unter Punkt 2) wird die genomische Organisation des Zielgens nach Insertion des zur Mutagenese verwendeten Konstruktes dargestellt. Punkt 3a) zeigt jeweils die genomische Organisation des Zielgens nach rekombinase- oder integrasevermittelter Deletion des in den zur Mutagenese verwendeten Nukleinsäurekonstrukten beinhalteten Resistenzgens (13). Unter Punkt 3b) ist das Transkript dargestellt, das von dem mutierten Gen transkribiert wird. Transkripte, die zu reifer mRNA prozessiert werden, sind mit durchgezogenen Linien dargestellt. Der NMD unterliegende Transkripte werden mit gepunkteten Linien gezeigt.

Ein Beispiel ist in Figur 10 gezeigt, wo schematisch bei 1a) ein Gen mit zwei Exons und 1b) dessen primäres Transkript, sowie unter 2) dasselbe Gen nach Insertion einer erfindungsgemäßen NMD-Kassette, die von Erkennungssequenzen für eine Rekombinase (20, 21) flankiert und mit einem Resistenzgen als Selektionsmarkergen (13) verbunden ist, das von Erkennungssequenzen für eine andere Rekombinase oder Integrase (14) flankiert ist. Die NMD-Kassette ist hier zunächst in ihrer inaktiven Orientierung in das Zielgen integriert, d.h. dass die funktionellen Elemente im nicht-kodogenen Strang des Gens lokalisiert sind, so dass sie nicht am Spleißvorgang des primären Transkripts teilnehmen können. Das Gen wird deshalb zunächst normal transkribiert und gespleißt und das Genprodukt exprimiert.

Zur zielgerichteten Insertion der NMD-Kassette einschließlich des mit ihr gekoppelten Selektionsmarkergens weist das Konstrukt jeweils endständig flankierende DNA-Sequenzabschnitte (nicht dargestellt) auf, die zu an den gewünschten Insertionsort angrenzende Abschnitten des Gens homolog sind und eine ausreichende Länge aufweisen, um die homologe Rekombination zu ermöglichen.

Das Resistenzgen ist so vollständig mit regulatorischen Elementen versehen, dass es unabhängig vom Insertionsort seinen Phänotyp exprimieren kann und zur Selektion von genetisch manipulierten Zellen verwendet werden kann.

Die NMD-Kassette ist von Erkennungssequenzen für eine Rekombinase (20, 21) flankiert, die entsprechend der bevorzugten Ausführungsform so angeordnet sind, dass sie bei Wechselwirkung mit ihrer spezifischen Rekombinase eine einmalige, d.h. irreversible Inversion ermöglichen. Die Irreversibilität der Inversion kann durch Verwendung von Mutanten natürlicher Erkennungssequenzen für Rekombinasen realisiert werden, beispielsweise Mutanten von loxP oder FRT, bei denen durch die Inversion die Erkennungssequenzen so verändert werden, dass sie anschließend nicht mehr zusammen von der Rekombinase genutzt werden können. Dies ist durch die schwarz-weiße Darstellung der Erkennungssequenzen angedeutet. Der jeweils andersfarbige innere Teil der Pfeilspitzen entspricht den Anteilen der Erkennungssequenzen, die an der Inversion teilnehmen und mit den äußeren Anteilen der jeweils anderen Erkennungssequenzen zu Erkennungssequenzen mit veränderter Basenabfolge rekombiniert.

Als eine Option ist es bevorzugt, das Selektionsmarkergen (13) durch die Gegenwart der Rekombinase oder Integrase zu deletieren, die für die letzteres flankierende Erkennungssequenzen (14) spezifisch ist. Das Ergebnis der Deletion des Selektionsmarkergens (13) ist unter 3a) dargestellt, wobei das primäre Transkript zwischen den Exons des ursprünglichen Zielgens als Teil der Intron-Sequenz die NMD-Kassette in ihrer inaktiven Orientierung enthält. Unter 3b) ist dargestellt, wie die NMD-Kassette als Teil des Introns aus dem Primärtranskript gespleißt wird, wodurch die reife mRNA des Zielgens unverändert bleibt und zur Expression des Genproduktes führt.

Die Aktivierung der NMD-Kassette zur Inaktivierung des Zielgens durch Anwesenheit der für die flankierenden Erkennungssequenzen (20, 21) spezifischen Rekombinase ist in Figur 10.4) dargestellt. Wie schematisch durch die schwarz bzw. weiß markierten Abschnitte der Erkennungssequenzen (20) bzw. (21) dargestellt wird, verändert die Invertierung beide Erkennungssequenzen, so dass eine anschließende Wechselwirkung mit dieser Rekombinase unmöglich wird. In der Folge ist die Invertierung der NMD-Kassette irreversibel und führt nun zur Degradation der mRNA, die durch Prozessieren aus dem primären Transkript entsteht, das unter b) dargestellt ist. Denn das Transkript enthält den Exon (4) der NMD-Kassette, so dass das darin beinhaltete prämature Stopkodon die Degradation der RNA auslöst.

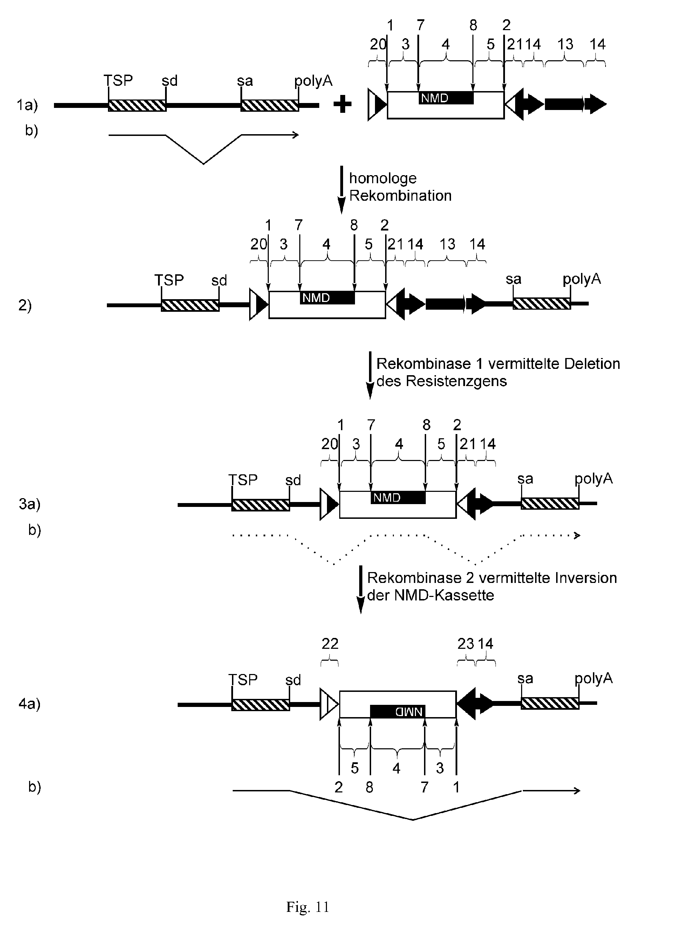

In der in Figur (11) dargestellten Ausführungsform der Erfindung wird eine von Erkennungssequenzen (20, 21) flankierte NMD-Kassette, die mit einem Resistenzmarkergen 13 gekoppelt ist, so in ein Zielgen inseriert, dass die funktionalen Elemente der NMD-Kassette im kodogenen Strang des Zielgens lokalisiert sind (Figur 11.2)). Wie auch in Figur 10) dargestellt ist, wird die Anwesenheit der Rekombinase oder Integrase für die Erkennungssequenzen (14), die das Resistenzmarkergen (13) flankieren, zu dessen Deletion genutzt.

Figur 11.3a) zeigt, das die Anwesenheit des NMD-vermittelnden Exons (4) im kodogenen Strang des Gens dessen unmittelbare Inaktivierung die Folge hat, da das in Exon (4) enthaltenen prämature Stopkodon die in Figur 11.3b) dargestellte NMD auslöst.
Figur 11.4) zeigt, dass die Anwesenheit der für die die NMD-Kassette flankierenden Erkennungssequenzen (20, 21) spezifischen Rekombinase 2 zur Invertierung der NMD-Kassette führt und damit zu deren inaktiven Orientierung und zur Anordnung ihrer funktionalen Elemente im nicht-kodogenen Strang des Gens. Als Ergebnis führt die Invertierung der NMD-Kassette in dem in Figur 11) dargestellten Fall also zur Reaktivierung eines zuvor durch eine NMD-Kassette inaktivierten Gens.

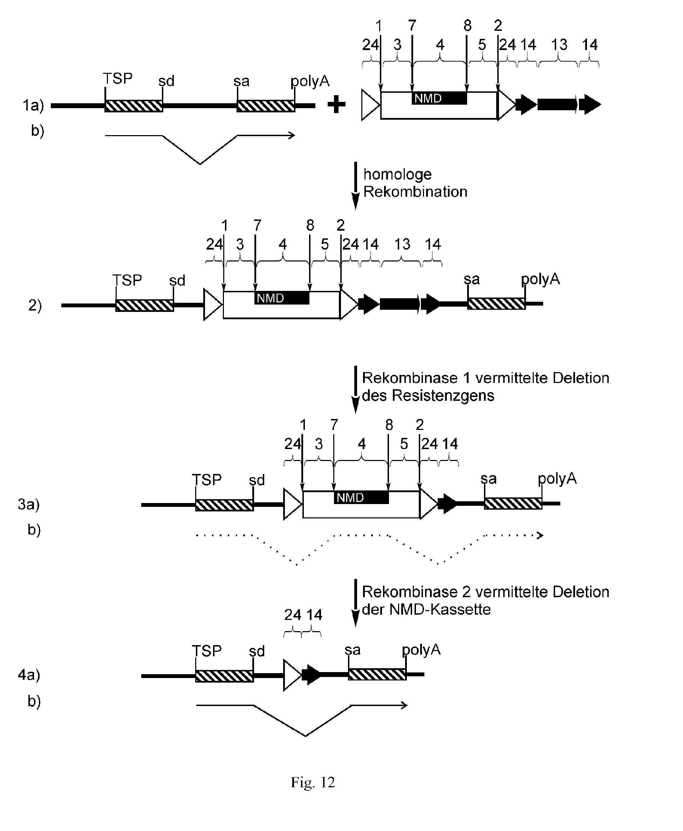

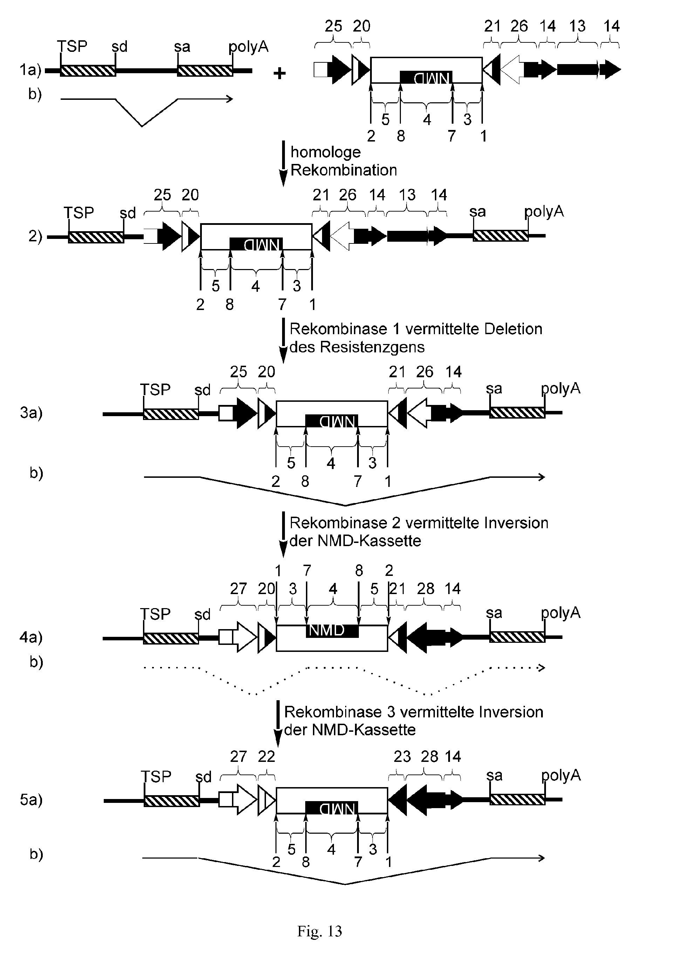

Ein weitere Ausführungsform zur konditionalen Reaktivierung eukaryontischer Gene ist in Figur 12) dargestellt. Dabei erfolgt die initiale Inaktivierung des Zielgens dadurch, das die funktionalen Elemente einer NMD-Kassette mit gleich orientierten Erkennungssequenzen für eine Rekombinase (24) flankiert im kodogenen Strang des Zielgens angeordnet werden (Figur 12.3a)), wodurch die Transkripte des mutierten Gens der NMD unterliegen (Figur 12.3b). Anwesenheit der Rekombinase 2 führt zur Deletion der von den Erkennungssequenzen (24) für diese mit Erkennungssequenzen für Rekombinase flankierten NMD-Kassette, wonach im Intron des Zielgens eine Erkennungssequenz (24) verbleibt (Figur 13.4a)). Abgesehen von dieser Erkennungssequenz (24) und der ebenfalls im Intron verbleibenden, von der Deletion des Resistenzgens herrührenden Erkennungssequenz (14) wird durch die Deletion der NMD-Kassette der Zustand des Wildtypgens wiederhergestellt, d.h. das Gen wurde reaktiviert und exprimiert die reife mRNA.

Die Deletion der NMD-Kassette bietet den Vorteil, dass nur kurze Sequenzabschnitte des eingebrachten Nukleinsäurekonstrukts im Genom der Zielzelle verbleiben. Außerdem bietet die Deletion den wesentlichen Vorteil, dass dafür auch Rekombinasen verwendet werden können, für die bisher keine zur einmaligen irreversiblen Invertierung geeignete Erkennungssequenzen verfügbar sind, wie z.B. der Rekombinase.

Die Figuren 13) bis 15) zeigen in Fortbildung der in den Figuren 10) bis 12) dargestellten Verfahren die Verwendung erfindungsgemäßer Nukleinsäurekonstrukte zur mehrfach konditionalen Änderung des Aktivitätszustandes eukaryontischer Gene. Dabei können die Gene zunächst, d.h. vor Anwendung einer Rekombinase, aktiv sein, d.h., dass sich die funktionalen Elemente der NMD-Kassette im nicht-kodogenen Strang des Gens befinden, oder durch die Anwesenheit der funktionalen Elemente einer NMD-Kassette im kodogenen Strang des Gens inaktiviert vorliegen.

Die mehrfache Konditionalität von NMD-Kassetten wird bevorzugt dadurch erreicht, dass die NMD-Kassette neben einem Paar Erkennungssequenzen (20, 21) für eine erste Rekombinase von zusätzlichen Paaren von Erkennungssequenzen für eine oder mehrere andere Rekombinasen flankiert ist. Die Erfindung sieht für die Herstellung mehrfacher Konditionalität mittels rekombinasevermittelter Inversion und Deletion optional weitere andere Anordnungen von Erkennungssequenzen für Rekombinasen vor, die hier nicht dargestellt werden. Z.B. kann die Anordnung so erfolgen, dass auf einer Seite der NMD-Kassette nur eine Erkennungssequenz für eine Rekombinase vorliegt und die zweite Erkennungssequenz für diese erste Rekombinase auf der anderen Seite der NMD-Kassette von Erkennungssequenzen für eine zweite andere Rekombinase flankiert wird. Eine weitere vorgesehene Alternative zur Herstellung von Konditionalität stellt die Verwendung eines "Flex-Switch", die ebenfalls nicht dargestellt wird.

Figur 13) zeigt schematisch, wie ein Konstrukt, das eine NMD-Kassette in inaktiver Orientierung mit flankierenden zwei Paaren Erkennungssequenzen (20, 21) bzw. (25, 26), die jeweils einmal zur irreversiblen Inversion geeignet sind, und ein damit gekoppeltes Resistenzmarkergen (13), das von zur Deletion geeigneten Erkennungssequenzen für eine Rekombinase (14) flankiert ist, in das Intron eines Zielgens inseriert wird. Unter 13.2) ist die genomische Organisation des Zielgens nach erfolgter homologer Rekombination des Konstruktes gezeigt. Nach Deletion des Resistenzmarkergens (13) mittels der Rekombinase , gezeigt unter 3a), führt die unter 3b) dargestellte Prozessierung des Primärtranskriptes zu einem gegenüber der 1b) dargestellten Wildtyp-mRNA unverändertem reifen Transkript, da die inserierten Sequenzen aufgrund ihrer Orientierung nicht vom Spleißapparat erkannt werden und als Anteile des natürlichen Introns entfernt werden.

In Anwesenheit der für die Erkennungssequenzen (25,26) spezifischen Rekombinase 2, die die NMD-Kassette und die Erkennungssequenzen (20,21) für die Rekombinase 3 flankieren, wird die NMD-Kassette zusammen mit den sie flankierenden Erkennungssequenzen (20,21) invertiert. Dadurch werden die funktionalen Elemente der NMD-Kassette in den kodierenden Strang angeordnet (Figur 13.4a), und das prozessierte Transkript beinhaltet das NMD vermittelnde Exon (4). Das in Exon (4) enthaltene prämature Stopkodon wird bei der Prozessierung des Transkripts erkannt, so dass es der Degradation unterliegt.

Durch Aktivität der Rekombinase 3 für das zweite Paar der die NMD-Kassette flankierenden Erkennungssequenzen (20, 21) wird die NMD-Kassette erneut invertiert, was zur Anordnung ihrer funktionalen Elemente im nicht-kodierenden Strang führt (Figur 13.5a). In der Folge wird das Gen reaktiviert und exprimiert seine normale, natürliche reife mRNA.

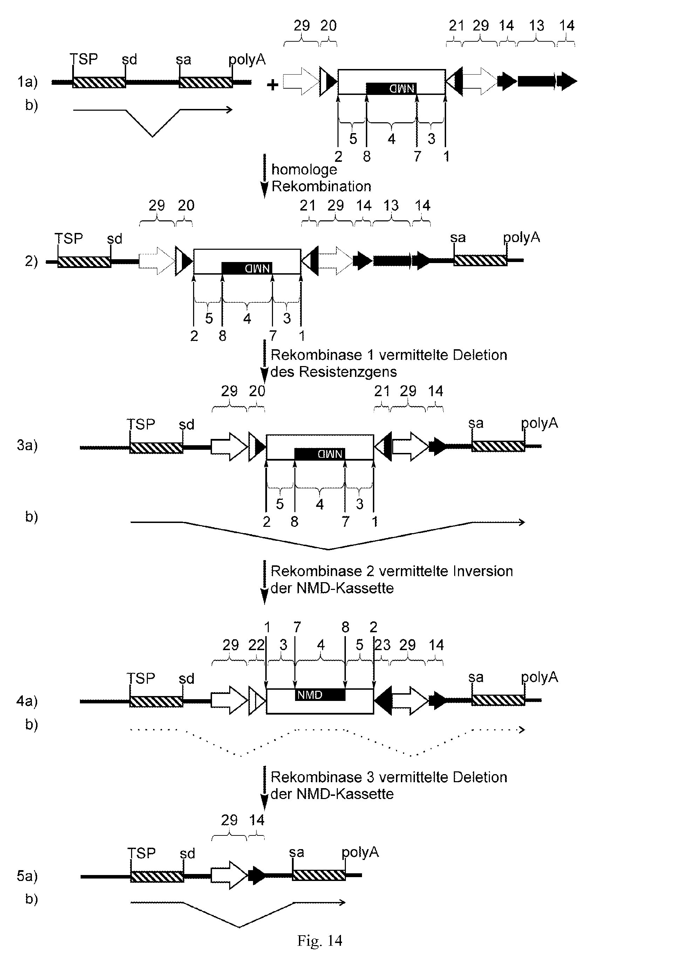

Figur 14 stellt schematisch die Verwendung der erfindungsgemäßen NMD-Kassette in Fortbildung der Erfindung dar, bei der zusätzlich zu den zur Invertierung vorgesehenen flankierenden Erkennungssequenzen (20, 21) ein weiteres Paar flankierender Erkennungssequenzen für eine andere Rekombinase (29) enthalten ist, was aufgrund deren gleicher Orientierung bei Anwesenheit der Rekombinase zur Deletion der NMD-Kassette führt.

Nach Insertion der Kassette in das Zielgen (Figur 14.2) und anschließender durch die Rekombinase 1 vermittelte Deletion des von für die Rekombinase 1 spezifischen Erkennungssequenzen (14) flankierten Resistenzgens (13) ist die inserierte NMD-Kassette zunächst in ihrer inaktiven Orientierung in dem Intron des Zielgens angeordnet (Figur 14.3a)), so dass das primäre Transkript durch Spleißen zu einer im Vergleich mit der Wildtyp-mRNA unveränderten mRNA prozessiert wird.

Die Anwesenheit der Rekombinase 2, die für die für die Inversion vorgesehenen unterschiedlich orientierten Erkennungssequenzen (20, 21) spezifisch ist, führt zur Inversion der NMD-Kassette, damit zu deren Anordnung im kodierenden Strang des Zielgens (Figur 14.4a)) und zur NMD des Transkripts (Figur 14.4b)).

Die Anwesenheit der Rekombinase 3, die für die gleich orientierten Erkennungssequenzen (29) spezifisch ist, führt zur Deletion der NMD-Kassette (Figur 14.5a)) und damit zur Reaktivierung des Gens, das dann wieder eine im Vergleich zur Wildtyp-mRNA unveränderte mRNA exprimiert (Figur 14.5b)).

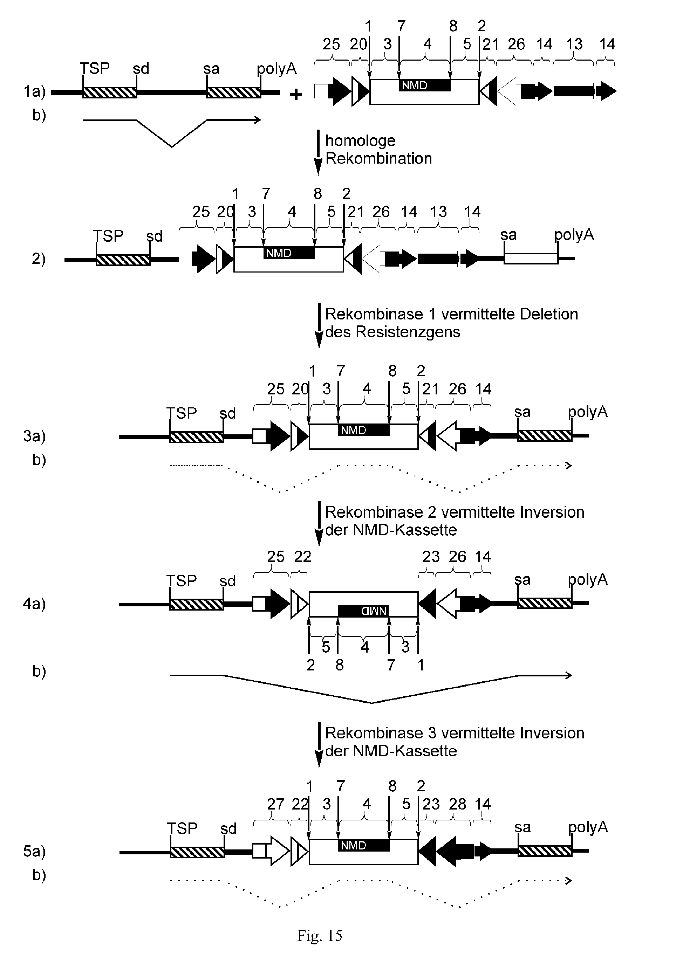

Figur 15 zeigt eine mehrfach konditionale Ausführungsform der Erfindung, bei der im Unterschied zu den in Figur 13) und 14) dargestellten initial konditional inaktivierbaren Genen ein Gen nach der Insertion des Konstruktes zunächst inaktiviert vorliegt und anschließend reaktiviert sowie im Anschluß daran gegebenenfalls erneut inaktiviert werden kann. Dies wird dadurch erreicht, dass die Insertion der NMD-Kassette in das Intron des Zielgens so erfolgt, dass sie in ihrer aktiven Orientierung angeordnet ist (Figur 15.2)) D.h., dass sich die funktionalen Elemente der NMD-Kassette im kodogenen Strang des Gens befinden. Auch hier wird zunächst das Resistenzgen (13) durch Anwesenheit der für seine flankierenden Erkennungssequenzen (14) spezifischen Rekombinase 1 deletiert. Nach der Deletion des Resistenzgens (Figur 15.3a) ist das Gen aufgrund der Anwesenheit der funktionalen Elemente der NMD-Kassette im seinem kodogenen Strang inaktiviert und exprimiert keine reife mRNA mehr (Figur 15.3b))

In Anwesenheit der Rekombinase 2, die spezifisch für die Erkennungssequenzen (20, 21) ist, wird die NMD-Kassette in ihre inaktive Orientierung invertiert, so dass sich die funktionellen Elemente der NMD-Kassette nicht mehr im kodogenen Strang des Gens befinden (Figur 15.4a)) und Spleißen nur zwischen den beiden endogenen Exons des Zielgens erfolgt (Figur 15.4b), was zu einem im Vergleich zur Wildtyp-mRNA unveränderten reifen Transkript führt.

Durch neuerliche Inversion, vermittelt durch die Wirkung der Rekombinase 3 auf das zweite Paar flankierender Erkennungssequenzen (25, 26), wird die NMD-Kassette im Zielgen erneut invertiert, so dass die funktionalen Elemente der NMD-Kassette wieder in den kodogenen Strang des Zielgens gelangen (Figur 15.5a) und dieses inaktivieren, indem das prämature Stopkodon des Exons (4) in der RNA erkannt wird und das Transkript der NMD zugeführt wird.

Neben dem Einsatz als Teil von Nukleinsäurekonstrukten zur gezielten Mutagenese mittels homologer Rekombination sieht die Erfindung die Verwendung von NMD-Kassetten in als Genfallen wirkenden Nukleinsäurekonstrukten vor. Dies bietet den Vorteil, dass auch Gene in Zielzellen mit NMD-Kassetten mutiert werden können, die keine oder eine unzureichende Fähigkeit zur homologen Rekombination aufweisen. Außerdem bieten Ausführungsformen erfindungsgemäßer NMD-Kassetten als Genfallen den Vorteil, dass damit auch unbekannte Gene mutiert werden können.

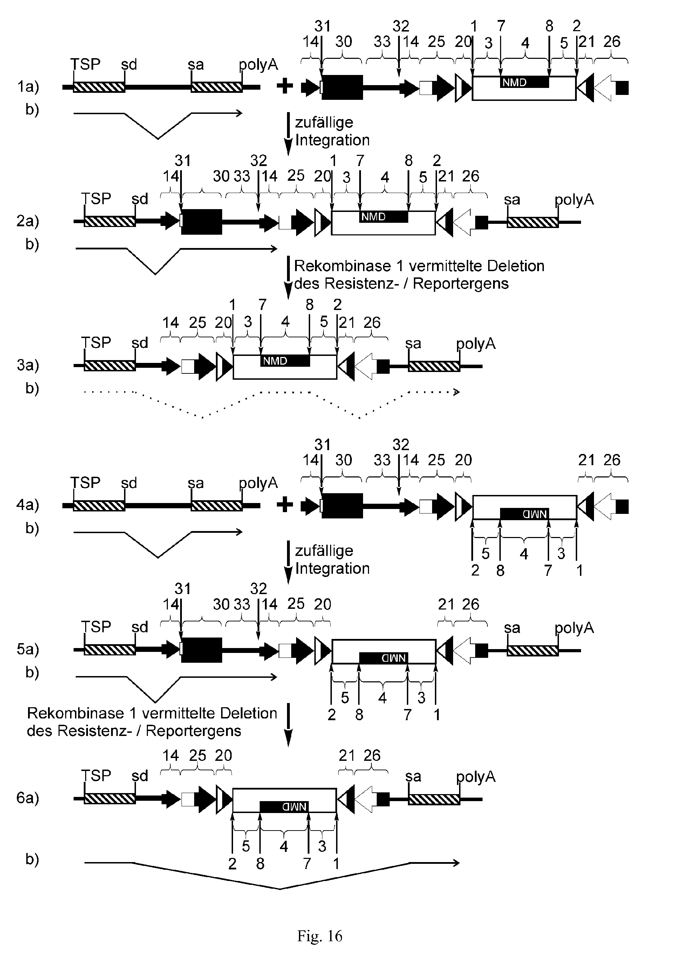

Für Genfallen sieht die Erfindung zwei Ausführungsformen von Genfallen vor: Zum einen sind Nukleinsäurekonstrukte vorgesehen, die, wie in Figur 16) gezeigt, neben der NMD-Kassette ein unvollständiges Selektionsmarker- und/oder Reportergen besitzen, dessen Funktionalität erst nach Insertion in ein Gen hergestellt wird, wenn Anteile des getroffenen Gens das Selektionsmarker- und/oder Reportergen komplettieren. Z.B. erfolgt in der bevorzugten Variante dieser Ausführungsform als Exonfalle die Expression eines in Form eines 3'-terminalen Exons ausgelegten Selektionsmarker- und/oder Reportergen, wenn die Genfalle stromabwärts einer Spleißdonorstelle eines Exons inseriert wurde. Entsprechend gilt für Promotor und polyA-Fallen, die hier nicht dargestellt werden. In der Kombination mit einem als Genfalle wirkenden Selektionsmarker- und/oder Reportergen sieht die Erfindung die NMD-Kassette zum einen dafür vor, hypomorphe Mutationen eines Gens, das durch das Selektionsmarker- und/oder Reportergen nicht komplett inaktiviert wurde, durch Deletion des Selektionsmarker- und/oder Reportergen komplett zu inaktivieren, indem die NMD-Kassette in eine wirksame Position gebracht wird. Zum anderen ist die NMD-Kassette dafür vorgesehen durch ein Selektionsmarker- und/oder Reportergen vollständig oder teilweise inaktiviertes Gen in eine mehrfach konditionale Form zu überführen. Nachteilig bei dieser Ausführungsform ist, dass im Fall der bevorzugten Variante als Exonfalle, drei unabhängige Konstrukte benötigt werden, die das Selektionsmarker- und/oder Reportergen in jeweils einer der drei möglichen Intronphasen beinhalten. Gegenüber der anderen Ausführungsform besteht aber der Vorteil, dass ein einzelnes Konstrukt zunächst zur Herstellung von Reporterallelen genutzt werden kann, mit dem das Expressionsmuster eines von der Genfalle getroffenen Gens ermittelt werden kann, und dass das Allel anschließend in ein mehrfach konditionales umgewandelt werden kann. Dieser Ausführungsform entsprechende Nukleinsäurekonstrukte sind neben der Verwendung als Genfallen auch für das sogenannte "targeted trapping" vorgesehen, bei dem ein ursprünglich als Genfalle vorgesehenes Nukleinsäurekonstrukt mit Sequenzen flankiert wird, die homolog zu Bereichen eines Gens im Genoms eines Zielorganismus sind, in das die Kassette mittels homologer Rekombination eingebracht werden soll. Da die genomische Organisation eines Locus, der mittels "targeted trapping" mit einem Nukleinsäurekonstrukt dieser Ausführungsform mutiert wurde, im Ergebnis der Organisation eines Lokus entspricht, in den eine entsprechende Genfalle inseriert wurde, wird das "targeted trapping" hier nicht extra dargestellt.

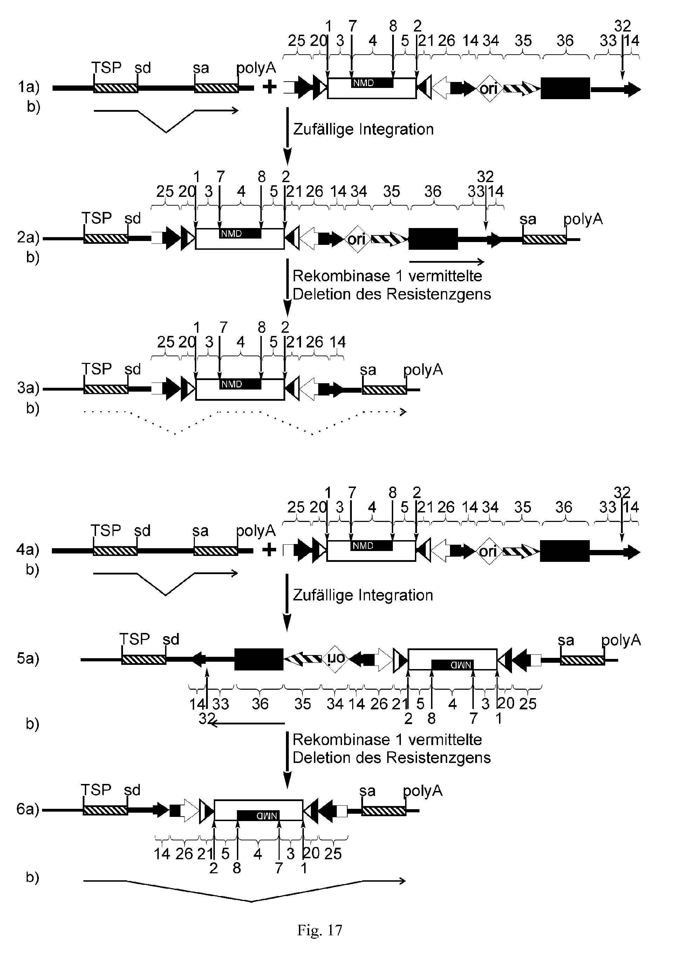

Für die in Figur 17) dargestellte zweite Ausführungsform ist vorgesehen, dass eine erfindungsgemäße NMD-Kassette selbst als Genfalle wirkt. Da in diesem Falle keine endogenen Funktionen des Zielgens zur Expression des Selektionsmarkergens genutzt werden können, sieht die Erfindung eine Verbindung der NMD-Kassette mit einer vollständigen Expressionskassette für ein Selektionsmarkergen vor, das bevorzugt in Eukaryonten und Prokaryonten selektierbar ist.

Die unabhängige Expression des Selektionsmarkergens bietet den Vorteil, dass damit auch Gene getroffen werden können, die in den Zellen inaktiv sind, in denen die Insertion stattfindet. Da die NMD-Kassette außerdem unabhängig von der Intronphase wirkt, sind alle Zellen mit Allelen, in denen eine NMD-Kassette in der bevorzugten Ausführungsform mit drei Stopkodons in jedem Leserahmen in ein Intron eines Gens inseriert wurde, nutzbar. Nachteilig bei dieser Ausführungsform ist, dass auch Zellen selektiert werden, in denen eine NMD-Kassette in intergenische Bereiche inseriert wurde. Da aber 20% des Genoms von Säugetieren aus Introns bestehen und die NMD-Kassette mittels Rekombinasen in ihre aktive Orientierung überführt werden kann, ist jede fünfte Zelle bei dieser Ausführungsform für die Herstellung von konditional inaktivierbaren und konditional reaktivierbaren Allelen nutzbar.

In den Figuren 16) und 17) ist jeweils nur die bevorzugte Ausführung einer zweifach invertierbaren NMD-Kassette gezeigt. Alternative Ausführungsformen, die die NMD-Kassette in einfach invertierbarer Form oder in sukzessiv invertierbarer und deletierbarer Ausbildung enthält sind nicht dargestellt. Außerdem ist in den beiden Figuren jeweils nur die Verwendung eines Konstrukts als Genfalle bis zur Deletion des Reporter- bzw. Selektionsmarkergens dargestellt, da sich die Wirkung der zweifach invertierbaren NMD-Kassetten und ihre weitere Verwendung für konditionale Zwecke nicht von den in den Figuren 13) und 15) durch homologe Rekombination in ein Intron eines Gens inserierten NMD-Kassetten unterscheidet.

Bei allen Ausführungsformen als Genfalle sieht die Erfindung die Flankierung der als Genfalle wirkenden Konstrukte mit zusätzlichen Sequenzen natürlichen oder synthetischen Ursprungs vor, die keine Spleißsignale oder andere regulatorische Sequenzen wie z.B. Promotoren oder polyA-Signale, enthalten. Diese Sequenzen sind dazu vorgesehen, die eigentliche Genfalle vor dem Verlust von Elementen zu schützen, da bei der zufälligen Insertion in die genomische DNA randständige Sequenzen häufig verloren gehen. Da diese Schutzsequenzen hinsichtlich der Inaktivierung eines Zielgens unerheblich sind, werden sie in den Figuren 16) und 17) nicht dargestellt.

Die Erfindung sieht vor, als Genfallen ausgeführte erfindungsgemäße Nukleinsäurekonstrukte mittels Transfektion in Zielzellen einzubringen. Dies sind bevorzugt murine ES-Zellen, in die die erfindungsgemäßen Nukleinsäurekonstrukte bevorzugt mittels Elektroporation transfiziert werden. Zielzellen können aber auch Zellen anderer Organismen sein, aus denen sich nicht-menschliche vollständige Organismen herstellen lassen.

Figur 16 zeigt eine Ausführungsform der Erfindung, in der die NMD-Kassette als Bestandteil eines Konstrukts verwendet wird, das als Genfalle eingesetzt wird. Zur Verwendung in einem Konstrukt als Genfalle ist die NMD-Kassette mit einem Reportergen und/oder Selektionsmarkergen (30) gekoppelt. Da in der bevorzugten Variante dieser Ausführungsform ein fusioniertes ß-Galaktosidase-/Neomycinresistenzgen vorgesehen ist, wird dies im folgenden als Reporter-/Selektionsgen bezeichnet. Das Reporter-/Selektionsgen ist vorzugsweise mit Erkennungssequenzen (14) für eine Rekombinase flankiert, die zur Deletion des Reporter-/Selektionsgens (30) eingesetzt werden können. Dabei bietet die erfindungsgemäße NMD-Kassette den Vorteil, dass sie gezielt in ihrer aktiven (Figur 16.1a)) oder inaktiven (Figur 16.4a)) Orientierung mit dem Reporter-/Selektionsgen (30) gekoppelt werden kann, und entsprechend nach der Insertion in ein zufällig getroffenes Gen, im Rahmen dieser Erfindung dennoch als Zielgen bezeichnet, von diesem abhängt. Durch die Deletion des Reporter-/Selektionsgens (30) durch die Rekombinase 1 wird das Zielgen jeweils in eine Form überführt, deren Aktivitätszustand sich zweifach konditional ändern läßt ((Figur 3a) und Figur 6a)).

Dabei ist bevorzugt, daß das Reporter-/Selektionsgen (30) als 3'-terminales Exon ausgeführt ist. Dazu weist es am 5'-Ende die für die Funktion eines Spleißakzeptors notwendigen Elemente auf. Dies sind in 5'→3' Richtung mindestens ein Verzweigungspunkt, ein Polypyrimidinabschnitt sowie einen Spleißakzeptorstelle. Zur Vereinfachung sind diese Elemente als (31) zusammengefaßt.

Am 3'-Ende stromabwärts des Stopkodons des Reporter-/Selektionsgens (30) ist eine für die Funktion als 3'-terminales Exon notwendige 3'-UTR (33) lokalisiert, die am 3'-Ende von einem polyA-Signal (32) begrenzt wird. In dieser bevorzugten Ausführungsform ist das Reporter-/Selektionsgen dazu vorgesehen nach dem Spleißen mit einem stromaufwärts lokalisierten Exon an das von den stromaufwärts der Genfalle lokalisierten Exons kodierte Peptid C-terminal fusioniert exprimiert zu werden. Deshalb sieht die Erfindung für diese Ausführungsform drei unabhängige Nukleinsäurekonstrukte vor, bei denen sich das Reporter-/Selektionsgen jeweils in einer anderen Intronphase befindet

Nach Insertion der als Genfalle eingesetzten Nukleinsäurekonstrukte in ein Gen kommt es für die Transformanten zur funktionalen Komplementierung des im Reporter-/Selektionsgen (30) fehlenden Elements, im dargestellten Fall zur Translation eines Fusionsproteins, das von einer mRNA kodiert wird, die aus den stromaufwärts der inserierten Genfalle lokalisierten Exons des Zielgens und dem das Reporter-/Selektionsgen (30) beinhaltenden 3'terminalen Exon nach korrektem Spleißen entsteht. Die entsprechenden Transkripte sind in Figur 16.2b) und 16.5b) dargestellt. In der Folge können derartige Fusionsproteine zur Selektion von Transformanten benutzt werden. Dabei können derartige Transformanten auf Grund der Aktivität des Reporter-/Selektionsgens (30) von solchen unterschieden werden, in denen das Konstrukt nicht im passenden Leserahmen integriert ist und folglich z.B. kein Fusionsprotein translatiert wird, sowie von Transformanten, in denen das Konstrukt in ein inaktives Gen, in der falschen Orientierung, in nicht transkribierte Bereiche eines anderweitig aktiven Gens oder in intergenische Bereiche integriert ist. Die Erfindung sieht außerdem vor, dass die das zur Selektion genutzte Fusionsprotein kodierende mRNA mittels geeigneter, dem Fachmann bekannter PCR-Methoden zur Identifikation des Insertionsorts benutzt wird.

Falls die Synthese des als Reporter und zur Selektion genutzten Fusionsproteins zur Unterbrechung der Wirkung des natürlichen Translationsprodukts führt, kann bereits die Insertion des Konstrukts zur Inaktivierung des betroffenen Allels führen. In Fällen, in denen dies nicht der Fall ist, also z.B. bei hypomorphen Mutanten, sieht die Erfindung vor, eine komplette Inaktivierung solcher Zielgene mittels der NMD-Kassette zu ermöglichen.

Dazu ist es erfindungsgemäß bevorzugt, die Abschnitte des als Genfalle eingesetzten Konstrukts, die für die Expression des Reporter-/Selektionsgen (30) notwendig sind, nach der Insertion durch Anwesenheit der für die diesen Bereich flankierenden Erkennungssequenzen (14) spezifischen Rekombinase 1 zu deletieren. Zusammen mit den kodierenden Sequenzen des Reporter-/Selektionsgens (30) werden die 5' davon lokalisierten Elemente die zum Spleißen des Exons notwendig sind (31), und die in 3' von den kodierenden Sequenzen lokalisierte 3'-UTR (33) sowie das polyA-Signal (32) deletiert.

In der Folge sind abhängig von der Orientierung der NMD-Kassette relativ zu dem Reporter-/Selektionsgen Zellen erhältlich, die eine in dieser Ausführungsform zweifach invertierbare NMD-Kassette in ihrer aktiven (Figur 16.3a)) oder inaktiven (Figur 16.6a)) Form in ein Gen integriert haben, das durch das Reporter-/Selektionsgen (30) identifiziert wurden. Im Fall, dass das NMD vermittelnde Exon (4) im gleichen Strang der DNA des Konstruktes wie das Reporter-/Selektionsgen (30) lokalisiert ist (Figur 16.1), führt die Deletion des Reporter-/Selektionsgens im Zielgen dazu, dass das Zielgen inaktiviert bleibt, bzw. im Fall hypomorpher Mutanten mit Restaktivität des Zielgens, vollständig inaktiviert wird, da seine Transkripte der NMD unterliegen (Figur 16.3b).

Im Fall, dass das NMD vermittelnde Exon (4) nicht im gleichen Strang der DNA des Konstruktes wie das Reporter-/Selektionsgen (30) lokalisiert ist (Figur 16.1), führt die Deletion des Reporter-/Selektionsgens im Zielgen dazu, dass das Zielgen reaktiviert wird, bzw., im Fall hypomorpher Mutanten, wieder seine volle Aktivität erreicht, wobei jeweils eine mature mRNA exprimiert wird, die nicht von der des Wildtypgens zu unterscheiden ist (Figur 16.6b).

Im Ergebnis entsprechen Gene, die mittels einer Genfalle der beschriebenen Ausführungsform mutiert wurden, nach der Deletion des Reporter-/Selektionsmarkergens Genen, in die eine doppelt invertierbare NMD-Kassette mittels homologer Rekombination eingeführt wurden (Figur 13) und Figur 15)). Da die weiteren möglichen Verwendungen von doppelt invertierbaren NMD-Kassetten anhand der Figuren 13) und 15) erläutert wurde, wird dies hier nicht gezeigt.

In den in Figur 17.1) und 17.4) dargestellten Varianten der bevorzugten Ausführungsform von erfindungsgemäßen NMD-Kassetten als Bestandteil von Genfallen wirkt die NMD-Kassette direkt selbst als inaktivierendes Element, d.h. unabhängig von unvollständigen Reporter /Selektionsgenen, wie dies bei der anderen Ausführungsform von Genfallen vorgesehen ist. Statt dessen kommt ein Selektionsmarkergen zur Anwendung, dass alle für seine Expression notwendigen regulatorischen Elemente beinhaltet. Bevorzugt sieht die Erfindung dafür Selektionsmarkergene vor, mit denen sowohl in Eukaryonten als auch in Prokaryonten selektiert werden kann, z.B. das Neomycin/Kanamycin-Resistenzgen. Deshalb ist vorgesehen, die kodierende Sequenz des Resistenzgens (36) mit einer bifunktionalen Promoterkassette (35) in 5' zu verbinden, die aus einer Fusion eines eukaryontischen und eines prokaryontischen Promotoren besteht. Am 3'-Ende schließen ein prokaryontisches Terminationssignal (nicht dargestellt) sowie eine eukaryontische 3'-UTR (33) an das Resistenzgen an, das in 3' von einem eukaryontischen polyA-Signal (32) begrenzt wird.

Es ist außerdem bevorzugt in dieser Ausführungsform das bifunktionale Resistenzgen mit einem prokaryontischen Replikationsursprung (34) zu koppeln. Dies bietet den Vorteil, dass ein genomisches Fragment, dass eine Genfalle dieser Ausführungsform beinhaltet, mittels "Plasmid Rescue" in Bakterien kloniert werden und der Insertionspunkt mittels Sequenzierung des Plasmids identifiziert werden kann. Daneben sieht die Erfindung vor, auf diese Art isolierte genomische Fragmente, die eine Genfalle im Intron eines Gens enthalten zur Erzeugung weiterer Mutanten mit einem anderen genetischen Hintergrund zu nutzen. Dazu wird ein in Bakterien vermehrtes, eine Genfalle in einem Intron eines Gens enthaltendes genomisches Fragment wieder linearisiert und homolog in den gleichen Lokus einer anderen Zielzelle rekombiniert. Dies bietet den erfindungsgemäßen Vorteil, dass die Mutation in einen anderen genetischen Hintergrund eingeführt werden kann, z.B. in einen genetisch unterschiedlichen Mausstamm, ohne auf die zeitaufwendigen, normalerweise dafür notwendigen Rückkreuzungen angewiesen zu sein. Außerdem kann dieses Verfahren dazu benutzt werden, die Mutation aus Zellen, die die Fähigkeit zur Keimbahn beizutragen verloren haben, z.B. ein differenzierter ES-Zell-Klon, auf Zellen zu übertragen, die diese Fähigkeit noch besitzen.

Die Figuren 17.1a) und 17.4a) zeigen die bevorzugte Flankierung der Bestandteile des bifunktionalen Resistenzgens (35, 36 33, 32) und des damit gekoppelten Replikationsursprungs (34) mit gleich orientierten Erkennungssequenzen für eine erste Rekombinase, die zur Deletion des Resistenzgens und des Replikationsursprungs vorgesehen sind.

Für die in den Figuren 17.1a) und 17.4a) dargestellte bevorzugte Ausführungsform von Genfallen werden invertierbare NMD-Kassetten bevorzugt, die mit dem bifunktionalen Resistenzgen gekoppelt sind. Weiterhin ist dabei die Ausführung der NMD-Kassette mit drei Stopkodons im NMD vermittelnden Exon (4), jeweils eins in jedem der drei möglichen Leserahmen und jedes mindestens 50 bp von der Spleißdonorstelle des Exons (4) beabstandet, bevorzugt, da diese Ausführungsform zur Inaktivierung aller Gene mit mindestens einem Intron geeignet ist.

In der dargestellten Variante ist das NMD vermittelnde Exon (4) im gleichen Strang lokalisiert wie die kodierenden Sequenzen des Resistenzgens (36). Alternativ können die beiden Elemente auch in unterschiedlichen Strängen lokalisiert sein (nicht dargestellt). Um die Invertierung der NMD-Kassette zu ermöglichen, wird sie von Erkennungssequenzen für Rekombinasen flankiert. Dabei ist die in den Figuren 17.1a) und 17.4a) gezeigte Flankierung mit zwei Paaren von Erkennungssequenzen (20 und 21 sowie 25 und 26), die jeweils nur eine irreversible Invertierung erlauben, bevorzugt. Andere Ausführungsformen der Flankierung mit Erkennungssequenzen für Rekombinase, z.B. für nur eine Invertierung oder für eine Invertierung und eine Deletion, sind nicht dargestellt.

Figur 17.2a) und 17.5a) zeigen die genomische Organisation von Zielgenen nach Insertion von Genfallen der bevorzugten Ausführungsform. Die genomische Organisation ist dabei abhängig von der Orientierung, in der die Genfalle in das Zielgen inseriert wurde. Da die Expression des Resistenzgens, angedeutet durch die Pfeile unter (Figur 17.2b)) bzw. über (Figur 17.5b)) der in Figur 17.2a) und dargestellten kodierenden Sequenz des Resistenzgens (36) und seiner 3'-UTR (33), unabhängig davon ist, ob die Genfalle in einem Intron eines Gens inseriert wurde, müssen zunächst Klone identifiziert werden, in denen die Insertion nicht in intergenischen Regionen oder in Exons erfolgte. Da das Säugergenom zu 20% aus Introns besteht, wird aber jeder fünfte Klon eine Genfalle des in Figur 17) dargestellten Typs in einem Intron aufweisen, die mittels dem Fachmann bekannter PCR-Methoden oder mittels des oben beschriebenen "Plasmid Rescue" identifiziert werden können.

Im Fall, dass das NMD vermittelnde Exon (4) nach der Insertion im kodogenen Strang des Zielgens lokalisiert ist (Figur 17.2a)) wirkt es nach Rekombinase vermittelter Deletion des Resistenzgens, deren Ergebnis in Figur 17.3a) dargestellt ist, unmittelbar inaktivierend auf das Zielgen, da eines der in der NMD-Kassette enthaltenen Stopkodons in den Leserahmen des Gens gelangt und die NMD seiner Transkripte auslöst (Figur 17.3b).

Die Figuren 17.5a) bis 17.6b) stellen den Fall dar, in dem dasselbe Nukleinsäurekonstrukt in gegensätzlicher Orientierung in das Zielgen inseriert wurde, wobei sich das in der NMD-Kassette enthaltene NMD vermittelnde Exon (4) nach der Insertion im nicht-kodogenen Strang des Zielgens befindet (Figur 17.4a)). Wegen der inaktiven Orientierung der NMD-Kassette exprimiert das Zielgen reife Transkripte (Figur 17.6b)), die nicht von denen des Wildtypgens zu unterscheiden sind, die in den Figuren 17.1b) und 17.4b) dargestellt sind.

Die bevorzugte zweifache Invertierbarkeit der NMD-Kassette in Genfallen des hier beschriebenen Typs bietet den erfindungsgemäßen Vorteil, dass im Fall, dass sich das NMD vermittelnde Exon (4) nach der Insertion im kodierenden Strang des Zielgens befindet und das Zielgen direkt inaktiviert ist (Figur 17.3a)), das Gen konditional, z.B. nur in einem bestimmten Gewebe und/oder zu einem bestimmten Zeitpunkt, reaktivieren läßt. Außerdem bietet sich der Vorteil, dass das Gen durch Invertierung mittels einer der für die Erkennungsstellen (20 und 21) oder (25 und 26) spezifischen Rekombinase in eine konditional inaktivierbare Form zu überführen, die dazu genutzt werden kann, das Gen konditional, z.B. nur in einem bestimmten Gewebe und/oder zu einem bestimmten Zeitpunkt, zu inaktivieren. Entsprechend kann ein Gen, dass, wie in Figur 17.6a) dargestellt, das NMD vermittelnde Exon (4) der Genfalle im nicht-kodogenen Strang enthält, direkt als konditional inaktivierbares Gen genutzt werden. Durch Invertierung der NMD-Kassette mittels einer der für die Erkennungsstellen (20 und 21) oder (25 und 26) spezifischen Rekombinasen kann das Gen inaktiviert werden, wobei diese Inaktivierung konditional mittels der jeweils anderen Rekombinase wieder aufgehoben werden kann. Die verschiedenen Möglichkeiten, die die mehrfache Inversion einer als Genfalle wirkenden NMD-Kassette bietet, wird hier nicht dargestellt, da die in den Figuren 17.3a) und 17.6a) dargestellten Gene, in die eine als Genfalle wirkende, doppelt invertierbare NMD-Kassette inseriert wurde, Genen entsprechen, in die eine zweifach invertierbare NMD-Kassette in der einen oder anderen Orientierung mittels homologer Rekombination eingeführt wurde, für die die weiteren möglichen Verwendungen von doppelt invertierbaren NMD-Kassetten anhand der Figuren 13) und 15) erläutert wurde.

Für Zellen, die schlecht oder nicht transfizierbar sind, sieht die Erfindung statt der Verwendung von Genfallen das Einbringen erfindungsgemäßer Nukleinsäurekonstrukte mittels retroviralem Gentransfer vor. Entsprechende Nukleinsäurekonstrukte werden genutzt, um in Verpackungszellen eine von dem Konstrukt transkribierte RNA zusammen mit den für die reverse Transkription und für die Insertion in das Genom einer Zelle notwendigen viralen Proteinen in Virushüllproteine zu verpacken. Die so hergestellten Viruspartikel sind dazu vorgesehen, die Zielzellen zu transduzieren. Nachdem die in den Virenpartikeln enthaltene RNA in der Zielzelle freigesetzt wurde, wird sie revers transkribiert und das entstehende DNA-Fragment zufällig in das Genom der Zielzelle inseriert.

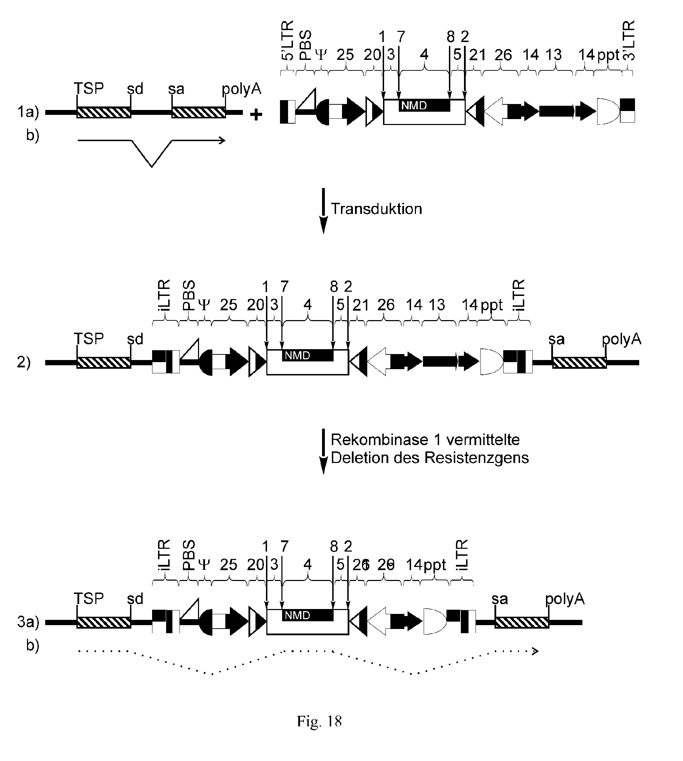

Für den retroviralen Gentransfer ist, wie in Figur 18.1a) dargestellt, vorgesehen, eine bevorzugt zweifach invertierbare NMD-Kassette, die mit einem von Erkennungssequenzen für eine Rekombinase (14) flankierten Resistenzgen (13) gekoppelt ist, mindestens in 5' mit einem retroviralen Verpackungssignal (Ψ) eines Retrovirus, einer Primerbindestelle (PBS, "primer binding site") für das Hybridisieren einer als Primer für die reverse Transkriptase dienenden tRNA, sowie einen 5' "long terminal repeat" (5'LTR) zu verbinden. In 3' wird das Konstrukt mindestens von einem Polypurinabschnitt (PPT) und einem 3' "long terminal repeat" (3'LTR) begrenzt. Dabei ist die Ausführung als selbstinaktivierende retrovirale Vektoren bevorzugt, bei denen die "long terminal repeats" im Zuge der reversen Transkription in nicht funktionale Formen (iLTR) umgewandelt werden, so dass sie nach der Insertion in ein Zielgen (Figur 18.2)) von einer reversen Transkriptase nicht mehr genutzt werden können und eine Mobilisierung mit Insertion in einem anderen Locus ausgeschlossen werden kann.

Nach Selektion für Transformanten, die das retrovirale NMD-Konstrukt in ihr Genom inseriert haben, kann mittels geeigneter PCR-Methoden der Insertionspunkt bestimmt werden. Dabei gilt wie für die in Figur 17) beschriebenen Genfallen, dass aufgrund des 20%igen Anteils der Introns am Genom in jeder fünften Zelle ein Intron eines Gens getroffen wird.

Nach Deletion des Resistenzgens verbleiben neben der NMD-Kassette und den sie flankierenden Erkennungssequenzen für Rekombinasen (14, 20, 21, 25 und 26) Anteile des Vektors im Lokus erhalten (iLTRs, PBS, Ψ und ppt). Im Fall, das sich das NMD vermittelnde Exon (4), wie in Figur 18.3a) dargestellt, nach der Insertion des retroviralen Konstrukts im kodogenen Strang des Zielgens befindet, ist dieses direkt inaktiviert, da seine Transkripte der NMD zugeführt werden (Figur 18.3b)).

Abgesehen von den retroviralen Sequenzen, die im Lokus verbleiben, entspricht die genomische Organisation des Lokus der in Figur 17.3a) für die Insertion von Genfallen. Die umgekehrte Orientierung retroviraler Konstrukte in einem Zielgen entspricht demgemäß der in Figur 17.6a) für Genfallen dargestellten Situation, d.h. das Gen ist nicht inaktiviert, und wird hier nicht gezeigt.

Mittels einer der beiden Rekombinasen, die für die Erkennungssequenzen (20) und (21) bzw. (25) und (26) spezifisch sind, kann der Aktivitätszustand eines Gens, in das ein erfindungsgemäßes retrovirales NMD-Konstrukt inseriert wurde, konditional verändert werden. Die in der bevorzugten Ausführungsform vorgesehene zweifache Inversion einer NMD-Kassette ist in den Figuren 13) und 15) für beide Orientierungen beschrieben und wird hier deshalb nicht gezeigt.

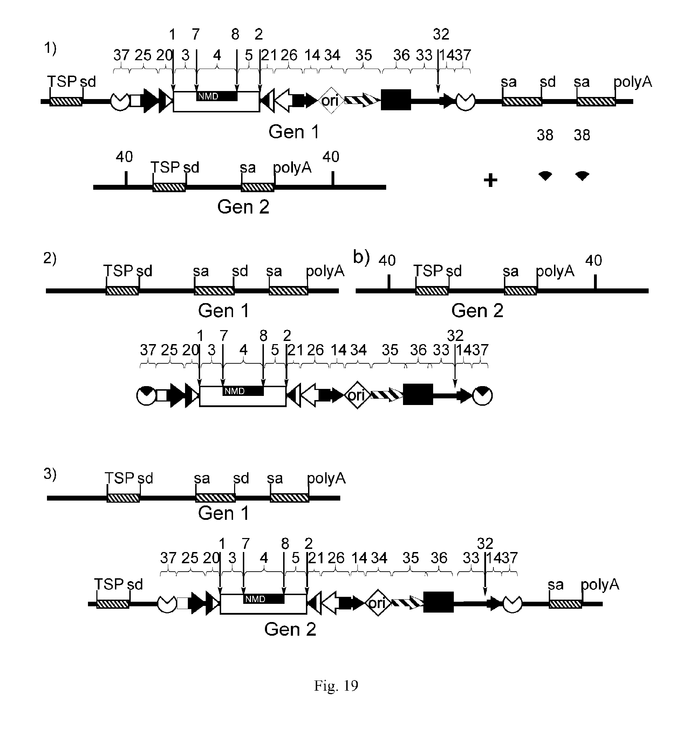

Als weitere Möglichkeit erfindungsgemäße Nukleinsäurekonstrukte durch zufällige Insertion in Zielgene einzubringen sieht die Erfindung die Verwendung von NMD-Kassetten in Transposons vor. Geeignet dafür sind alle Ausführungsformen der NMD-Kassette, bevorzugt solche in Verbindung mit einem Selektionsmarker und/oder Reportergen, wie sie für Genfallen in den Figuren 16) und 17) dargestellt sind. Bei den in Figur 19) dargestellten als Transposons vorgesehenen erfindungsgemäßen Nukleinsäurekonstrukten für Anwendungen in eukaryontischen Zellen oder Organismen grenzen statt der bei Genfallen verwendeten flankierenden Sequenzen Erkennungssequenzen für Transposasen (37) die NMD-Kassette und die mit ihr verbundenen Erkennungssequenzen für Rekombinasen sowie das angrenzende Resistenzgen. Dabei sind die Erkennungssequenzen für die Transposasen "sleeping beauty" und "frog prince" bevorzugt. Abhängig davon, wie ein entsprechendes Nukleinsäurekonstrukt zunächst in das Genom einer Zielzelle inseriert werden soll, kann das Konstrukt außerhalb der Transposaseerkennungssequenzen (37) von weiteren Sequenzen flankiert werden (nicht dargestellt), z.B. mit zu einem Zielgen homologen Bereichen, wenn die Insertion gezielt erfolgen soll, oder bei zufälliger Insertion in Form einer Genfalle mit Sequenzen, die lediglich dem Schutz der funktional wichtigen Elemente dienen, da bei der zufälligen Insertion in die genomische DNA randständige Sequenzen verloren gehen können.

Unabhängig von der Art der initialen Insertion, sieht die Erfindung vor als eukaryontische Transposons ausgeführten Nukleinsäurekonstrukte *in vivo* durch Expression der spezifischen Transposase (38) zu mobilisieren und von einem Lokus (Gen 1) in einen anderen (Gen 2) zu überführen (Figur 19.1)), indem die dazu benötigte Transposase zur Anwesenheit gebracht wird, in Zellen z.B. durch Transfektion eines Transposase-Expressionsplasmid, in Mäusen z.B. durch Verpaaren mit einer Mauslinie, die die Transposase in der Keimbahn exprimiert. Die Anwesenheit der Transposase führt zur Excission des gesamten NMD-Konstrukts (Figur 19.2), das in der Folge in einen anderen Lokus (Gen 2) inseriert wird (Figur 19.3). Zellen oder Tiere, in denen die NMD durch die Transposase in eine anderes Gen inseriert wurde, können durch erneute Expression der Transposase als Substrat für die Überführung der NMD-Kassette in weitere Gene dienen.

Nach Rekombinase vermittelter Deletion des Resistenzmarkers (nicht gezeigt) verbleibt in einem Gen, in das eine NMD-Kassette durch eine Transposase inseriert wurde, nur die NMD-Kassette sowie die sie flankierenden Erkennungssequenzen für Rekombinasen und für die Transposase. Abhängig von der resultierenden Orientierung der NMD-Kassette (die zweite mögliche Orientierung ist nicht dargestellt) nach der Insertion entspricht dies bei Verwendung einer zweifach invertierbaren NMD-Kassette den in den Figuren 13) und 15) für homolog in einen Lokus rekombinierten NMD-Kassetten sowie den Figuren 16) und 17) für in Form von Genfallen in einen Gen inserierten NMD-Kassetten. Die Folgen der Anwesenheit der für die Erkennungssequenzen (20) und (21) sowie für die Erkennungssequenzen (25) und (26) sind in den Figuren 13) und 15) gezeigt und werden deshalb hier nicht dargestellt.

Bei Verwendung von erfindungsgemäßer Transposons, die wie in Figur 19) dargestellt, ein Resistenzgen (36) mit bifunktionalem Promotor (35) sowie pro- und eukaryontischen Transkriptionsterminationssignalen (32,33) und eine prokaryontischen Replikationsursprung aufweisen, kann der Insertionspunkt mittels "plasmid rescue" erfolgen. Alternativ können geeignete PCR-Methoden dafür angewendet werden.

Im Fall, dass ein erfindungsgemäßes Transposon zusätzlich zu einer NMD-Kassette ein Reporter-/Selektionsgen beinhaltet, wie für Genfallen in Figur 16) beschrieben, kann eine Identifizierung von Zellen in denen ein Transposon in ein aktives Gen inseriert wurde, zunächst anhand eines Nachweises von Reporter-/Selektionsgenexpression erfolgen. Nachteilig dabei ist, dass die Insertion in der richtigen Intronphase erfolgen muß, weshalb die Erfindung für diese Ausführungsform von NMD-Kassetten beinhaltenden Transposons je ein Konstrukt mit einem Reporter-/Selektionsgen in einer der drei möglichen Intronphasen vorsieht.

Da die Wirkung erfindungsgemäßer NMD-Kassetten unabhängig von der Intronphase ist, die ein Intron aufweist, in das eine NMD-Kassette inseriert wird, und die NMD-Kassette mit einem deletierbaren Selektionsmarkergen verbunden werden kann, ist ein einzelner Klonierungsschritt für die Herstellung von Vektoren für die Generierung komplett inaktivierter, konditional inaktivier- oder reaktivierbarer Gene sowie Gene mit mehrfach konditional veränderbarer Aktivität ausreichend. Außerdem bieten NMD-Kassetten den Vorteil, dass ihre Wirkung im wesentlichen davon unabhängig ist, in welches Intron eines Gens und in welche Position in einem Intron sie inseriert werden.

Deshalb sieht die Erfindung erfindungsgemäße NMD-Kassetten neben der herkömmlichen Klonierung von Restriktionsfragmenten und dem auf homologer Rekombination in *E. coli* beruhenden ET-Klonieren die Herstellung von für die homologe Rekombination in eukaryontische Zielgene geeignete Vektoren auch die zufällige Transposase vermittelte Insertion von NMD-Kassetten in klonierte genomische Fragmente vor. Die Erfindung sieht außerdem vor, erfindungsgemäße NMD-Kassette zur direkten Subklonierung von für die homologe Rekombination in eukaryontische Zielgene geeigneten Vektoren aus größeren klonierten Fragmenten, z.B. aus in BACs klonierten genomischen Fragmenten, zu nutzen. Weiterhin ist erfindungsgemäß vorgesehen Bibliotheken von für die homologe Rekombination in Eukaryonten geeigneten Vektoren aus Gemischen klonierter oder unklonierter genomischer Fragmente herzustellen. Dabei bietet die NMD-Kassette in der bevorzugten Ausführung, in der sie mehrfach invertiert werden kann, den erfindungsgemäßen Vorteil, dass ihre Orientierung *in vitro,* in Bakterien oder nach der homologen Rekombination in der Zielzelle geändert werden kann, wenn die Fragestellungen dies erfordern.

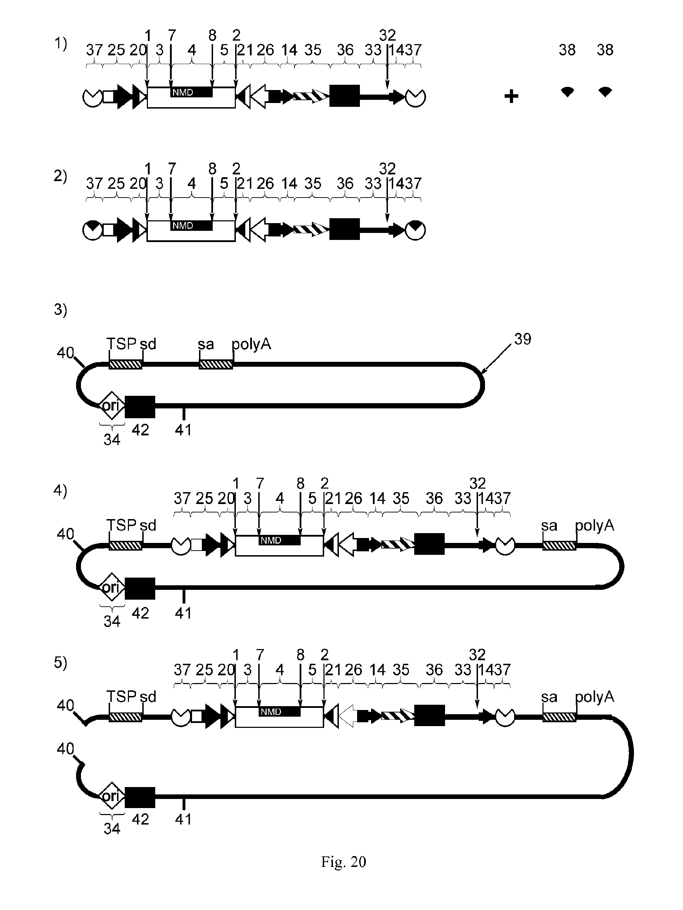

In Figur 20.1) ist ein erfindungsgemäßes Konstrukt zur Herstellung eines Vektors für die gezielte Mutagenese in Eukaryonten dargestellt. Er beinhaltet die minimale NMD-Kassette, die das NMD vermittelnde Exon (4) mit einem erfindungsgemäßen Stopkodon in jedem der drei Leserahmen sowie die flankierenden Intronsequenzen (3) und (5) enthält. Die NMD-Kassette wird flankiert von Erkennungssequenzen (20 und 21 sowie 25 und 26) für zwei unabhängige Rekombinasen (20), die jeweils eine irreversible Invertierung der NMD-Kassette zulassen. Zur Vermittlung von Resistenz in Prokaryonten und in den eukaryontischen Zielzellen ist die NMD-Kassette mit einem bifunktionalen Resistenzgen gekoppelt, das aus einer in Prokaryonten und Eukaryonten aktiven Promotorkassette (35), der kodierenden Sequenz des Resistenzgens (36) sowie einer eukaryontischen 3'-UTR (33), einem eukaryontischen polyA-Signal (32) sowie einem prokaryontischen Transkriptionsterminationssignal (nicht gezeigt) besteht. Bevorzugt ist dabei eine Kanamycin /Neomycin-Resistenzkassette. An den Enden wird das Konstrukt von Erkennungssequenzen (37) für eine prokaryontische Transposase (38) begrenzt. Bevorzugt werden dafür "19 bp mosaic end sequences", die von der Transposase EZ-TN5 erkannt werden. Zusammen bilden die genannten Elemente ein synthetisches Transposon.

Zur *in vitro* Transposition dieses Konstruktes wird dieses zunächst mit der Transposase (38) inkubiert, wobei diese an ihre Erkennungsstellen (37) bindet (Figur 20.2)) und ein Transposom bildet.

In Figur 20.3) ist das Plasmid dargestellt, in dessen genomisches Insert (39), das in die Restriktionsschnittstellen (40) und (41) kloniert wurde, das Transposon inseriert wird. Das Plasmid beinhaltet außerdem einen prokaryontischen Replikationsursprung (34) sowie ein Resistenzgen (42), das ein anderes als das (36) in dem Transposon enthaltene ist, z.B. das Ampicillinresistenz vermittelnde β-Lactamasesgen.

Für die eigentliche Transposition werden der Zielvektor und das Transposom in einem Verhältnis gemischt, das eine Insertion pro Plasmid bevorzugt, und inkubiert. Dabei wird das in dem Transposom enthaltene Transposon von der Transposase zufällig in den Zielvektor inseriert. Nach Termination der Reaktion werden die Reaktionsprodukte in geeignete *E .coli* Stämme transformiert und entsprechend dem in dem Transposon enthaltenen Resistenzgen (36) selektiert.

Aus überlebenden Klonen werden dann die Plasmide isoliert und der jeweilige Insertionspunkt der NMD-Kassette mittels Sequenzierung ermittelt. Darunter finden sich mit einer Wahrscheinlichkeit, die abhängig von der Größe des Vektors und von der Summe der Länge darin enthaltenen Introns des interessierenden Gens ist, solche, in denen sich die NMD-Kassette in einem Intron des interessierenden Gens befindet (Figur 20.4) und die deshalb für die gezielte Mutagenese des Gens in den Zielzelle, z.B. murine ES-Zellen, geeignet sind. Beinhaltet das klonierte Fragment mehrere Gene, können mit diesem Vorgehen unabhängige Vektoren für die gezielte Mutagenese dieser Gene identifiziert werden.

Für die homologe Rekombination in eukaryontischen Zellen wird der resultierende Vektor anschließend an einer der Restriktionsschnittstellen (40) in die das Insert kloniert wurde, linearisiert und in die Zielzellen, z.B. murine ES-Zellen, transfiziert (Figur 20.5)).

In Fortbildung des in Figur 20) dargestellten Verfahrens ist vorgesehen, statt eines Plasmids mit einem klonierten genomischen Fragment ein Gemisch von Plasmiden mit verschiedenen klonierten genomischen Fragmenten zur Herstellung von Bibliotheken von Vektoren für die gezielte Mutagenese in Eukaryonten zu verwenden.

In Fortbildung des in Figur 20) dargestellten Nukleinsäurekonstruktes sieht die Erfindung außerdem die Klonierung von für die gezielte Mutagenese in Eukaryonten geeigneten Vektoren aus größeren, z.B. in BACs klonierten, Fragmenten sowie aus Gemischen klonierter genomischer Fragmente, z.B. BAC-Bibliotheken, oder nicht klonierten genomischen Fragmenten vor.

Dazu sieht die Erfindung Nukleinsäurekonstrukte vor, die zusätzlich zu den in Figur 20.1) erläuterten Elementen einen mit den Elementen des Resistenzgen (35, 36, 33, 32) verbundenen prokaryontischen Replikationsursprung (34) enthalten, der zusammen mit diesen von den Erkennungssequenzen (14) für eine Rekombinase flankiert ist, so dass er in Anwesenheit der spezifischen Rekombinase zusammen mit dem Resistenzgen aus dem Konstrukt entfernt wird.

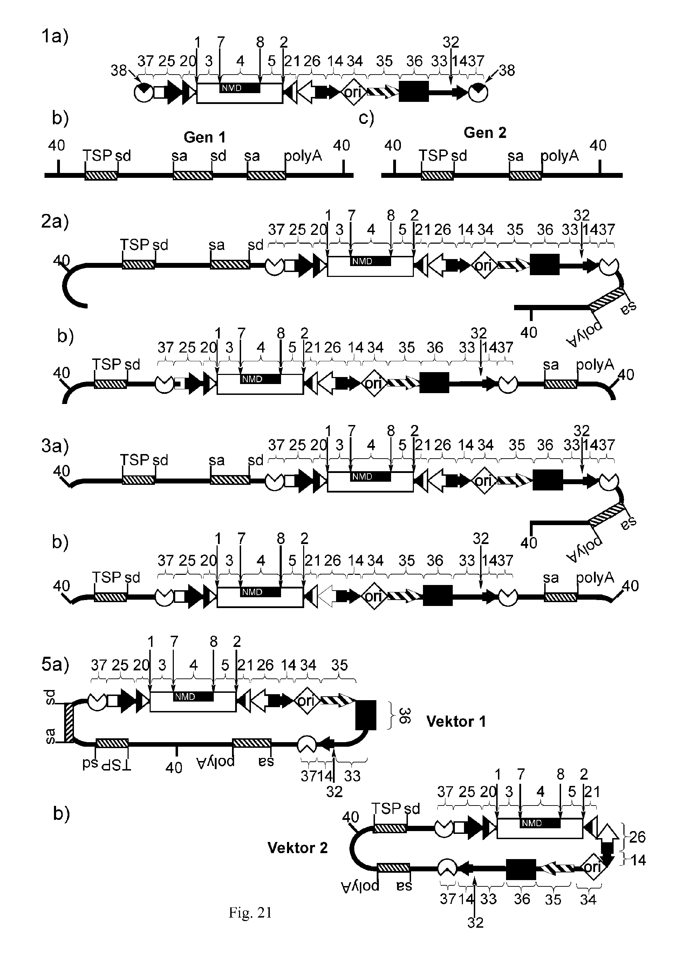

Figur 21.1 a) zeigt ein entsprechendes Transposom, das bereits die für die Erkennungsstellen (37) spezifische (38) Transposase gebunden hat. In Figur 21.1a) und 21.1b) sind zwei unterschiedliche Gene (Gen 1 und Gen 2) eines eukaryontischen Zielorganismus dargestellt, die gemeinsam auf einem klonierten Fragment liegen, unabhängig von einander in verschiedene Plasmide kloniert sein, oder sich auf nicht klonierten genomischen Fragmenten befinden können.

Die Figuren 21.2a) und 21.2b) zeigen das Ergebnis der Transposition, die wie in Figur 21) gezeigt durchgeführt wird. Beide Gene enthalten das erfindungsgemäße Transposon in einem ihrer Introns.

Zur Klonierung von Fragmenten, die ein erfindungsgemäße Nukleinsäurekonstrukt enthalten, wird die gesamte DNA der Reaktion aufgereinigt und anschließend mit einem Restriktionsenzym geschnitten, für das keine Schnittstellen (40) in dem erfindungsgemäßen Transposon vorliegen.

Die in Figur 21.3a) und b) dargestellten resultierenden Fragmente werden anschließend mittels Ligation zirkularisiert (Vektor 1 und Vektor 2 in Figur 21.5a) und b)), in *E. coli* transformiert und vermehrt. Durch Sequenzierung der Umgebung des inserierten Transposons kann der Insertionsort sequenzgenau bestimmt und Vektoren ausgewählt werden, die für die gezielte Mutagenese des jeweils interessierenden Gens geeignet sind, d.h., dass sie eine erfindungsgemäße NMD-Kassette im Intron eines Gens tragen.

Für die Transfektion werden die Vektoren mit demselben Restriktionsenzym (40) linearisiert, dass zur Erzeugung der in Figur 21.3a) und b) dargestellten Fragmente benutzt wurde. Die mit der NMD-Kassette verbundenen genomischen Sequenzen weisen darin dieselbe Abfolge und Orientierung auf wie in dem Genom, aus dem die genomische DNA ursprünglich stammte.

Neben den beschriebenen *in vitro* Anwendungen von als prokaryontische Transposons ausgeführten erfindungsgemäßen Konstrukten sieht die Erfindung die Anwendung derselben auch *in vivo* vor. Dafür werden Transposons, die mit der für die Transposaseerkennungssequenzen (37) spezifischen Transposase (38) in Bakterien eingebracht, die ein in einen Vektor kloniertes genomisches Fragment enthalten. Alternativ kann ein erfindungsgemäßes prokaryontisches Transposon auch ohne die Transposase in Bakterien eingebracht werden, die ein in einen Vektor kloniertes genomisches Fragment enthalten, und die spezifische Transposase gleichzeitig exprimieren.

### Beispiel 1: Inaktivierung des murinen β7- Integringens

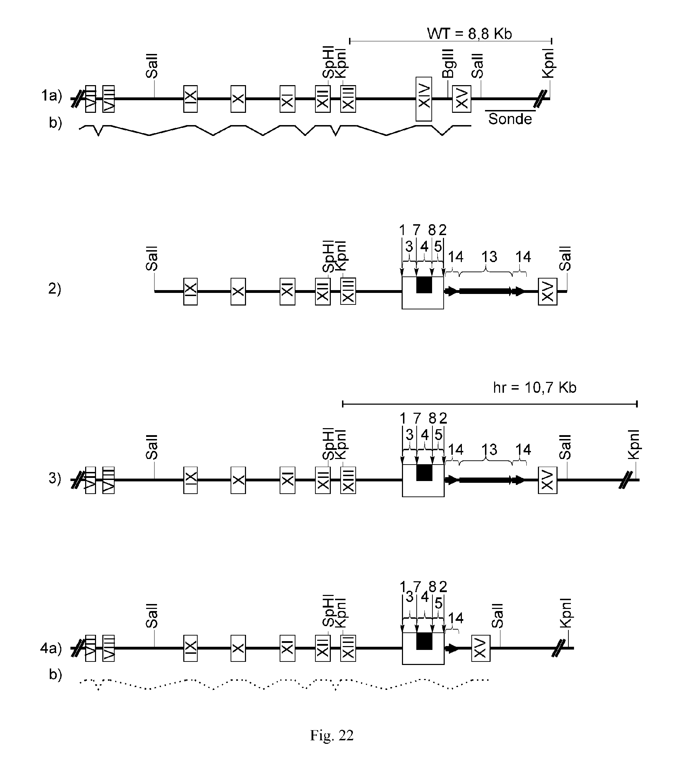

Die Funktionalität erfindungsgemäßer Nukleinsäurekonstrukte *in vivo* konnte durch Einführen eines NMD vermittelnden Exons (4), das ein prämatures Stopkodon enthält, in das β7-Integringen der Maus nachgewiesen werden (Figur 22). Dabei ersetzt das erfindungsgemäße Exon (4) das endogene Exon 14 (XIV) des Gens.

Als Grundlage für die Sequenz des NMD vermittelnden Exons wurde das endogene Exon 14 (XIV) genutzt, in dem ein als prämatures Stopkodon wirkendes Triplet (TAG) 87 bp stromaufwärts von der Spleißdonorstelle des Exons erzeugt wurde. Die flankierenden Introns blieben bei diesem Experiment unverändert, d.h. die erfindungsgemäß vorgesehenen Elemente Spleißakzeptor- (7) und Spleißdonorstelle (8), flankierende Intron abschnitte (3) und (5), Polypyrimidinabschnitt (PP) und Verzweigungspunkt (6) sind identisch mit den entsprechenden Elementen im murinen β7-Integringen.

Die NMD-Kassette war zusätzlich mit einem neo-Resistenzgen als Selektionsmarkergen (13) gekoppelt, das von Erkennungssequenzen (14) für flp-Rekombinase flankiert war. Der schematische Aufbau des Vektors für die homologe Rekombination in den murinen β7-Integrin-Locus ist in Figur 22) gezeigt.

Das NMD vermittelnde Exon wurde mittels PCR erzeugt, wobei ein kloniertes genomisches Fragment des murinen β7-Integringens als Matrize diente. Als Primer dienten zum einen Oligonukleotide, die der Wildtypsequenz entsprachen und primer, die bis auf das eingeführte Stopkodon den Sequenzen von Exon 14 (XIV) entsprechen.

In zwei unabhängigen Reaktionen wurden zunächst zwei PCR-Produkte amplifiziert, von denen das eine Teile von Exon 13 (XIII) mit einer darin lokalisierten SpHI Restriktionsschnittstelle, das gesamte Intron 13 sowie am 3'-Ende Teile von Exon 14 (XIV) mit der gewünschten Mutation enthielt. Das zweite Produkt enthielt das mutierte Exon 14 (XIV) an seinem 5'-Ende sowie Teile des 14. Introns mit einer darin lokalisierten BglII Restriktionsschnittstelle.

In einer weiteren PCR wurde aus den vorgenannten Produkten ein diese umfassendes Amplifikat hergestellt. Mittels Restriktion mit SpHI/BgIII wurden die Enden des PCR-Produktes entfernt und das resultierende Fragment dazu benutzt, das entsprechende Fragment in einem klonierten Sall-Fragment des murinen β7-Integringens zu ersetzen.

Anschließend wurde in den resultierenden Vektor eine mit frt-Erkennungssequenzen (14) flankierte neo-Selektionskassette in die identische BglII-Schnittstelle im Intron 14 (XIV) eingeführt.

Der resultierende Vektor ist in Figur 22.2) dargestellt und die DNA-Sequenz der in dem Konstrukt enthaltenen NMD-Kassette ist als Seq.-ID Nr. 1 bezeichnet.

Zum Einführen des erfindungsgemäßen NVD vermittelnden Exons (4) in das murine Genom wurden 30 µg des Konstrukts mit XhoI linearisiert und anschließend durch Elektroporation in embryonale Stammzellen (IB10) transfiziert. Diese elektroporierten ES-Zellen wurden anschließend auf neo-resistenten, wachstumsinhibierten embryonalen Mausfibroblasten ausgesät und zwei Tage nach der Transfektion einer Selektion mit G418 ausgesetzt, um Klone zu selektieren, die das Konstrukt stabil in ihr Genom eingebaut hatten.

Anschließend wurden homolog rekombinierte ES-Zellklone identifiziert, bei denen die Sequenzen des beschriebenen Vektors die Wildtypsequenzen in einem Allel des β7-Intergringens der ES-Zelle ersetzen, indem genomische DNA aus ihnen isoliert, mit dem Restriktionsenzym KpnI verdaut, auf einem Agarosegel aufgetrennt und mit einer spezifischen Sonde im Southern Blot hybridisiert wurde. Diese Sonde hybridisiert außerhalb des 3'-Endes der zur Herstellung des transfizierten Konstrukts benutzten Sequenzen und identifiziert das Wildtypfragment (WT) von 8,8 kb (Figur 22.1)) und bei homologer Rekombination des Vektors ein 10,7 kb großes Fragment (hr), das in Figur 22.3) gezeigt ist. Dabei konnten ES-Zellklone mit homolog rekombiniertem β7-Integringen identifiziert werden (nicht gezeigt).

Zur Deletion des neo-Resistenzgens (13) aus dem mutierten β7-Integrinallel wurde ein Expressionsplasmid für FLP-Rekombinase transient in Zellen des homolog rekombinierten Klons transfiziert. Resultierende Klone weisen die in Figur 22.4a) gezeigte genomische Struktur auf und wurden mittels einer PCR-Reaktion auf den Verlust des neo-Gens untersucht, die bei erfolgreicher Deletion neben einem 116 bp langen Wildtyp-Produkt ein 185 bp langes Amplifikat ergibt, wobei die größere Länge durch die im mutierten Allel verbleibende FRT-Sequenz verursacht wird (nicht gezeigt).

Homolog rekombinierte Klone, in denen das Resistenzgen neo erfolgreich entfernt wurde, wurden zur Generierung entsprechender chimärer Mäuse mittels Blastozysteninjektion benutzt. Aus den erhaltenen Chimären wurde eine Mauslinie durch Verpaaren mit Wildtyp-Mäusen etabliert, die das prämature Stopkodon in Exon 14 (XIV) des β7-Integringens stabil integriert enthält. Das Verpaaren heterozygoter Tiere dieser Linie wurde zur Generierung von Tieren eingesetzt, die diese Mutation in beiden Allelen tragen.

Blutproben aus homozygoten und heterozygoten Tiere sowie aus Mäusen mit zwei Wildtyp-Allelen wurden mittels FACS-Analyse auf die Expression des β7-Integrins auf Lymphozyten untersucht. Dazu wurde ein spezifischer Antikörper eingesetzt, der gegen die extrazelluläre Domäne des β7-Integrins gerichtet ist. Zur Kontrolle wurde Blut von Mäusen eingesetzt, in denen das β7-Integrin durch Insertion eines neo-Gens in Exon 12 und die gleichzeitige Deletion der Exon 13 und 14 komplett inaktiviert wurde (Wagner, Net al. (1996): Essential role for β7 integrins in the formation of the gut associated lymphoid tissue. Nature 382:366-370).

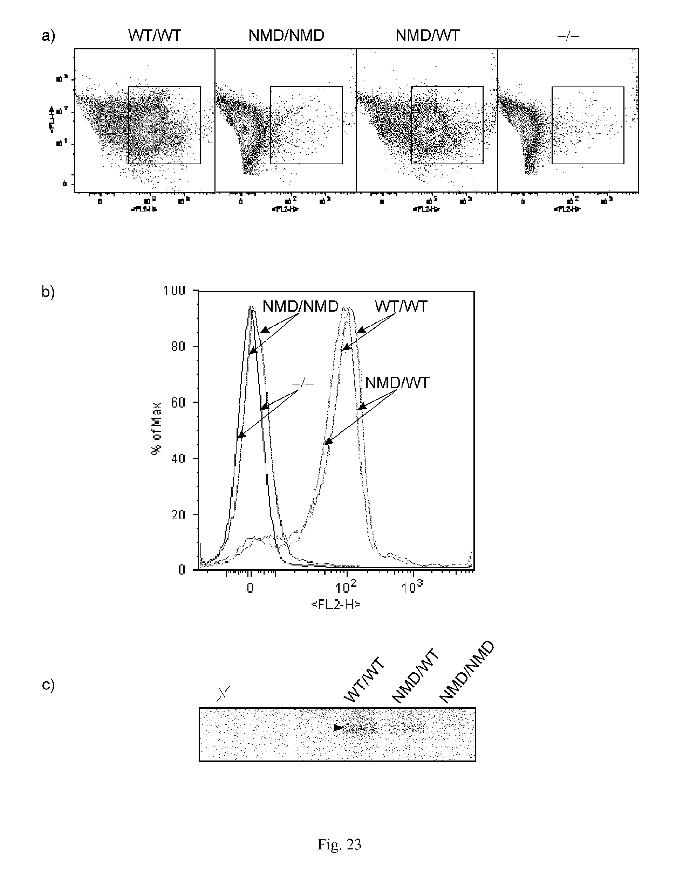

Die Ergebnisse der FACS-Analyse sind in Figur 23a) dargestellt. Dabei ist die die durch den β7-Integrin Antikörper verursachte Fluoreszenz jeweils auf der X-Achse, eine Kontrollfluoreszenz für DNA auf der Y-Achse aufgetragen. Der Bereich, in dem β7-Integrin-positive Zellen liegen, ist jeweils eingerahmt. Das Maß an Hintergrundfärbung in dem Kanal, in dem die β7-Integrin-spezifische Fluoreszenz gemessen wurde, kann anhand der Punkte innerhalb der eingerahmten Fläche des Diagrams für die Kontrolltiere (-/-) ersehen werden. Wildtyp-Mäuse (WT/WT) zeigen die normale Expression des β7-Integrins auf der Oberfläche der Lymphozyten. Heterozygote Tiere (NMD/WT) wiesen annähernd die gleiche Expression auf. Die Kontrolltiere, in denen das β7-Integringen komplett inaktiviert war (-/-), zeigten keine nachweisbare Expression des β7-Integrins. Auch erfindungsgemäße Tiere, in denen das prämature Stopkodon homozygot in Form der NMD-Kassette vorlag (NMD/NMD), zeigten ein nahezu vollständiges Fehlen von β7-Integrin auf der Oberfläche von Lymphozyten. Figur 23b) stellt die gleichen Ergebnisse als Histogramm dar.

Zum Nachweis, dass die mangelnde Expression des β7-Integrins in den Tieren mit dem erfindungsgemäß mutierten Exon 14 des β7-Integringens tatsächlich aufNMD beruht, wurden Northern-Blots mit RNA aus der Milz von Wildtyp-Mäusen, Kontrolltieren sowie erfindungsgemäßen homo- und heterozygoten Tieren mit einer Sonde für Transkripte aus dem β7-Integringen durchgeführt. Das Ergebnis ist in Figur 23c) gezeigt und macht deutlich, dass in erfindungsgemäßen homozygoten Tieren (NMD/NMD) nur sehr geringe Mengen der mRNA für β7-Integrin nachweisbar waren, während bei Kontrolltieren mit vollständig inaktiviertem β7-Integringen (-/-) keine spezifische mRNA nachweisbar war.
Der Nachweis geringer Mengen von mRNA für β7-Integrin wird darauf zurückgeführt, dass alternatives Spleißen dazu führt, dass Transkripte von β7-Integrin vorkommen, die kein Exon 14 aufweisen. Tatsächlich ist alternatives Spleißen des Exon 14 des Integringens inzwischen nachgewiesen worden und in der "Alternative Splicing Database" (Stamm S et al. (2006): ASD: a bioinformatics resource on alternative splicing. Nucleic Acids Res 34:D46-D55) annotiert: Bei murinen β7-Integrintranskripten kann ein Spleißen von Exon 13 auf Exon 15 erfolgen, wodurch das Exon 14 zusammen mit den Introns 13 und 14 aus dem Transkript gespleißt wird.

Abgesehen von dem endogen verursachten alternativen Spleißen des Exon 14 zeigen die mit dem murinen β7-Integringen erzielten Ergebnisse, dass erfindungsgemäße Nukleinsäurekonstrukte zur Inaktivierung eukaryontischer Gene *in vivo* eingesetzt werden können.

### Beispiel 2: Universell einsetzbare NMD-Kassetten

Die Erfindung bevorzugt die Verwendung von nicht alternativ gespleißten Exons in NMD-Kassetten. Deshalb wurde nach Genen gesucht, die in Maus und Mensch kein oder nur wenig alternatives Spleißen aufweisen. Von der weiteren Verwendung ausgeschlossen, wurden solche Exons, die für die geplanten Anwendungen störende Spleißsignale im nicht-kodierenden Strang aufwiesen. Entsprechend der für das Exon 14 des murinen β7-Integringens beschriebenen PCR-Methodik wurden in Frage kommende Exons zusammen mit flankierenden Intronsequenzen, die den bevorzugten Intronabschnitten (3) und (5) entsprechen, so amplifiziert, dass drei prämaturen Stopkodons in der erfinderisch vorgesehenen Position in das Exon eingeführt wurden. Außerdem wurden durch die Primer jeweils ClaI-Restriktionsschnittstellen an den Enden des PCR-Produktes erzeugt. Die PCR-Produkte wurden zunächst in einen TA-Klonierungsvektor ligiert und die Identität des Produktes mittels Sequenzierung verifiziert.

Die PCR-Produkte wurden dann so in das Intron eines Luciferasereportergenvektors kloniert, dass das NMD vermittelnde Exon im gleichen Strang wie die Exons des Luciferasegens lokalisiert war. Die Vektoren wurden dann transient in A293 Zellen transfiziert und 24 Stunden später die Reportergenaktivität ermittelt. Dabei verursachte eine der getesteten NMD-Kassetten eine völlige Inaktivierung des Reportergens (Daten nicht gezeigt). Diese als NMD4 bezeichnete Kassette beinhaltet, einen 87 bp 3'-terminalen Abschnitt des Introns 22 (3), ein mit drei Stopkodons erfindungsgemäß modifiziertes Exon 23 (4) sowie 114 bp des 5'-Endes des Introns 23 des humanen Retinoblastomgens (RB1). Die Sequenz wird als Seq.-ID Nr. 2 bezeichnet.

Da die Erfindung neben NMD-Kassetten, die Gene vollständig inaktivieren, auch solche vorsieht, die nur eine anteilige Inaktivierung von Genen verursachen, um gezielt hypomorphe Mutanten herstellen zu können, wurde aus dem Exon 14 und flankierenden Intronsequenzen des murinen Integringens eine universell einsetzbare NMD-Kassette mit prämaturen Stopkodons in allen drei Leserahmen entwickelt, deren Sequenz als Seq.-ID Nr. 3 bezeichnet wird. An den Enden wird die itgb7-NMD-Kassette von PstI Restriktionsschnittstellen flankiert.

### Beispiel 3: Steuerung der Aktivität eines Zielgens durch Inversion von darin enthaltenen NMD-Kassetten

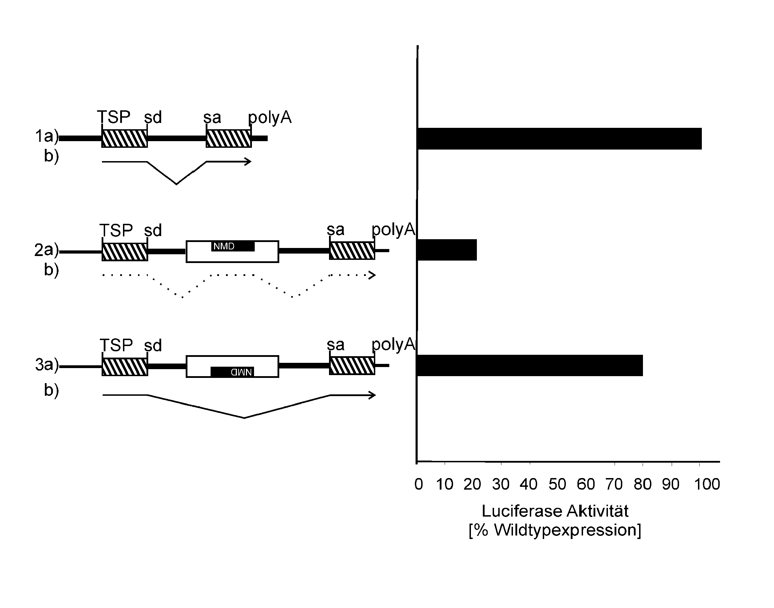

Als Beispiel für ein zu inaktivierendes Gen wurde der Reportergenvektor pCMVluc-lambdaIn2-m2A benutzt, der ein mit einem Intron aus dem lambda2-Gen der Maus in zwei Exons geteiltes Luziferasegen aus *Photinus pyralis* enthält. In das Intron des Luciferasegens wurde ein zusätzlicher Polylinker mit einer PstI und einer ClaI Restriktionsschnittselle kloniert Figur 24.1a). In die PstI-Schnittstelle wurde die NMD1-Kassette, die das mutierte Exon 14 des murinen β7-Integringen und die flankierenden Intronbereiche enthält, in beiden Orientierungen kloniert. Die resultierenden Vektoren werden hier als Luc-NMD1 und Luc-1DMN bezeichnet, wobei LucNMD1 das NMD vermittelnde Exon im kodierenden Strang des Luciferasegens enthält (Figur 24.2a)), und Luc1DMN dasselbe im nicht-kodierenden Strang trägt (Figur 24.3a)).

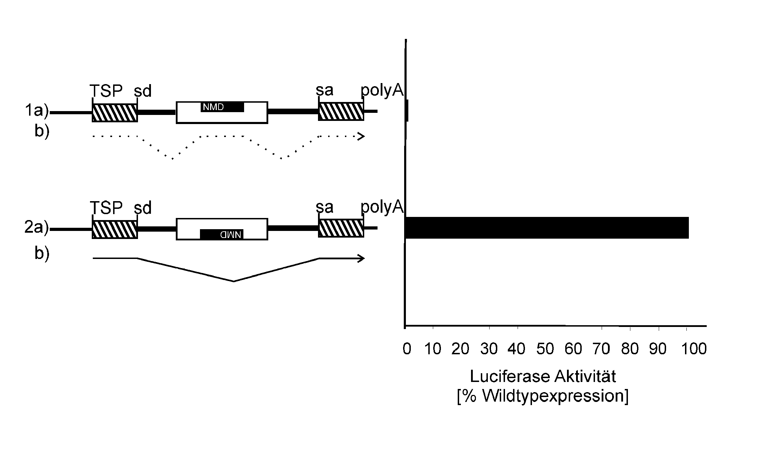

Die NMD4-Kassette mit dem mutierten Exon 23 und den flankierenden Intronsequenzen wurde ebenfalls in beiden Orientierungen in die ClaI Schnittstelle des Reportergenvektors kloniert. Die resultierenden Vektoren werden hier als LucNMD4 (Figur 25.1a)) und Luc4DMN (Figur 25.2a) bezeichnet, wobei der erstgenannte das NVD vermittelnde Exon im kodierenden Strang trägt und der andere es im nicht-kodierenden Strang aufweist.

Zur Prüfung der Wirksamkeit der beiden NMD-Kassetten in der jeweiligen Orientierung wurden die Vektoren Luc-NMDl, Luc-1DMN, Luc-NMD4 und Luc-4DMN transient in Zellen der Linie A293 transfiziert. Als Kontrolle wurde der Ursprungsvektor ohne NMD-Kassette verwendet. Zum Abgleich eventuell auftretender Unterschiede wurde ein zweites Plasmid co-transfiziert, das ein Luciferasegen aus *Renilla reniformis* enthält, welches unabhängig von der *Photinus* Luciferase gemessen werden kann.

In drei unabhängigen Experimenten wurden jeweils fünf unabhängige Transfektionen durchgeführt. 24 Stunden nach der Transfektion wurden die Zellen lysiert und die Aktivitäten der beiden Luciferasen luminometrisch bestimmt.

Mit dem Vektor Luc1DMN, d.h. bei der Verbindung des nicht-kodogenen Stranges des mutierten Exon 14 des murinen β7-Integrin-Gens mit dem kodierenden Strang des Reportergens, war im Vergleich zu dem Wildtyp-Reportergenvektor pCMVluc-lambdaIn2-m2A (Figur 24.1)) keine wesentliche Abnahme der Reportergenaktivität festzustellen (Figur 24.3)). Demgegenüber wiesen Extrakte von Zellen, die mit dem Vektor Luc1DMN transfiziert wurden, in dem das mutierte Exon 14 des murinen β7-Integrin-Gens mit dem kodogenen Strang des Reportergens verbunden ist (Figur 24.2)), eine um 80% verringerte Luciferaseaktivität im Vergleich zum Wildtyp-Konstrukt auf. Dies zeigt zum einen, daß NMD-Kassetten prinzipiell geeignet sind, die Expression von eukaryontischen Genen zu minimieren. Zum anderen wird anhand dieses Ergebnisses deutlich, daß die 20-prozentige Restaktivität auf alternativem Spleißen beruht, bei dem das NMD-vermittelnde Exon aus dem Primärtranskript herausgespleißt wird, da Transkripte, die das als NMD-Kassette wirkende Exon enthalten aufgrund der drei in diesem Exon beinhalteten Stopkodons selbst dann kein funktionelles reifes Luciferase-Protein kodieren könnten, wenn entsprechende Transkripte nicht dem NMD unterliegen würden.

Im Gegensatz dazu wurde mit dem Vektor Luc-NMD4, der das mutierte Exon 23 des humanen RB1-Gens im kodogenen Strang des Reportergens enthält, eine im Vergleich zum Kontrollvektor pCMVluc-lambdaln2-m2A vollständige (>99%) Inaktivierung des Reportergens beobachtet (Figur 25.1)). Da die NMD4-Kassette außerdem die Reportergenaktivität unbeeinflußt läßt, wenn sie sich im nicht-kodogenen Strang des Reportergens befindet (Figur 25.2), erfüllt sie die erfindungsgemäßen Anforderungen an eine NMD-Kassette.

### Beispiel 4: Als Transposon geeignete, zweifach invertierbare NMD-Kassette

Um erfindungsgemäßes Nukleinsäurekonstrukt mit den optional vorgesehenen Erkennungssequenzen für Cre-Rekombinase und flp-Rekombinase sowie mit 19ME Erkennungssequenzen für die prokaryontische Transposase EZ-Tn5 zu versehen und die für weitere Klonierungsschritte notwendigen Restriktionsschnittstellen bereitzustellen, wurde zunächst ein komplett synthetisches DNA-Fragment hergestellt, das die genannten Elemente in der für ihre Funktionalität notwendigen Orientierung enthält. Die Sequenz dieses Fragmentes ist in der Sequenz des hier beschriebenen Konstruktes annotiert. Das synthetische Fragment wurde in die KpnI und die SacI Schnittstellen eines Klonierungsvektors ligiert und dem resultierenden Vektor erfolgten die weiteren Klonierungsschritte.

Als nächstes wurde die NMD4-Kassette mit dem Restriktionsenzym ClaI aus dem Vektor, in den das ursprüngliche PCR-Produkt kloniert worden war, ausgeschnitten und in die dafür vorgesehene ClaI-Schnittstelle des synthetischen Fragmentes ligiert. Durch Sequenzierung wurde die Orientierung der NMD4-Kassette überprüft. Im nächsten Schritt wurde der resultierende Vektor mit SalI in dem synthetischen Fragment aufgeschnitten. In diese Schnittstelle wurde dann ein prokaryontisch und eukaryontisch selektierbares neo-Resistenzgen ligiert, das von attP und attB Erkennungssequenzen für die phiC31-Integrase flankiert ist und mit SalI und XhoI aus seinem Vektor isoliert wurde.
Der resultierende Vektor entspricht dem in Figur 20.1 dargestellten Vektor und beinhaltet die folgenden Elemente bezogen auf die in 5'→3' Richtung des in der NMD4-Kassette beinhalteten NMD vermittelnden Exons 23 aus dem humanen RB1-Gen :
- Schnittstellen für die Restriktionsenzyme BelI, MfeI, NdeI, PciI und PshAI,
- 19ME-Erkennungsequenz für EZ-Tn5 Transposase (37)
- Schnittstellen für die Restriktionsenzyme XhoI, SciI und HindIII,
- frtLE Erkennungssequenz (25) für flp-Rekombinase
- lox66 Erkennungssequenz (20) für Cre-Rekombinase
- 3'-Ende des Introns 22 des humanen RB1-Gens (3)
- Exon 23 des humanen RB1-Gens, versehen mit drei Stopkodons in jedem möglichen Leserahmen (4) mit Spleißakzeptor- (7) und Spleißdonorstelle (8)
- 5'-Ende des Introns 23 des humanen RB1-Gens (5)
- 1ox71 Erkennungssequenz (21) für Cre-Rekombinase, in entgegengesetzter Orientierung zu 1ox66 (209
- frtRE Erkennungssequenz (26) für flp-Rekombinase in entgegengesetzter Orientierung zu frtLE (25)
- attP Erkennungssequenz (14) für phiC31-Integrase
- Resistenzgen, bestehend aus eukaryontischem PGK- und prokaryontischem gb2-Promotoren (35), offener Leserahmen für neo/Kanamycin-Resistenzgen (36), eukaryontische und prokaryontische Transkriptionsterminationssignale (32,33)
- attB Erkennungssequenz (14) für phiC31-Integrase
- 19ME-Erkennungsequenz für EZ-Tn5 Transposase (37)

Eine Überprüfung der Funktionalität der Rekombinaseerkennungsstellen erfolgte in Bakterien, die Cre- oder flp-Rekombinase exprimierten. In beiden Bakterienstämmen wurde die NMD-Kassette jeweils irreversibel invertiert (nicht gezeigt).

Der Nachweis, dass das Nukleinsäurekonstrukt für die Herstellung von Vektoren für die gezielte Geninaktivierung geeignet ist, erfolgte entsprechend dem in Figur 20) beschriebenen Verfahren. Dazu wurde ein PsHAI Fragment aus dem Vektor isoliert, dass an den Enden von 19ME-Erkennungssequenzen für die EZ-TN5-Transposase flankiert ist und die von Erkennungssequenzen für Cre- und flp-Rekombinase flankierte NMD-Kassette sowie das mit Erkennungssequenzen für phiC31-Integrase flankierte Resistenzgen enthält. Dieses Fragment wurde mit rekombinanter EZ-ZTn5 Transposase zur Bildung von Transposomen inkubiert. Die so gebildeten Transposomen wurden dann mit einem Plasmid mit Ampicillinresistenzgen (42) gemischt, das ein 6 kb langes kloniertes genomisches Fragment des murinen HPRT-Gens (39) enthielt. Nach Termination der Transposomenreaktion wurden die Reaktionsprodukte in E. coli transformiert und auf Agarplatten selektiert, die Kanamycin und Ampicillin als Antibiotika enthielten. Aus darauf gewachsenen Klonen wurden die Plasmide isoliert und mittels Restriktionsverdaus (nicht gezeigt) sowie Sequenzierung die jeweiligen Insertionspunkte der Transposons und ihre Orientierung ermittelt.

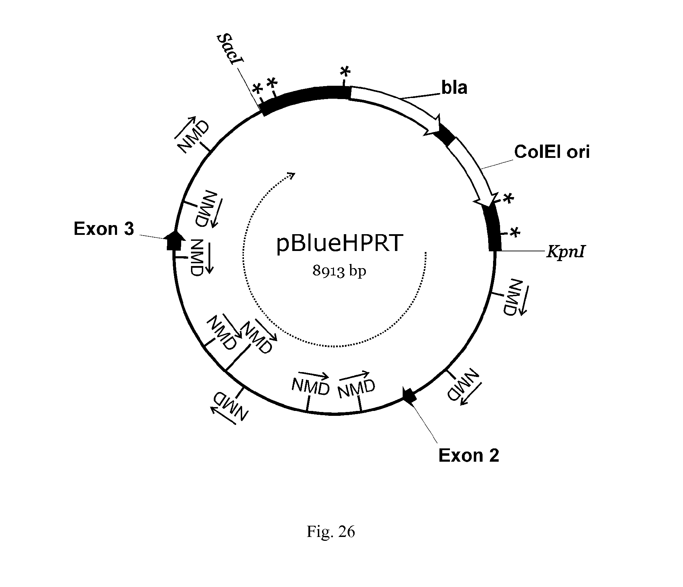

Die ermittelten Insertionspunkte der Transposons und ihre jeweilige Orientierung in 14 einzelnen Plasmiden sind in Figur 26) dargestellt. Der Vektor pBueHPRT besteht aus dem als schwarzer Balken dargestellten Klonierungsvektor pBluescript II mit Ampicillin-Resistenz (bla) und eine ColEl-Replikationsursprung (ColEl ori), der zwischen seinen KpnI- und SacI-Schnittstellen ein als schwarze Linie dargestelltes genomisches Fragment aus dem murinen HPRT-Gen mit dem 2. und 3. Exon des Gens (Exon 2, Exon 3) enthält. Die Pfeilspitze der gepunkteten Linie stellt die Transkriptionsrichtung des HPRT-Gens dar. Inserierte erfindungsgemäße Transposons sind mit NMD und einem Pfeil gekennzeichnet, wobei die Pfeilrichtung die 5'→3' Richtung des NMD vermittelnden Exons (4) angibt. Transposons, deren Insertion so erfolgte, dass das NMD vermittelnde Exon (4) in dem gleichen Strang lokalisiert ist wie die Exons des HPRT-Gens (Exon 2, Exon 3), sind außerhalb der das HPRT-Fragment darstellenden Linie gezeigt. Transposons, die in der entgegengesetzten Orientierung inseriert wurden sind innerhalb dieser Linie dargestellt. Transposons, die innerhalb des pBluscript-Anteils des Vektors inserierten, sind durch Sterne gekennzeichnet (*).

Von 20 untersuchten Plasmiden, wiesen 19 jeweils nur ein inseriertes Transposon auf, in ein Plasmid waren zwei Transposons inseriert worden. Von den 19 Plasmiden mit nur einem Transposon wiesen fünf das Transposon im pBluescript II-Anteil auf. Bei den anderen 14 erfolgte die Insertion in das genomische Fragment des HPRT-Gens. Aufgrund der doppelten Länge (6 kb) des Inserts im Vergleich zum Vektor (3 kb) ist eigentlich zu erwarten, dass die Anzahl der in den Vektoranteil inserierten Transposons ungefähr halb so groß ist wie die Anzahl der in das Insert inserierten. Die geringere beobachtete Anzahl von Insertionen in den Vektoranteil ist darauf zurückzuführen, dass Insertionen in das β-Lactamasegen (bla) und in den ColE1 Replikationsursprung (ColE1 ori) zum Verlust der Resistenz bzw. der Replikationsfähigkeit führen. Beides führt dazu, dass Bakterien, die derart von den Transposons getroffene Plasmide aufgenommen haben, in Gegenwart von Ampicillin nicht wachsen können, und entsprechende Klone nicht nachweisbar sind.

Bei fünf der 14 Plasmide mit einem Transposon im genomischen HPRT-Fragment konnte der genaue Insertionsort mit dem betriebenen Sequenzieraufwand nicht genau bestimmt, da sie in repetitive Elemente inseriert wurden, deren Sequenz mehrfach in dem Fragment auftaucht, was größere Leseweiten bzw. zusätzliche Sequenzreaktionen erfordert hätte. In den verbleibenden neun Plasmiden konnte jeweils der genaue Insertionsort des Transposons bestimmt werden. Fünf enthielten das Transposon in der Orientierung, in der das NMD vermittelnde Exon (4) sich im nicht-kodogenen Strang des HPRT-Fragmentes befand. Diese Vektoren könnten also direkt zur Herstellung eines konditional inaktivierbaren Allels in ES-Zellen mittels homologer Rekombination genutzt werden, wie dies in Figur 13) dargestellt ist. Die verbleibenden vier Plasmide enthielten das Transposon in der umgekehrten Orientierung, in der das NMD vermittelnde Exon (4) Teil desselben Stranges wie die beiden Exons des HPRT-Gens ist. Dementsprechend können diese Vektoren benutzt werden, um ein HPRT-Allel in ES-Zellen zunächst direkt in eine inaktivierte, konditional reaktivierbare Form zu überführen, wie dies in Figur 15) beschrieben wurde. Entsprechend der in den Figuren 13) und 15) dargestellten Vorgehensweise kann in beiden Fällen eine erste konditionale Änderung des Aktivitätszustandes des HPRT-Gens von einer zweiten gefolgt werden.

### Bezugszeichenliste:

1 = 5'-Ende einer minimalen NMD-Kassette / 5'-Ende des ersten Intronabschnitts (3)
2 = 3'-Ende einer minimalen NMD-Kassette / 3'-Ende des zweiten Intronabschnitts (5)
3 = erster Intronabschnitt
4 = NMD vermittelndes Exon
5 = zweiter Intronabschnitt
6 = Verzweigungspunkt
7 = Spleißakzeptorstelle
8 = Spleißdonorstelle
9 = dritter Intronabschnitt, dessen 5'-Ende (16) zusammen mit Sequenzen (15) eines Exons, in das die NMD-Kassette inseriert wurde, eine neue Spleißdonorstelle (18) ergibt
10 = vierter Intronabschnitt, dessen 3'-Ende (17) zusammen mit Sequenzen (15) eines Exons, in das die NMD-Kassette inseriert wurde, eine neue Spleißakzeptorstelle (19) ergibt
11 = zu mutierendes Kodon (Beispiel GAG)
12 = Stopkodon, das durch Ersetzen des Exons B durch das in der NMD-Kassette enthaltene Exon B' inseriert wurde
13 = Resistenzgen (Promotor/ORF/3'-UTR/polyA-Signal)
14 = Erkennungssequenzen für Rekombinase 1
15 = Exonabschnitt, der eine Protospleißstelle bildet, die zusammen mit Anteilen (16 und 17) der Enden der NMD-Kassette nach Insertion derselben funktionale Spleißdonor- (18) und Spleißakzeptorstelle (19) ergibt
16 = intronischer Anteil einer Spleißdonorstelle
17 = intronischer Anteil einer Spleißakzeptorstelle
18 = durch Insertion eines Nukleinsäurekonstruktes neu erzeugte Spleißdonorstelle
19 = durch Insertion eines Nukleinsäurekonstruktes neu erzeugte Spleißakzeptorstelle
20, 21 = unterschiedliche, entgegengesetzt orientierte Mutanten von Erkennungssequenzen für Rekombinase 2
22, 23 = durch Inversion neu erzeugte Erkennungsstellen für Rekombinase 2, die nicht mehr miteinander interagieren können
24 = gleiche Erkennungssequenzen für Rekombinase 2 in gleicher Orientierung
25, 26 = unterschiedliche, entgegengesetzt orientierte Mutanten von Erkennungssequenzen für Rekombinase 3
27, 28 = durch Inversion neu erzeugte Erkennungsstellen für Rekombinase 3, die nicht mehr miteinander interagieren können
29 = gleiche Erkennungssequenzen für Rekombinase 3 in gleicher Orientierung
30 = Reportergen/Selektionsmarker
31 = Spleißakzeptor für Reportergen/Selektionsmarker
32 = polyA-Signal für Reportergen/Selektionsmarker
33 = 3'-UTR für Reportergen/Selektionsmarker
34 = prokaryontischer Replikationsursprung (ori)
35 = prokaryontischer / eukaryontischer Promotor
36 = ORF für prokaryontisch und eukaryontisch selektierbaren Selektionsmarker
37 = Erkennungssequenzen für Transposase
38 = Transposase
39 = Zwischen Restriktionsschnittstellen (40 und 41) kloniertes genomisches Fragment
40 = Restriktionsschnittstelle
41 = Restriktionsschnittstelle für anderes Enzym als (40)
42 = Resistenzgen mit anderer Spezifität als (36)

PP = Polypyrimidinabschnitt
A, B, C usw. = Exons von Zielgenen
A', B' usw. = mutierte Exons, die Wildtypexons (A, B) ersetzen
A1, A2 und B1, B2 = Exons, die durch Zerteilung des Exons (A) b zw. des Exons (B) mittels Insertion eines erfindungsgemäßen Nukleinsäurekonstrukts generiert wurden
sa = Spleißakzeptorstelle
sd = Spleißdonorstelle
TSP = Transkriptionsinitiationspunkt
polyA = poly-Adenylierungssignal
polyA1, polyA2 = poly-Adenylierungssignale alternativ gespleißter 3'-terminaler Exons
stop = Stopkodon
VII - XIV = Exons des murinen β7-Integrin-Gens (Exons I bis VI nicht dargestellt)
WT = hybridisierendes Fragment im Wildtyp
hr = hybridisierendes Fragment im homolog rekombinierten Allel
Sonde = Sonde zur Hybridisierung eines Southern blots
Bg1II, KpnI, SpHI, usw. = Schnittstellen für Restriktionsenzyme gleichen Namens
WT/WT = Mäuse, in den beide Allele des β7-Integringens in Wildtypkonfiguration vorliegen NMD/WT = Mäuse mit einen Wildtyp und einem erfindungsgemäß mutierten β7-Integrinallel NMD/NMD = Mäuse, in den beide Allele des β7-Integringens erfindungsgemäß mutiert sind
-/- = Mäuse, in denen beide β7-Integrinallele durch Insertion eines neo-Gens in ein Exon inaktivert sind
   →
NMD = als Transposon ausgeführtes erfindungsgemäßes Nukleinsäurekonstrukt

## Patentansprüche

1. Nukleinsäure zur Inaktivierung eukaryontischer Gene, umfassend eine NMD-Kassette, die die folgenden funktionalen Elemente aufweist:
a. eine Spleißakzeptorstelle (7),
b. in 5' Position von der Spleißakzeptorstelle (7) einen synthetischen oder natürlichen ersten Intronabschnitt (3),
c. in 3'-Richtung von der Spleißakzeptorstelle (7) eine Spleißdonorstelle (8),
d. wobei zwischen Spleißakzeptorstelle (7) und Spleißdonorstelle (8) ein funktionales Exon (4) gebildet wird, das mindestens ein Stopkodon enthält,
e. in 3'-Richtung von der Spleißdonorstelle (8) einen synthetischen oder natürlichen zweiten Intronabschnitt (5).

2. Nukleinsäure nach Anspruch 1, **dadurch gekennzeichnet, dass**
der erste Intronabschnitt (3) ein unvollständiges Intron ist, dem nur an seinem 5'-Ende (1) die zum Spleißen notwendigen funktionalen Elemente fehlen, und der zweite Intronabschnitt (5) ein unvollständiges Intron ist, dem nur an seinem 3'-Ende (2) die zum Spleißen notwendigen funktionalen Elemente fehlen.

3. Nukleinsäure nach Anspruch 1 oder 2, **dadurch gekennzeichnet, dass** sie innerhalb des Intronabschnitts (3) einen um 10 bis 50 Nukleotide in 5' von der Spleißakzeptorstelle (8) beabstandeten Verzweigungspunkt (6) aufweist.

4. Nukleinsäure nach Anspruch 1 und 2, **dadurch gekennzeichnet, dass** sie einen in 3' Richtung an den Verzweigungspunkt angrenzenden Polypyrimidinabschnitt (PP) aufweist.

5. Nukleinsäure nach Anspruch 3, **dadurch gekennzeichnet, dass** der Polypyrimidinabschnitt (PP) 10 - 50 Nukleotide umfasst.

6. Nukleinsäure nach einem der vorangehenden Ansprüche, **dadurch gekennzeichnet, dass** der erste Intronabschnitt (3) 5' von dem Verzweigungspunkt keine Spleißdonorstellen, Spleißakzeptorstellen, Verzweigungspunkte und keinen Polypyrimidinabschnitt aufweist.

7. Nukleinsäure nach einem der vorangehenden Ansprüche, **dadurch gekennzeichnet, dass** der zweite Intronabschnitt (5) 3' von den intronischen Anteilen der Spleißdonorstelle (8) keine Spleißdonorstellen, Spleißakzeptorstellen, Verzweigungspunkte und keinen Polypyrimidinabschnitt aufweist.

8. Nukleinsäure nach einem der Ansprüche 7 und 8, **dadurch gekennzeichnet, dass** der 5' vom Verzweigungspunkt lokalisierte Sequenzabschnitt des ersten Intronabschnitts (3) 10 bis 100 bp umfaßt und/oder der 3' von den intronischen Anteilen der Spleißdonorstelle lokalisierte Sequenzabschnitt des zweiten Intronabschnitts (5) 10 bis 100 bp umfaßt.

9. Nukleinsäure nach einem der vorangehenden Ansprüche, **dadurch gekennzeichnet, dass** das mindestens eine Stopkodon drei Stopkodons sind, davon eins in jedem möglichen Leserahmen des Exons (4).

10. Nukleinsäure nach einem der vorangehenden Ansprüche, **dadurch gekennzeichnet, dass** das mindestens eine Stopkodon mehr als 50, bevorzugt mehr als 55 bp von der Spleißdonorstelle (8) beabstandet ist.

11. Nukleinsäure nach einem der vorangehenden Ansprüche, **dadurch gekennzeichnet, dass** das Exon (4) eine nicht glatt durch 3 teilbare Anzahl von Nukleotiden aufweist

12. Nukleinsäure nach Anspruch 11, **dadurch gekennzeichnet, dass** das Exon (4) kein Stopkodon aufweist.

13. Nukleinsäure nach einem der vorangehenden Ansprüche, **dadurch gekennzeichnet, dass** der komplementäre Strang keine funktionalen Exons und keine funktionalen Spleißsignale enthält.

14. Nukleinsäure nach Anspruch 13, **dadurch gekennzeichnet, dass** die funktionalen Spleißsignale ausgewählt sind unter Spleißdonorstellen, Spleißakzeptorstellen, Polypyrimidinabschnitten und Verzweigungspunkten.

15. Nukleinsäure nach einem der vorangehenden Ansprüche, **gekennzeichnet durch** jeweils beidseitig flankierende Erkennungssequenzen (20, 21, 24) für mindestens eine Rekombinase oder Integrase.

16. Nukleinsäure nach Anspruch 15, **dadurch gekennzeichnet, dass** die Erkennungssequenzen für mindestens eine Rekombinase oder Integrase zwei oder mehr Paare von Erkennungssequenzen (20, 21, 24, 25, 26, 29) für verschiedene Rekombinasen oder Integrasen sind.

17. Nukleinsäure nach Anspruch 15 oder 16, **dadurch gekennzeichnet, dass** die Erkennungssequenzen für Rekombinase ausgewählt sind aus der Gruppe, die
a. die von Cre-Rekombinase benutzten loxP-Sequenzen sowie mutierte Formen davon, bevorzugt lox66 und lox71,
b. die von Flp-Rekombinase erkannten frt-Sequenzen sowie mutierte Formen davon, bevorzugt frtLE und frtRE,
c. rox-Erkennungssequenzen für Dre-Rekombinase sowie
d. attP- und attB-Erkennungssequenzen für phiC31-Integrase
umfasst.

18. Nukleinsäure nach einem der Ansprüche 15 bis 17, **dadurch gekennzeichnet, dass** weitere Paare von Erkennungssequenzen für andere Rekombinasen oder Integrasen die funktionalen Elemente flankieren, die die Invertierung oder oder die Deletion der zwischen ihnen angeordneten funktionalen Elemente jeweils irreversibel zulassen.

19. Nukleinsäure nach einem der vorangehenden Ansprüche, **gekennzeichnet durch** die Kopplung mit einer Expressionskassette für ein Selektionsmarkergen

20. Nukleinsäure nach einem der vorangehenden Ansprüche, **gekennzeichnet dadurch, dass** das Selektionsmarkergen in Eukaryonten positiv oder positiv und anschließend negativ selektierbar ist.

21. Nukleinsäure nach einem der vorangehenden Ansprüche, **gekennzeichnet dadurch, dass** die Expressionskassette des Selektionsmarkergens sowohl in Eukaryonten als auch in Prokaryonten selektierbar ist.

22. Nukleinsäure nach einem der vorangehenden Ansprüche, **gekennzeichnet durch** einem prokaryontischen Replikationsursprung.

23. Nukleinsäure nach einem der vorangehenden Ansprüche, **gekennzeichnet durch** ein Reportergen in einer Expressionskassette mit interner Ribosomeneintrittsstelle (IRES) oder ein Reportergen, das im Leserahmen in einer Expressionskassette eines Selektionsmarkergens enthalten ist.

24. Nukleinsäure nach einem der Ansprüche 20 bis 23, **gekennzeichnet dadurch, dass** der Expressionskassette des Selektionsmarkergens oder des Reportergens ein zur Expression notwendiges regulatorisches Element fehlt, das durch eukaryontische regulatorische Elemente ersetzbar ist.

25. Nukleinsäure nach einem der Ansprüche 19 bis 24, **gekennzeichnet dadurch, dass** die Expressionskassette für das Selektionsmarkergen oder das Reportergen ein 3'-terminales Exon ist, das an seinem 5'-Ende eine Spleißakzeptorstelle aufweist und stromaufwärts einen Polypyrimidinabschnitt sowie daran anschließend einen Verzweigungspunkt aufweist und stromabwärts des Stopkodons des Selektionsmarkergens oder des Reportergens ein Polyadenylierungsignal angeordnet ist.

26. Nukleinsäuren nach Anspruch 25, **gekennzeichnet dadurch, dass** das terminale Exon in den Intronphasen 0, 1 oder 2 vorliegt.

27. Nukleinsäure nach einem der Ansprüche 19 bis 26, **dadurch gekennzeichnet, dass** die Expressionskassette des Reportergens und/oder des Selektionsmarkergens (13) von Erkennungssequenzen (14) flankiert ist, die bei Wechselwirkung mit der spezifischen Rekombinase oder Integrase die Deletion des Reporter- und/oder des Selektionsmarkergens (13) ermöglichen, wobei die Erkennungssequenzen für eine andere Rekombinase oder Integrase spezifisch sind als diejenigen, die die NMD-Kassette flankieren.

28. Nukleinsäure nach einem der Ansprüche 19 bis 27, **dadurch gekennzeichnet, dass** die funktionalen Elemente der NMD-Kassette und die kodierenden Sequenzen des Selektionsmarkergens und/oder des Reportergens im gleichen Strang des DNA-Moleküls angeordnet sind.

29. Nukleinsäure nach einem der Ansprüche 19 bis 27, **dadurch gekennzeichnet, dass** die funktionalen Elemente der NMD-Kassette im anderen Strang des DNA-Moleküls als die kodierenden Sequenzen des Selektionsmarkergens und/oder des Reportergens angeordnet sind.

30. Nukleinsäure nach einem der vorangehenden Ansprüche, **dadurch gekennzeichnet, dass** in 5'-Richtung vom ersten Intronabschnitt (3) ein dritter Intronabschnitt (9) angeordnet ist, der seinem 5'-Ende die intronischen Anteile einer Spleißdonorstelle (16) aufweist, und zusammen mit dem ersten Intronabschnitt (3) ein vollständiges Intron bildet.

31. Nukleinsäure nach einem der vorangehenden Ansprüche, **dadurch gekennzeichnet, dass** in 3'-Richtung vom zweiten Intronabschnitt (5) ein vierter Intronabschnitt (10) angeordnet ist, der an seinem 3'-Ende die intronischen Anteile einer Spleißakzeptorstelle (17) und 5' davon einen funktionalen Poylpyrimidinabschnitt sowie einen funktionalen Verzweigungspunkt aufweist, und zusammen mit dem zweiten Intronabschnitt (5) ein vollständiges Intron bildet.

32. Nukleinsäure nach einem der Ansprüche 30 und 31, **gekennzeichnet dadurch, dass** die intronischen Anteile der Spleißdonor- (16) und der Spleißakzeptorstelle (17) an den Enden des dritten (9) und vierten Intronabschnittes (10) eingerichtet sind, nach Insertion in eine in einem Exon (A) eines Zielgens enthaltene Protospleißstelle (15) eine funktionelle Spleißdonor- (18) und eine funktionelle Spleißakzeptorstelle (19) zu bilden, und ein Exon (A), in das die Nukleinsäure integriert ist, in zwei getrennte Exons (A1, A2) geteilt ist.

33. Nukleinsäure nach einem der vorangehenden Ansprüche, **gekennzeichnet durch** zwei flankierende DNA-Bereich (9, 9'), die eine zur homologen Rekombination ausreichende Länge und ausreichend hohe Homologie zu DNA-Abschnitten eines Zielgens aufweisen.

34. Nukleinsäuren nach einem der vorangehenden Ansprüche, **gekennzeichnet dadurch, dass** sie von weiteren Sequenzen flankiert sind, die in keinem der beiden DNA-Stränge Exons, Spleißsignale wie Spleißdonorstellen, Spleißakzeptorstellen, Polypyrimidinabschnitte oder Verzweigungspunkte, und auch keine das Spleißen beeinflussende Sequenzen aufweisen.

35. Nukleinsäure nach einem der vorangehenden Ansprüche, **gekennzeichnet dadurch, dass** sie von retroviralen Sequenzen flankiert wird, deren Transkripte eingerichtet sind, in virale Partikel verpackt zu werden.

36. Nukleinsäure nach einem der vorangehenden Ansprüche, **gekennzeichnet dadurch, dass** sie von Erkennungssequenzen für eine eukaryontische und/oder prokaryontische Transposase flankiert ist.

37. Nukleinsäure nach einem der vorangehenden Ansprüche, **gekennzeichnet dadurch, dass** sie an ihren Enden einmalige identische Restriktionsschnittstellen aufweist.

38. Verwendung einer Nukleinsäure einem der vorangehenden Ansprüche zur Inaktivierung, konditionalen Inaktivierung oder konditionalen Reaktivierung und/oder zur mehrfachen konditionalen Änderung des Aktivitätszustandes eukaryontischer Gene in Zellen.

39. Verwendung einer Nukleinsäure nach einem der Ansprüche 1 bis 37 zur gezielten Mutagenese mittels homologer Rekombination in eukaryontischen Zellen, ausgenommen menschliche embryonale Stammzellen und menschliche Keimzellen.

40. Verwendung einer Nukleinsäure nach einem der Ansprüche 1 bis 37 als Genfalle in eukayontischen Zellen.

41. Verwendung einer Nukleinsäure nach einem der Ansprüche 1 bis 37 als Transposon in eukayontischen Zellen.

42. Verwendung einer Nukleinsäure nach einem der Ansprüche 1 bis 37 zur Herstellung einer pharmazeutischen Zusammensetzung zur Gentherapie.

43. Viraler Partikel, der eine Nukleinsäure nach einem der Ansprüche 1 bis 37 enthält.

44. Eukaryontische Zelle, ausgenommen menschliche Keimzelle, die mit einem viralen Partikel nach Anspruch 43 kontaktiert ist.

45. Verfahren zur Erzeugung eukaryontischer Zellen oder Organismen, ausgenommen Menschen, **gekennzeichnet durch** die Verwendung einer Nukleinsäure nach einem der Ansprüche 1 bis 37.

46. Eukaryontische Zelle oder nicht-menschlicher eukaryontischer Organismus, **gekennzeichnet durch** einen Gehalt an einer Nukleinsäure nach einem der Ansprüche 1 bis 37.
